(19) Europäisches Patentamt
European Patent Office
Office européen des brevets

(11) **EP 4 653 446 A1**

(12) **EUROPEAN PATENT APPLICATION**
published in accordance with Art. 153(4) EPC

(43) Date of publication:
**26.11.2025 Bulletin 2025/48**

(21) Application number: **24744230.4**

(22) Date of filing: **16.01.2024**

(51) International Patent Classification (IPC):
***C07D 519/00*** *(2006.01)* ***A61K 31/437*** *(2006.01)*
***A61P 35/00*** *(2006.01)* ***A61P 17/00*** *(2006.01)*
***A61P 19/02*** *(2006.01)* ***A61P 29/00*** *(2006.01)*
***A61P 5/48*** *(2006.01)* ***A61P 1/00*** *(2006.01)*
***A61P 11/00*** *(2006.01)* ***A61P 25/00*** *(2006.01)*

(86) International application number:
**PCT/CN2024/072461**

(87) International publication number:
**WO 2024/153054 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.01.2023 CN 202310059321**

(71) Applicant: **Hangzhou Yuhong Pharmaceutical Technology Co., Ltd.**
**Hangzhou, Zhejiang 310018 (CN)**

(72) Inventors:
- **CHEN, Binhui**
  **Hangzhou, Zhejiang 310018 (CN)**
- **HE, Qiaojun**
  **Hangzhou, Zhejiang 310018 (CN)**
- **WENG, Qinjie**
  **Hangzhou, Zhejiang 310018 (CN)**

(74) Representative: **Winter, Brandl - Partnerschaft mbB**
**Alois-Steinecker-Straße 22**
**85354 Freising (DE)**

(54) **CRYSTAL FORM OF AROMATIC HETEROCYCLIC COMPOUND, AND COMPOSITION COMPRISING SAME, PREPARATION METHOD THEREFOR AND USE THEREOF**

(57) The present disclosure provides a crystal form of aromatic heterocyclic compound, composition thereof, production method thereof and uses thereof. The crystal forms include: crystal form I and crystal form III, using X-ray diffraction method to have characteristic diffraction peaks at about 8.4°, 10.0°, 13.6°, 16.4°, and 19.7°, respectively; and at about 8.2°, 9.8°, 17.2°, and 26.8°; the aromatic heterocyclic compound is added with a suitable solvent for stirring, filtering, and drying to obtain crystal form I or III; and the use of crystal form I or crystal form III in the production of drugs for preventing or treating diseases caused by abnormal JAK-STAT signaling pathways. It can be used for autoimmune diseases, cancer, and myeloproliferative diseases.

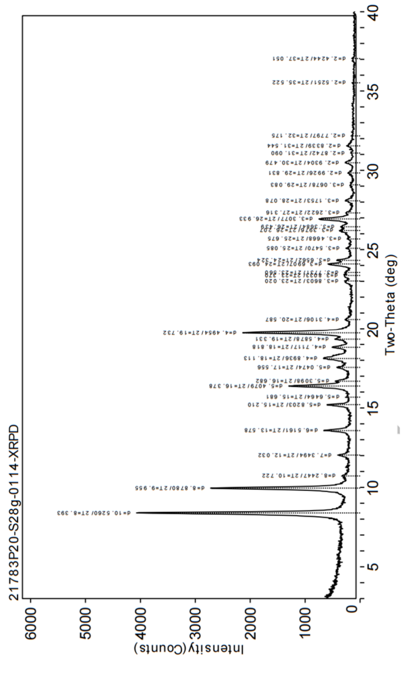


FIG.1



EP 4 653 446 A1

## Description

### Technical Field

The present disclosure belongs to the technical field of pharmaceutical chemistry, and specifically relates to a crystal form of an aromatic heterocyclic compound with Janus kinase (JAK) inhibitory activity, a composition thereof, a production method thereof and uses thereof.

### Background Art

Janus kinases (JAKs) are cytoplasmic tyrosine protein kinases responsible for transducing many inflammation-related cytokine signals from cytokine membrane receptors to STAT transcription factors. Abnormal JAK/STAT signal transduction is associated with many diseases, including immune-inflammatory diseases such as organ transplant rejection, multiple sclerosis, rheumatoid arthritis, type I diabetes, lupus, psoriasis, asthma, food allergies, atopic dermatitis, rhinitis, and rash. It has also been reported this it is closely related to the occurrence and development of solid and hematological malignancies and myeloproliferative disorders (including lung cancer, breast cancer, chronic spontaneous myelofibrosis, polycythemia, essential thrombocythemia, etc.).

JAK3 is mainly expressed in various hematopoietic tissue cells, including bone marrow cells, thymocytes, NK cells, activated B lymphocytes, T lymphocytes, etc. Its physiological effects are only derived from the signal transduction process of the common γ cytokine receptor family. Therefore, highly selective action on JAK3 kinase can avoid unnecessary side effects. Therefore, improving the activity and selectivity of JAK3 kinase inhibitors can further improve the clinical use effect of JAK3 kinase inhibitors, which have significant clinical advantages compared with the currently clinically used Pan-JAK inhibitors and selective JAK1 and JAK2 inhibitors.

The applicant's prior application (application number CN202211019771.6) studied the JAK3 kinase inhibitory activity and immunosuppressive activity (see paragraphs 425-510 of the specification and Figures 1-5) of the aromatic heterocyclic compounds (

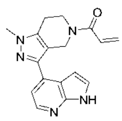

). It can be seen that the aromatic heterocyclic compound has good JAK3 kinase inhibitory activity, has a significant immunosuppressive effect on SRBC-induced delayed hypersensitivity in mice, can improve the symptoms of rheumatoid arthritis, can alleviate colorectal damage in mice with inflammatory bowel disease, and can significantly reduce the number of inflammatory cell infiltration in the lungs of mice caused by radiation.

Drug polymorphism is one of the important factors affecting drug quality and clinical efficacy. Different crystal forms will lead to differences in stability, absorption and bioavailability, so as to affect the clinical efficacy of drugs. Therefore, the crystal form of the aromatic heterocyclic compound and its production technology are of great significance to the application of the drug.

### Summary

The present disclosure intends to provide a crystal form of an aromatic heterocyclic compound, a composition thereof, a production method thereof and an use thereof. The aromatic heterocyclic compound crystal form can be used as a JAK kinase inhibitor for the treatment and prevention of clinical applications associated with abnormal activity of these kinases, including autoimmune diseases, cancer, myeloproliferative diseases and other diseases.

The present disclosure provides an aromatic heterocyclic compound having the structural formula:

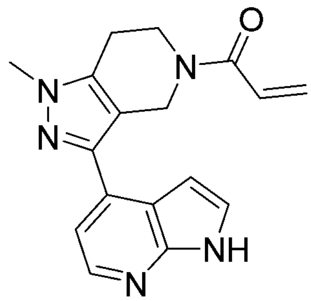

The aromatic heterocyclic compound may exist in a variety of different crystal forms.

The crystal form I of an aromatic heterocyclic compound, using X-ray diffraction, has characteristic diffraction peaks at 2θ angles of 8.4°, 10.0°, 13.6°, 16.4°, and 19.7°.

The crystal form II of an aromatic heterocyclic compound, using X-ray diffraction, has characteristic diffraction peaks at 2θ angles of 7.6°, 9.9°, 15.1°, 16.1°, and 19.7°.

The crystal form III of an aromatic heterocyclic compound, using X-ray diffraction, has characteristic diffraction peaks at 2θ angles of 8.2°, 9.8°, 17.2°, and 26.8°.

The crystal form IV of an aromatic heterocyclic compound, using X-ray diffraction, has characteristic diffraction peaks at 2θ angles of 7.9°, 9.4°, 15.7°, and 19.2°.

The crystal form V of an aromatic heterocyclic compound, using X-ray diffraction, has characteristic diffraction peaks at 2θ angles of 12.8°, 13.0°, 17.2°, and 22.8°.

The crystal form VI of an aromatic heterocyclic compound, using X-ray diffraction, has characteristic diffraction peaks at 2θ angles of 7.7°, 9.2°, and 19.7°.

The crystal form VII of an aromatic heterocyclic compound, using X-ray diffraction, has characteristic diffraction peaks at 2θ angles of 7.9°, 8.3°, and 9.9°.

The crystal form VIII of an aromatic heterocyclic compound, using X-ray diffraction, has characteristic diffraction peaks at 2θ angles of 8.0°, 9.6°, 18.8°, and 19.2°.

The crystal form IX of an aromatic heterocyclic compound, using X-ray diffraction, has characteristic diffraction peaks at 2θ angles of 8.1°, 9.6°; wherein, the error range of the 2θ angle is ±0.2°.

Furthermore, the crystal form I, using the X-ray diffraction method, also has characteristic diffraction peaks at 2θ angles of about 15.2°, 18.1°, 24.1°, and 26.9°; the crystal form IV, using the X-ray diffraction method, also has characteristic diffraction peaks at 2θ angles of about 16.6° and 18.6°; the crystal form V, using the X-ray diffraction method, also has characteristic diffraction peaks at 2θ angles of about 18.6° and 20.2°; the crystal form VII, using the X-ray diffraction method, also has characteristic diffraction peaks at 2θ angles of about 19.7°, 25.1°, and 33.5°; wherein, the error range of the 2θ angle is ±0.2°.

Furthermore, crystal form I has the X-ray diffraction diagram characteristics shown in FIG. 1; crystal form II has the X-ray diffraction diagram characteristics shown in FIG. 8; crystal form III has the X-ray diffraction diagram characteristics shown in FIG. 17; crystal form IV has the X-ray diffraction diagram characteristics shown in FIG. 25; crystal form V has the X-ray diffraction diagram characteristics shown in FIG. 29; crystal form VI has the X-ray diffraction diagram characteristics shown in FIG. 35; crystal form VII has the X-ray diffraction diagram characteristics shown in FIG. 42; crystal form VIII has the X-ray diffraction diagram characteristics shown in FIG. 51; crystal form IX has the X-ray diffraction diagram characteristics shown in FIG. 60;

Furthermore, the melting point of crystal form I is about 196°C. The melting point of crystal form II is about 184.4°C. The melting point of crystal form III is about 201°C. The melting point of crystal form IV is about 202°C. The melting point of crystal form V is about 223°C. The melting point of crystal form VI is about 202°C. The melting point of crystal form VII is about 194°C. The melting point of crystal form VIII is about 204°C.

The present disclosure further intends to provide a composition of the crystal forms of aromatic heterocyclic compound, which can be used as a JAK kinase inhibitor for the treatment and prevention of clinical applications associated with abnormal activity of these kinases, including autoimmune diseases, inflammatory diseases, cancer and other diseases.

A composition of crystal forms of the aromatic heterocyclic compound, including one or a combination of two or more of the crystal form I, crystal form II, crystal form III, crystal form IV, crystal form V, crystal form VI, crystal form VII, crystal form VIII and crystal form IX; and the crystal form I and/or the crystal form III account for 50% or more of the weight of the crystal composition. Preferably, the composition includes one or two of the crystal form I and the crystal form III; and the crystal form I or/and the crystal form III account for 50% or more of the weight of the composition.

The present disclosure further intends to provide a composition of the crystal forms of aromatic heterocyclic compound, which can be used as a JAK kinase inhibitor for the treatment and prevention of clinical applications associated

with abnormal activity of these kinases, including autoimmune diseases, inflammatory diseases, cancer and other diseases.

A composition of crystal forms of the aromatic heterocyclic compound, including one or a combination of two or more of the crystal form I, crystal form II, crystal form III, crystal form IV, crystal form V, crystal form VI, crystal form VII, crystal form VIII and crystal form IX; and the crystal form I and/or the crystal form III account for 80% or more of the weight of the crystal composition. Preferably, the composition includes one or two of the crystal form I and the crystal form III; and the crystal form I or/and the crystal form III account for 80% or more of the weight of the composition.

The present disclosure further intends to provide a composition of the crystal forms of aromatic heterocyclic compound, which can be used as a JAK kinase inhibitor for the treatment and prevention of clinical applications associated with abnormal activity of these kinases, including autoimmune diseases, inflammatory diseases, cancer and other diseases.

A composition of crystal forms of the aromatic heterocyclic compound, including one or a combination of two or more of the crystal form I, crystal form II, crystal form III, crystal form IV, crystal form V, crystal form VI, crystal form VII, crystal form VIII and crystal form IX; and the crystal form I and/or the crystal form III account for 90% or more of the weight of the crystal composition. Further preferably, the composition includes one or two of the crystal form I and the crystal form III; and the crystal form I or/and the crystal form III account for 90% or more of the weight of the composition.

The present disclosure further intends to provide a clinical application of the pharmaceutical composition containing the above mentioned crystal forms for treating and preventing abnormal activities of these kinases, including autoimmune diseases, inflammatory diseases, cancers and other diseases.

A pharmaceutical composition, including the crystal form I and/or the crystal form III, and a pharmaceutically acceptable carrier or excipient.

Another object of the present disclosure is to provide a method for producing crystal form I, wherein adding a solvent to the aromatic heterocyclic compound to form a suspension, stirring at room temperature, filtering, and drying to obtain a white solid.

Further, when producing the crystal form I, the solvent is one or a combination of two or more of acetone, methanol, ethanol, water, ethyl acetate, toluene, methyl tert-butyl ether, and n-heptane, and the amount of the organic solvent is 3 to 50 times the weight of the aromatic heterocyclic compound.

Preferably, a method for producing the crystal form I, wherein adding a solvent to the aromatic heterocyclic compound to form a suspension, stirring at room temperature, filtering, and drying to obtain a white solid crystal form I; the solvent is selected from water and ethanol. For 1 mg of aromatic heterocyclic compound, the required solvent volume is 0.01~0.2mL.

Another object of the present disclosure is to provide a method for producing crystal form I, wherein adding a solvent to the aromatic heterocyclic compound, dissolving, and volatilizing.

Furthermore, the solvent is one or a combination of two or more of acetone, chloroform, ethanol, tetrahydrofuran, 1,4-dioxane, and water.

Another object of the present disclosure is to provide a method for producing crystal form I, wherein adding a first organic solvent into the aromatic heterocyclic compound, heating to dissolve, and then adding a second organic solvent; then stirring and crystallizing; filtering and drying.

Furthermore, the first organic solvent is one or a combination of two or more of methanol, ethanol, dichloromethane, chloroform, and dimethyl sulfoxide; methanol is more preferably. The amount of the first organic solvent is 10 to 50 times the weight of the aromatic heterocyclic compound. The second organic solvent is one or a combination of two or more of isopropyl ether, n-hexane, n-heptane, methyl tert-butyl ether, and water; isopropyl ether is more preferably. The amount of the second organic solvent is 10 to 50 times the weight of the aromatic heterocyclic compound. When methanol is selected as the first organic solvent, the volume of methanol required for 1 mg of the aromatic heterocyclic compound is 0.1-0.3 mL; when isopropyl ether is selected as the second organic solvent, the volume of isopropyl ether required for 1 mg of the aromatic heterocyclic compound is 0.5-1.3mL. The volume ratio of isopropyl ether to methanol is 4-7:1, more preferably 5-6:1.

Another object of the present disclosure is to provide a method for producing crystal form III, wherein adding a first organic solvent into the aromatic heterocyclic compound, heating to dissolve, and then adding a second organic solvent; then stirring and crystallizing; filtering and drying.

Furthermore, when producing crystal form III, the first organic solvent is one or a combination of two or more of methanol, ethanol, tetrahydrofuran, 1,4-dioxane, and acetonitrile. The amount of the first organic solvent is 10 to 50 times the weight of the aromatic heterocyclic compound. The second organic solvent is one or a combination of two or more of water and ethyl acetate, and the amount of the second organic solvent is 10 to 50 times the weight of the aromatic heterocyclic compound.

As further preferred, when producing crystal form III, the first organic solvent is selected from ethanol and methanol; and the second organic solvent is selected from water. For 1 mg of aromatic heterocyclic compound, the volume of the first organic solvent required is 0.1-0.3 mL; the volume of the second organic solvent required is 0.5-1.6 mL; the

volume ratio of the second organic solvent to the first organic solvent is 4-7:1; more preferably 5-6:1.

Another object of the present disclosure is to provide an use of the above-described crystal forms in the production of a drug for preventing or treating a disease caused by an abnormal JAK-STAT signaling pathway. The drug can be used for autoimmune diseases, cancer, and myeloproliferative diseases.

Furthermore, the autoimmune disease is selected from one or more of alopecia areata, lupus, multiple sclerosis, amyotrophic lateral sclerosis, rheumatoid arthritis, rheumatoid arthritis, psoriasis, complications caused by organ transplantation, atopic dermatitis, autoimmune thyroid disease, ulcerative colitis, Crohn's disease, Sjögren's syndrome, vitiligo, autoimmune kidney damage, autoimmune liver damage, and chronic obstructive pulmonary disease; the cancer is selected from one or more of colon cancer, gastric adenocarcinoma, bladder cancer, breast cancer, kidney cancer, liver cancer, lung cancer, thyroid cancer, head and neck cancer, prostate cancer, pancreatic cancer, CNS (central nervous system) cancer, and malignant glioma; the bone marrow proliferation disease is selected from one or more of chronic myelomonocytic leukemia, atypical chronic myelocytic leukemia and juvenile myelomonocytic leukemia.

The aromatic heterocyclic compound described in the present disclosure exists in a variety of crystal forms. When using hot stage XRPD to study the crystal forms of the compound, researchers found that at 155°C, the sample transformed into another thermodynamically more stable anhydrous crystal form, which defined as crystal form I. At the same time, eight other crystal forms were found, including crystal form II, crystal form III, crystal form IV, crystal form V, crystal form VI, crystal form VII, crystal form VIII and crystal form IX, wherein crystal form I and crystal form III were anhydrous crystal forms. It can be seen that the aromatic heterocyclic compound of the present disclosure has polymorphism, most of which are solvates or hydrates. Anhydrous crystal forms cannot be obtained under most production conditions. Therefore, the production of anhydrous crystal forms I and III has great technical difficulty. Solvates or hydrates are prone to desolvation and crystal transformation during storage. The crystal composition ratio of the compound is different at different storage times, which will lead to unstable quality of the compound. The crystal form I and III provided by the present disclosure are anhydrous crystal forms, and no crystal transformation occurs during storage, which can overcome the above-mentioned defects of the compound.

The researchers compared the above nine crystal forms through hot stage XRPD, TG, DSC, DVS etc. Hot stage XRPD analysis revealed that almost all crystal forms would transform into form I at 150-170°C. TG and DSC analyses also revealed that form I was the most stable during heating. DVS and isotherm curves show that the structure of crystal form I is stable under desorption and adsorption conditions.

At the same time, the researchers conducted competitive tests on the above-mentioned crystal forms in water and ethanol to examine the stability and transformation of various crystal forms in solvents. Crystal competition experiments showed that crystal forms I and III had good crystal stability in water, while other crystal forms were unstable in water. Water is the most commonly used solvent in pharmaceutical productions, and solid powders are also prone to absorb water when exposed to air. Therefore, the crystal forms I and III provided in the disclosure have good stability in the field of pharmaceutical applications and are suitable for development as medicinal crystal forms. Crystal form I has good crystal stability in the presence of ethanol. Ethanol is widely used in the production of pharmaceutical solvents and pharmaceutical productions. The above experiments show that crystal form I has significant stability advantages in the development of pharmaceutical productions in the presence of ethanol.

The researchers conducted a 15-day high temperature and high humidity test on crystal form I, and a long-term, accelerated 3-month test. The test results show that all of them is crystal form I, further proving the stability of crystal form I during storage.

At the same time, the researchers conducted solubility tests on crystal form I at different pH values. The test results show that the solubility increased significantly as the pH value decreased, indicating that the samples can dissolve quickly when entering the human stomach.

## BRIEF DESCRIPTION OF THE DRAWINGS

FIG. 1 is an X-ray diffraction diagram of crystal form I in the present disclosure.
FIG. 2 is an X-ray diffraction peak data diagram of crystal form I in the present disclosure.
FIG. 3 is a TG diagram of crystal form I in the present disclosure.
FIG. 4 is a DSC diagram of crystal form I in the present disclosure.
FIG. 5 is a DVS diagram of crystal form I in the present disclosure.
FIG. 6 is an isothermal curve diagram of crystal form I in the present disclosure.
FIG. 7 is a PLM diagram of crystal form I in the present disclosure.
FIG. 8 is an X-ray diffraction diagram of crystal form II of the present disclosure.
FIG. 9 is an X-ray diffraction peak data diagram of crystal form II in the present disclosure.
FIG. 10 is a TG diagram of crystal form II in the present disclosure.

FIG. 11 is a DSC diagram of crystal form II of the present disclosure.
FIG. 12 is a DVS diagram of crystal form II of the present disclosure.
FIG. 13 is an isothermal curve diagram of crystal form II of the present disclosure.
FIG. 14 is a PLM diagram of crystal form II of the present disclosure.
FIG. 15 is a hot-stage XRPD diagram of crystal form II of the present disclosure.
FIG. 16 is a hot stage $^1$H-NMR diagram of crystal form II of the present disclosure.
FIG. 17 is an X-ray diffraction diagram of crystal form III of the present disclosure.
FIG. 18 is an X-ray diffraction peak data diagram of crystal form III in the present disclosure.
FIG. 19 is a TG diagram of crystal form III of the present disclosure.
FIG. 20 is a DSC diagram of crystal form III of the present disclosure.
FIG. 21 is a DVS diagram of crystal form III of the present disclosure.
FIG. 22 is an isothermal curve diagram of crystal form III of the present disclosure.
FIG. 23 is a PLM diagram of crystal form III of the present disclosure.
FIG. 24 is a hot stage XRPD diagram of crystal form III of the present disclosure.
FIG. 25 is an X-ray diffraction diagram of crystal form IV of the present disclosure.
FIG. 26 is an X-ray diffraction peak data diagram of crystal form IV of the present disclosure.
FIG. 27 is a TG diagram of crystal form IV of the present disclosure.
FIG. 28 is a DSC diagram of crystal form IV of the present disclosure.
FIG. 29 is an X-ray diffraction diagram of crystal form V of the present disclosure.
FIG. 30 is an X-ray diffraction peak data diagram of crystal form V in the present disclosure.
FIG. 31 is a TG diagram of crystal form V in the present disclosure.
FIG. 32 is a DSC graph of crystal form V in the present disclosure.
FIG. 33 is a PLM diagram of crystal form V of the present disclosure.
FIG. 34 is a hot stage $^1$H-NMR graph of crystal form V in the present disclosure.
FIG. 35 is an X-ray diffraction diagram of crystal form VI of the present disclosure.
FIG. 36 is an X-ray diffraction peak data diagram of crystal form VI in the present disclosure.
FIG. 37 is a TG diagram of crystal form VI of the present disclosure.
FIG. 38 is a DSC graph of crystal form VI of the present disclosure.
FIG. 39 is a PLM diagram of crystal form VI of the present disclosure.
FIG. 40 is a hot stage XRPD diagram of crystal form VI of the present disclosure.
FIG. 41 is a hot stage $^1$H-NMR diagram of crystal form VI of the present disclosure.
FIG. 42 is an X-ray diffraction diagram of crystal form VII of the present disclosure.
FIG. 43 is an X-ray diffraction peak data diagram of crystal form VII of the present disclosure.
FIG. 44 is a TG diagram of crystal form VII of the present disclosure.
FIG. 45 is a DSC diagram of crystal form VII of the present disclosure.
FIG. 46 is a DVS diagram of crystal form VII of the present disclosure.
FIG. 47 is an isothermal curve diagram of crystal form VII of the present disclosure.
FIG. 48 is a PLM diagram of crystal form VII of the present disclosure.
FIG. 49 is a hot stage XRPD diagram of crystal form VII of the present disclosure.
FIG. 50 is a hot stage $^1$H-NMR diagram of crystal form VII of the present disclosure.
FIG. 51 is an X-ray diffraction diagram of crystal form VIII in the present disclosure.
FIG. 52 is an X-ray diffraction peak data diagram of crystal form VIII in the present disclosure.
FIG. 53 is a TG diagram of crystal form VIII in the present disclosure.
FIG. 54 is a DSC diagram of crystal form VIII in the present disclosure.
FIG. 55 is a DVS diagram of crystal form VIII in the present disclosure.
FIG. 56 is an isothermal curve diagram of crystal form VIII in the present disclosure.
FIG. 57 is a PLM diagram of crystal form VIII of the present disclosure.
FIG. 58 is a hot stage XRPD diagram of crystal form VIII in the present disclosure.
FIG. 59 is a hot stage $^1$H-NMR diagram of crystal form VIII of the present disclosure.
FIG. 60 is an X-ray diffraction diagram of crystal form IX in the present disclosure.
FIG. 61 is an X-ray diffraction peak data diagram of crystal form IX in the present disclosure.
FIG. 62 is a TG diagram of crystal form IX in the present disclosure.
FIG. 63 is a DSC diagram of crystal form IX of the present disclosure.
FIG. 64 is a PLM diagram of crystal form IX in the present disclosure.
FIG. 65 is a hot stage XRPD diagram of crystal form IX in the present disclosure.
FIG. 66 is a hot stage $^1$H-NMR diagram of crystal form IX of the present disclosure.
FIG. 67 is an X-ray diffraction diagram of crystal form I in the present disclosure at high temperature for 15 days.
FIG. 68 is an X-ray diffraction peak data diagram of crystal form I in the present disclosure at high temperature for 15

days.

FIG. 69 is an X-ray diffraction diagram of crystal form I in the present disclosure at high humidity for 15 days.

FIG. 70 is an X-ray diffraction peak data diagram of crystal form I in the present disclosure at high humidity for 15 days.

FIG. 71 is the X-ray diffraction diagram of crystal form I in the present disclosure over a long period of 3 months.

FIG. 72 is an X-ray diffraction peak data diagram of crystal form I in the present disclosure over a long period of 3 months.

FIG. 73 is an X-ray diffraction diagram of the present disclosure accelerated for 3 months.

FIG. 74 is an X-ray diffraction peak data diagram in the present disclosure accelerated for 3 months.

FIG. 75 is the solubility diagram of crystal form I sample at different pH values.

FIG. 76 shows the immunosuppressive activity of the compounds in the SRBC mouse model.

FIG. 77 shows the immunosuppressive effects of the compounds on the collagen-induced arthritis model in mice.

FIG. 78 shows the immunosuppressive effects of the compounds on the dextran sodium sulfate (DSS)-induced mouse inflammatory bowel disease model.

FIG. 79 shows the inhibitory effect of the compounds on TNF-α levels in mice with acute radiation-induced lung injury.

FIG. 80 shows that the compound reduces the number of inflammatory cell infiltration in the lungs of mice with acute radiation lung injury.

## DETAILED DESCRIPTION

In order to explain the present disclosure more clearly, the present disclosure is further described below in conjunction with preferred embodiments. Those skilled in the art should understand that the following specific description is illustrative rather than restrictive, and should not be used to limit the scope of protection of the present disclosure.

All numerical specifications herein (eg, temperature, time, concentration, and weight, etc., including ranges for each thereof) may generally be varied (+) or (-) by increments of 0.1 or 1.0 as appropriate. All numerical specifications may be understood as being preceded by the term "about".

**Example 1 (Comparative Example):** Aromatic Heterocyclic Compound

Referring to the production process of Example 91 of application number CN202211019771.6, the production of the aromatic heterocyclic compound is carried out as follows:

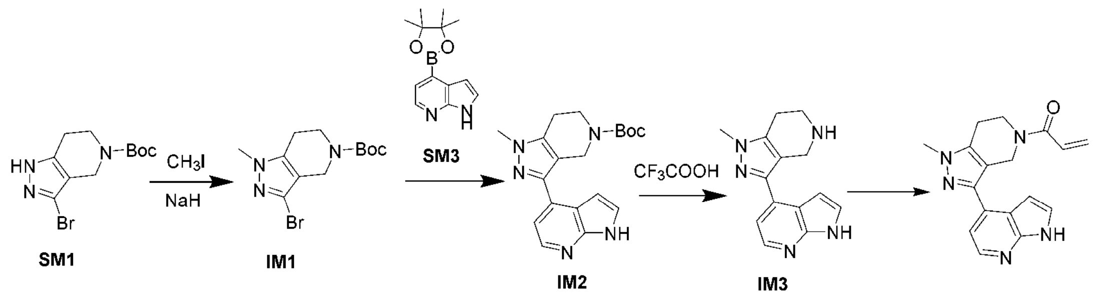

SM1 IM1 IM2 IM3

Step 1: The intermediate SM1 (150g, 0.50mol), sodium hydrogen (25g, 1.04mol), and iodomethane (73g, 0.52mol) were successively added into a 2000mL three-necked flask containing 1000mL DMF. Afer being stirred at 0°C for 2 hours, the reaction mixture was poured into water, extracted with dichloromethane three times.The organic phases were combined, washed twice with saturated sodium chloride, dried on anhydrous sodium sulfate, and concentrated under reduced pressure. The crude product was purified by silica gel column chromatography to obtain 100 g of intermediate IM1 as a yellow solid with a yield of 63.3%.

Step 2: Under the protection of nitrogen, the intermediate **IM1** (100g, 0.32mol), tetrakis (triphenylphosphine) palladium (38g, 33mmol), the intermediate SM3 (121g, 0.5mol) and potassium carbonate (137g, 0.99mol) were sequentially added into a 2000ml three-necked flask containing 1000mL of 1,4-dioxane. Afte being stirred at 95°C for overnight, the reaction mixture was cooled to room temperature and poured into 1000ml water. The whole was extractd with ethyl acetate three times.The organic phases were combined, washed twice with saturated sodium chloride, dried over anhydrous sodium sulfate, and concentrated under reduced pressure. The crude product was purified by silica gel column chromatography to obtain 50g of intermediate **IM2** as a light yellow solid with a yield of 44.3%.

Step 3: The intermediate **IM2** (50g, 0.14mol) was dissolved in dichloromethane, and an equal volume of trifluoroacetic acid was added. After reacting at room temperature for overnight, the whole was acidified to pH 8-9, and extracted with dichloromethane three times. The organic layers were combined, washed twice with saturated sodium chloride, dried over anhydrous sodium sulfate, and concentrated under reduced pressure. The crude product was purified

by silica gel column chromatography to obtain 20g of intermediate IM3 as a white solid with a yield of 56.4%.

Step 4: The intermediate **IM3** (20g, 78.9mmol) was dissolved in a mixture of dichloromethane(4000 ml) and triethylamine (24.5g, 243mmol), and to the solution was added acryloyl chloride (8.66g, 95mmol) slowly under ice bath. After reacting at room temperature for 3 hours, a saturated aqueous sodium bicarbonate solution was added. The whole was extracted with dichloromethane for 3 times. The organic layers were combined, dried over anhydrous sodium sulfate, and concentrated under reduced pressure. The crude product was purified by silica gel column chromatography with developing agent dichloromethane and methanol to obtain 15g white solid target compound with a yield of 62%. $^1$H NMR (400 MHz, DMSO-d6) δ 11.65 (s, 1H), 8.28 (d, *J* = 4.8 Hz, 1H), 7.47 (s, 1H), 7.10 - 7.03 (m, 1H), 7.00 (d , *J* = 4.7 Hz, 1H), 6.95 - 6.85 (m, 1H), 6.13 (d, *J* = 15.1 Hz, 1H), 5.73 (d, *J* = 12.3 Hz, 1H), 4.81 (d, *J* =14.2 Hz, 2H), 3.91 (d, *J* = 5.2 Hz, 2H), 3.83 (s, 3H), 2.83 (s, 2H) ESI(M+H)+=308.14959.

The obtained white solid X-ray diffraction diagram showed characteristic diffraction peaks at 8.11° and 9.62° at 2θ angles (see FIG. 60 and 61); TGA showed a weight loss of 4.2% before 130°C, a weight loss of 3.7% before 130°C-210°C, and the sample decomposed at 361°C (see FIG. 62); DSC showed a desolvation peak at 20°C-140°C, an endothermic peak at 171°C, and a melting point of 199.8°C (see FIG. 63). PLM showed that the sample was a flaky crystal (see FIG. 64); hot stage XRPD showed that it transformed into crystal form I at 155°C and remained crystal form I when cooled to room temperature (see the two XRD diagrams marked as 155°C and RT in FIG. 65, respectively) (in the Figure, Form 1 is crystal form I, produced in Example 4); $^1$H-NMR showed that the marked peaks were water peaks and methanol peaks (see FIG. 66), which was a hydrate; the 1-(1-methyl-3-(1H-pyrrolo[2,3-b]pyridin-4-yl)-1,4,6,7-tetrahydro-5H-pyrazolo[4,3-c]pyridin-5-yl)prop-2-en-1-one obtained by the above production method was crystal form IX.

It can be seen that crystal form IX is not an anorthite form and is prone to crystal transformation during storage, resulting in unstable quality during long-term storage.

### Example 2

20mg of the aromatic heterocyclic compound obtained in Example 1 was added to 4ml of methanol and 2ml of chloroform. The mixture was dissolved by ultrasonication and evaporated to dryness in an open air at room temperature to obtain a solid.

The obtained solid X-ray diffraction diagram shows as crystal form IX.

### Example 3

10mg of the aromatic heterocyclic compound obtained in Example 1 was added to 4ml of methanol. The mixture was dissolved under ultrasonication, and evaporated to dryness in an open air at room temperature to obtain a solid.

The obtained solid X-ray diffraction diagram shows as crystal form IX.

### Example 4

20mg of the aromatic heterocyclic compound obtained in Example 1 was added to 2ml of water to form a suspension, the suspension was stirred at room temperature for 4 days, centrifuged, and dried at 45°C to obtain a white solid.

The obtained white solid X-ray diffraction diagram showed that the 2θ angles were at 8.39°, 9.96°, 13.58°, 15.21°, 16.38°, 18.11°, 19.73°, 24.09° and 26.93° with characteristic diffraction peaks, respectively (see FIG. 1 and 2). TGA showed a weight loss of 0.93% before 150°C, and the sample decomposed at 354°C (see FIG. 3) and was anhydrous. DSC showed a melting point of about 196°C (see FIG. 4). DVS and isotherm curves show that the moisture absorption rate is about 2.4% (see FIG. 5 and 6), and the desorption and adsorption curves are almost overlapping, indicating that the structure has not changed before and after; PLM shows that the sample is fine particles (see FIG. 7); the white solid obtained by the above production method is crystal form I.

### Example 5

20mg of the aromatic heterocyclic compound obtained in Example 1 and 4ml of acetone were dissolved by stirring and filtered, the filtrate was added dropwise to nheptane(14ml) , cooled to 4°C, and stirred to crystallize overnight, and the precipitated solid was centrifuged and dried at 45°C to obtain a white solid.

The obtained white solid X-ray diffraction diagram showed that the 2θ angles were at 6.68°, 26.86°, 7.59°, 9.91°, 15.06°, 16.15°, 19.08°, and 19.71° with characteristic diffraction peaks (see FIG. 8 and 9). TGA showed a weight loss of 0.7% before 60°C and a weight loss of 8.02% from 60°C to 200°C, and the sample decomposed at 355°C (see FIG. 10). DSC showed desolvation peaks at 20°C-70°C and 70°C-160°C, and the melting point was about 184.4°C (see FIG. 11). DVS and isotherm curves showed that the moisture absorption rate was about 2.4% (see FIG. 12 and 13); PLM showed

that the sample was flaky crystals (see FIG. 14); hot stage XRPD showed that the crystal transformed into crystal form I at 150°C and remained crystal form I at room temperature (see FIG. 15); $^1$H-NMR showed that the marked peak was a water peak (see FIG. 16), which was a hydrate; the white solid obtained by the above production method was crystal form II.

### Example 6

20mg of the aromatic heterocyclic compound obtained in Example 1 and 3ml of ethanol were dissolved by stirring and filtered, the filtrate was added dropwise to water(16 ml), cooled to 4°C, and stirred to crystallize overnight, and the precipitated solid was centrifuged, and dried at 45°C to obtain a white solid.

The obtained white solid X-ray diffraction diagram showed characteristic diffraction peaks at 2θ angles of 8.18°, 9.76°, 17.18°, and 26.81° (see FIG. 17 and 18), TGA showed a weight loss of 2.38% at 125°C, and the sample decomposed at 359°C (see FIG. 19). DSC showed a desolvation peak at 20°C-140°C, an endothermic peak at 190°C, and a melting point of about 201°C (see FIG. 20). DVS and isotherm curves showed that the moisture absorption rate was about 2.2% (see FIG. 21 and 22); PLM showed that the sample was fine particles (see FIG. 23); hot stage XRPD showed that there was no crystal transformation at 140°C (see FIG. 24); the white solid obtained by the above production method was crystal form III.

### Example 7

20mg of the aromatic heterocyclic compound obtained in Example 1 and 3ml of methanol were dissolved by stirring and filtered, the filtrate was added dropwise to water(16ml), cooled to 4°C, stirred to crystallize overnight, and the precipitated solid was centrifuged and dried at 45°C to obtain a white solid.

The obtained white solid X-ray diffraction diagram shows as crystal form III.

### Example 8

50mg of the aromatic heterocyclic compound obtained in Example 1 and 10ml of dichloromethane were dissolved by stirring and filtered; the filtrate was dried under reduced pressure to obtain a white solid.

The obtained white solid X-ray diffraction diagram showed characteristic diffraction peaks at 2θ angles of 7.88°, 9.44°, 15.71°, and 19.24° (see FIG. 25 and 26). TGA showed a weight loss of 11.97% at 160°C, the sample decomposed at 350°C, and contained 0.5 parts of dichloromethane solvate (see FIG. 27). DSC showed a desolvation peak at 40°C-170°C, and a melting point of about 202°C (see FIG. 28). The obtained white solid X-ray diffraction diagram shows as crystal form IV.

### Example 9

10mg of the aromatic heterocyclic compound obtained in Example 1 was added 4ml of 1,4-dioxane, the mixture was dissolved by ultrasonication and filtered. The filtrate was evaporated to dryness at 40° C and grinded to obtain a white solid.

The obtained white solid X-ray diffraction diagram showed that the 2θ angles were at 8.49°, 11.44°, 12.79°, 12.97°, 17.20°, 18.59°, 20.19°, 21.90°, 22.78°, 25.39°, and 28.52° with characteristic diffraction peaks (see FIG. 29 and 30). TGA showed a weight loss of 9.65% before 125°C, and the sample decomposed at 354°C (see FIG. 31). DSC showed a desolvation peak at 85°C-140°C, and a melting point of about 223°C (see FIG. 32). PLM showed that the sample was fine particles (see FIG. 33); $^1$H-NMR showed that the peak at the mark was 1,4-dioxane (see FIG. 34), which was 0.5 parts of 1,4-dioxane solvate; the white solid obtained by the above production method was crystal form V.

### Example 10

20mg of the aromatic heterocyclic compound obtained in Example 1 was added 3ml of methanol to dissolve. The mixture was added 16ml of isopropyl ether to precipitate a solid, centrifuged, and dried the obtained solid at 45° C to obtain a white solid.

The obtained white solid X-ray diffraction diagram shows as crystal form I.

### Example 11

10mg of the aromatic heterocyclic compound obtained in Example 1 was added to 4ml of n-butanol and 5ml of dichloromethane, the mixture was dissolved under ultrasonication and filtered, the filtrate was evaporated to dryness at 40° C and grinded to obtain a white solid.

The obtained white solid X-ray diffraction diagram showed characteristic diffraction peaks at 2θ angles of 7.66°, 9.18°, and 19.66° (see FIG. 35 and 36); TGA showed a weight loss of 10.9% before 150°C, and the sample decomposed at 357°C (see FIG. 37); DSC showed a desolvation peak at 105°C~155°C, and a melting point of about 202°C (see FIG. 38). PLM showed that the sample was block crystals (see FIG. 39); hot stage XRPD showed that the crystal transformed into crystal form I at 150°C and remained crystal form I at room temperature (see FIG. 40); $^1$H-NMR showed that the peak at the marked point was the n-butanol peak (see FIG. 41), which was 0.5 parts of n-butanol solvate; the white solid obtained by the above production method was crystal form VI.

**Example 12**

10mg of the aromatic heterocyclic compound obtained in Example 1 was added to 4ml of tetrahydrofuran, the mixture was dissolved under ultrasonication and filtered, the filtrate was evaporated to dryness at 50° C and grinded to obtain a white solid.

The obtained white solid X-ray diffraction diagram showed characteristic diffraction peaks at 2θ angles of 7.88°, 8.28°, 9.92°, 19.66°, 25.09°, and 33.45° (see FIG. 42 and 43). TGA showed a weight loss of 6.0% before 200°C, and the sample decomposed at 358°C (see FIG. 44). DSC showed a desolvation peak at 20°C ~ 145°C, and a melting point of about 194°C (see FIG. 45). DVS and isotherm curves showed that the moisture absorption rate was about 1.3% (see FIG. 46 and 47); PLM showed that the sample was flaky crystals (see FIG. 48); hot stage XRPD showed that the crystal transformed to crystal form I at 150°C and remained crystal form I at room temperature (see FIG. 49); $^1$H-NMR showed that the marked peak was a water peak (see FIG. 50), which was a hydrate; the white solid obtained by the above production method was crystal form VII.

**Example 13**

10mg of the aromatic heterocyclic compound obtained in Example 1 was added to 4ml of acetonitrile. The mixture was dissolved by ultrasonication and filtered.The filtrate was evaporatied to dryness at 40° C, grinding to obtain a white solid.

The obtained white solid X-ray diffraction diagram showed characteristic diffraction peaks at 2θ angles of 8.03°, 9.59°, 18.78°, and 19.22° (see FIG. 51 and 52); TGA showed a weight loss of 2.5% before 125°C, a weight loss of 4.0% before 125°C-145°C, and the sample decomposed at 362°C (see FIG. 53); DSC showed desolvation peaks at 15°C-130°C and 130°C-170°C, an endothermic peak at 181°C, and a melting point of about 204°C (see FIG. 54). DVS and isotherm curves showed that the moisture absorption rate was about 1.3% (see FIG. 55 and 56); PLM showed that the sample was block crystals (see FIG. 57); hot stage XRPD showed that most of the crystals were transformed into crystal form I at 150°C (see FIG. 58); $^1$H-NMR showed that the marked peaks were water peaks and solvent acetonitrile peaks (see FIG. 59), which were hydrates; the white solid obtained by the above production method was crystal form VIII.

**Example 14**

30mg of the aromatic heterocyclic compound obtained in Example 1 was added to 2ml of ethanol to form a suspension, the suspension was stirred at room temperature for 4 days, and centrifuged, the resulting solid was dried at 45°C to give a white solid.

The obtained white solid X-ray diffraction diagram shows as crystal form I.

**Example 15**

10g of the aromatic heterocyclic compound obtained in Example 1 was added to 100 ml of ethanol to form a suspension, the suspension was stirred at room temperature for 24 days, and the precipitated solid product was filtered and dried to obtain 8.9g of a white solid with a yield of 89%.

The obtained white solid X-ray diffraction diagram shows as crystal form I.

**Example 16**

2mg each of the aromatic heterocyclic compound of crystal form I, crystal form II, crystal form III, crystal form IV, crystal form V, crystal form VI, crystal form VII, crystal form VIII and crystal form IX (a total of 18mg) were added to 3ml of water to form a suspension, the suspension was stirred at room temperature for 4 days and 7 days, and samples were taken on the 4th day and the 7th day for X-ray diffraction detection, and the results show crystal form I and crystal form III, and there is no obvious change on the 4th day and the 7th day.

The above is a crystal competition experiment, which shows that crystal forms I and III have good crystal stability

in water, while other crystal forms are unstable in water. Water is the most commonly used solvent in pharmaceutical productions, and solid powders are also prone to absorb water when exposed to air. Therefore, the crystal forms I and III provided in the disclosure have good stability in the field of pharmaceutical applications and are suitable for development as medicinal crystal forms.

**Example 17**

2mg each of the aromatic heterocyclic compound of crystal form I, crystal form II, crystal form III, crystal form IV, crystal form V, crystal form VI, crystal form VII, crystal form VIII and crystal form IX was added to 3ml of anhydrous ethanol to form a suspension. After being stirred at room temperature for 4 days, samples were taken for X-ray diffraction detection, and the results show crystal form I, and the characteristic peaks of other forms disappear.

The above is a crystal competition experiment (ethanol stimulation), which shows that crystal form I has good crystal stability in the presence of ethanol. Ethanol is widely used in the production of pharmaceutical solvents and pharmaceutical productions. The above experiments show that crystal form I has significant stability advantages in the development of pharmaceutical productions in the presence of ethanol.

**Example 18**

Samples of crystal form I produced in Example 15 were taken, which were 15 samples of 50mg each, and sealed with aluminum foil bags. The crystal stability of the above samples were studied under high temperature (60±2°C), high humidity (RH90%±5%), long-term test (25°C±2°C, RH 60%±5%) and accelerated test (40°C±2°C, RH 75%±5%) conditions. The test results show that the crystal form I is stable after 15 days of high temperature and high humidity test. The XRD diagram after 15 days of high temperature and high humidity test is shown in FIGs. 67-70. The long-term and accelerated 3-month crystal form is stable. The long-term and accelerated 3-month XRD diagrams are shown in FIGs. 71-74. The influencing factors and stability test results fully demonstrate that crystal form I can be stored stably.

Influencing factors test crystal form test results

| Influencing factor test | Test conditions | Preservation mode | Test results | | |
|---|---|---|---|---|---|
| | | | 0 days | 5 days | 15 days |
| High temperature test | 60±2°C | Inner packaging transparent ziplock bag, outer packaging aluminum foil bag sealed | Crystal form I | Crystal form I | Crystal form I |
| High humidity test | RH90%±5% | | | Crystal form I | Crystal form I |

Stability test crystal form test results

| Influencing factors Test | Test conditions | Preservation mode | Test results | | |
|---|---|---|---|---|---|
| | | | 0 days | 1 month | 3 month |
| Long-term test | 25°C±2°C, RH 60%±5% | Inner packaging transparent ziplock bag, outer packaging aluminum foil bag sealed | Crystal form I | Crystal form I | Crystal form I |
| Accelerated test | 40°C±2°C, RH 75%±5% | | | Crystal form I | Crystal form I |

**Example 19**

Excess samples of crystal form I were respectively added to 10 mL of solutions with different pH value. After being shaken for 24 hours by a constant temperature shaker at 32°C at 100 rpm, the saturated solutions (with undissolved solids at the bottom) were centrifuged and filtered, then detrmined linear solutions (formulated with reference substances) with different concentrations and saturated solutions with different pH values under the content item. The solubility of the samples at different pH values were calculated using the standard curve as follows, see FIG. 75 for details.

| Buffer solution | Hydrochloric acid solution | | Phosphate buffered solution | | | | |
|---|---|---|---|---|---|---|---|
| pH value | 1.0 | 2.0 | 3.8 | 4.5 | 6.8 | water | 8.0 |
| Solubility (μg/ml) | 14531.36 | 2735.78 | 118.36 | 68.62 | 58.93 | 53.23 | 60.77 |

The test results show that the solubility increases significantly with decreasing pH value, especially the solubility of crystal form I at pH 1.0 reaches 14531.36 μg/ml, indicating that the sample can dissolve quickly in the human stomach and crystal form I is suitable for development as an oral solid production.

**Example 20 Activity verification of aromatic heterocyclic compounds**

The activities verified for the aromatic heterocyclic compound (produced in Example 91 of the prior application (application number CN202211019771.6), referred to as YZ001052) included JAK3 kinase inhibitory activity and immune activity, as shown in Examples 20A to 20F.

**Example 20A JAK3 kinase inhibitory activity of aromatic heterocyclic compounds (the experiment was commissioned by Sundia Pharmaceutical Technology (Shanghai) Co., Ltd.)**

**Experimental Purpose:**

Mobility shift assay was used to detect the in vitro inhibitory activity of the test compounds (listed below) on JAK3 kinase activity. Cerdulatinib (supplier: selleckchem; Article number: S7634) was used as a positive control compound.

**Experimental methods:**

1. Compound formulation

Compound was dissolved in 100% DMSO to formulate a 10 mM stock solution and stored in a -20°C refrigerator away from light.

2. Kinase reaction process

(1) 1 × Kinase buffer was prepared.
(2) formulating compound concentration gradient: the test compounds (including the example compound and PF-06651600) were tested at a concentration of 10000nM, 10-fold dilution, 10 concentrations, single-well detection. After being gradient dilution to a 100-fold final concentration, 250nL of solutions were transfered to a 384-well plate using Echo550. 250nL of 100% DMSO were added to each of the negative control wells and the positive control wells, respectively.
(3) 2.5 times a final concentration of kinase solution was prepared with 1 × Kinase buffer.
(4) 10μL of kinase solutions at 2.5 times were respectively added to the final concentration to the compound wells and positive control wells; and 10μL of 1× Kinase buffer was added to the negative control wells.
(5) The reaction mixtures were centrifuged at 1000 rpm for 30 seconds, shaken to mix, and incubated at room temperature for 10 minutes.
(6) A mixed solution of 25/15 the final concentration of ATP and Kinase substrate 22 was prepared with 1 × Kinase buffer.
(7) The reaction was initiated by adding 15μL of a mixed solution of 25/15 times the final concentration of ATP and substrate to the compound wells, positive control wells, and negative control wells of the 384-well plate.
(8) The reaction mixtures in the 384-well plate were centrifuged at 1000 rpm for 30 seconds, shaken to mix, and incubated at room temperature for 30 minutes.
(9) 30μL of stop detection solution was added to stop the kinase reaction, and the reaction mixtures were centrifuged at 1000 rpm for 30 seconds, and shaken to mix.
(10) The conversion rate was read by Caliper EZ Reader.

3. Data Analysis

(1) Calculation formula

$$\% \text{ Inhibition} = (\text{Conversion\%_max} - \text{Conversion\%_sample})/(\text{Conversion\%_max} - \text{Conversion\%_min}) \times 100$$

Wherein: Conversion%_sample is the conversion rate reading of the sample; Conversion%_min: the mean value of the negative control wells, representing the conversion rate reading of the wells without enzyme activity; Conversion% _max: the mean value of the positive control wells, representing the conversion rate reading of the wells without compound inhibition; %Inhibition represents the percentage inhibition rate.

(2) Fitting the dose-effect curve

Dose-effect curves were fitted using the log (inhibitor) vs. response -Variable slope of the analysis software GraphPad Prism 5. which used the log value of the concentration as the X-axis and the percentage inhibition rate as the Y-axis to obtain the IC50 value of each compound on the enzyme activity, The calculation formula is Y=Bottom + (Top-Bottom)/ (1+10^((LogIC50-X)*HillSlope)).

**Experimental results:**

| Compound No. | JAK3 IC50 (nM) | Compound No. | JAK3 IC50 (nM) |
|---|---|---|---|
| YZ001052 | 0.25 | PF-06651600 | 18 |

The data in the above table show that YZ001052 has better JAK3 in vitro kinase inhibitory activity than PF-06651600.

**Example 20B Cell activity experiment**

**Experimental Purpose:**

Based on the mechanism of JAK-STAT pathway, the effect of the compounds on the JAK-STAT pathway were evaluated by using cytokine IL-15 to stimulate hPBMC cells, and using the downstream STATS phosphorylation level as the detection index.

**Experimental methods:**

1. PBMC cell counting plate: 90μL PBMC was seeded in a 96-well plate (PBMC cell density 80,000/well).
2. Treatment with test compounds: After cells being seeded, drug treatment was carried out immediately. The drugs to be tested at 5 μL/well (final concentrations 1, 0.5, 0.1, 0.05, 0.01, and 0.005 μM) were incubated at 37°C for 45 min.
3. Cytokine IL-15 stimulation: The cells were stimulated by 5 μL IL-15 , and incubated at 37°C for 30 min.
4. Protein sample collection: The cells were collected in a centrifuge tube and centrifuged for 5 minutes.The supernatant was discarded and cells were lysed with 1× Cell Extraction Buffer PTR.
5. p-STAT5 detection: Perform detection according to the requirements of the ELISA kit.

**Experimental results:** $IC_{50}$ values of compounds on p-STAT5 under IL-15 stimulation in PBMC

| Compound No. | IC50 (nM) |
|---|---|
| YZ001052 | 10.74 |
| PF-06651600 | 85.02 |

The datas in the above table show that the compounds in the disclosure have a good inhibitory effect on the JAK3-STATs signaling pathway at the cellular level, and some compounds show an $IC_{50}$ that is equivalent to or lower than that of positive drugs.

**Example 20C Immunosuppressive Effect of Compound on Delayed-Type Hypersensitivity (DTH) in Mice**

**Experimental Purpose:**

To test the immunosuppressive activity of YZ001052, YZ001054, YZ001065 and YZ001085 in SRBC mouse model. PF-06651600 was used as the positive control compound. The structures of YZ001054, YZ001065, and YZ001085 were shown in paragraph 49 of the specification of the prior application (application number CN202211019771.6).

**Experimental methods:**

Drug formulation: The compound was added to 0.5% CMC-Na solution to formulate a drug suspension (dosage is 10 mg/mL).

Induction and administration of delayed-type hypersensitivity (DTH): 30 male Balb/c mice were weighed and randomly divided into 6 groups according to body weight, with 5 mice in each group. The experimental period lasted for 7 days. On day 0, mice were sensitized by subcutaneous injection of sheep red blood cells (SRBC). The mice were performed gavage administration at 100 mg/kg (once a day) from day 0 to day 6. On day 6, the mice were injected SRBC into the right hind foot pad for challenge. Measurements were taken on the 7th day of the experiment, and the experiment was concluded.

Pharmacodynamic detection indicators of disease models: the detection and observation indicators are the redness and swelling degree and thickness of the paw pads of SRBC-induced mice. The thickness of the right paw was measured on day 6 before the SRBC injection challenge as the baseline. The thickness of the right paw was measured again at the end of the experiment, the difference in paw thickness before and after the second injection was calculated.

Statistical analysis: The experimental data were expressed as mean $\pm$ standard deviation (Mean $\pm$ SD). All data were statistically analyzed using T-test, with $P < 0.05$ considered statistically significant. The results are shown in FIG. 76.

**Experimental results:**

As can be seen from FIG. 76, the thickness of the right paw of mice increased significantly after SRBC induction (modeling group), and the thickness of the right paw of mice improved to varying degrees after drug treatment (including compounds YZ001052, YZ001054, YZ001065, and YZ001085), indicating that compounds YZ001052, YZ001054, YZ001065, and YZ001085 have significant immunosuppressive effects on delayed-type hypersensitivity reactions in mice induced by SRBC, and their effects are not inferior to PF-06651600.

**Example 20D: the immunosuppressive effects of the compounds on the collagen-induced arthritis model in mice**

**1. Construction of collagen-induced arthritis model in mice (mCIA)**

**Experimental purpose:** To test the immunosuppressive activity of YZ001052 in the mCIA model. PF-06651600 was used as a positive control compound.

**Experimental methods:** 32 female DBA/1J mice were divided randomly into 4 groups: blank control group, model group, model + YZ001052 administration group, and model + PF-06651600 administration group. After successful arthritis modeling, YZ001052 or PF-06651600 as drug intervention were gave, 50 mg/kg, once a day. Complete and incomplete Freund's adjuvants with chicken type II collagen solution were mixed in equal proportions to form emulsions. Except for the blank control group, a mixture of complete Freund's adjuvant and chicken type II collagen solution was injected subcutaneously into the base of the tail and thigh of mice on Day 0. Each mouse was injected subcutaneously with 100$\mu$L at the base of the tail, and injected 50$\mu$L subcutaneously at the base of both thighs (primary immunization). On day 21, mice were injected subcutaneously with 200$\mu$L of a mixture of incomplete Freund's adjuvant and chicken type II

collagen as a booster injection (second immunization). Arthritis was considered to occur if the clinical score of at least one limb was ≥2 points. Paw thickness measurement: Using a vernier caliper to measure the thickness of the left and right hind feet of the mouse and record it, which is the degree of paw swelling. The detection frequency was once every 3 days.

Experimental results: As can be seen from FIG. 77, after the second immunization, the inflammation in the paws of the mice in the model group continued to worsen, the swelling gradually spread to the entire paw, and the arthritis score increased significantly on the 16th day. After treatment with YZ001052 or PF-06651600, the paw swelling of CIA mice was improved to varying degrees, and the arthritis score was significantly decreased (compared with the model group).

Consistent with the arthritis scores, the paws of mice in the model group showed obvious swelling, and after treatment with YZ001052 or PF-06651600, the degree of paw swelling of mice was improved to varying degrees. The above results indicate that YZ001052 or PF-06651600 can improve the symptoms of rheumatoid arthritis.

### Example 20E: the immunosuppressive effects of the compounds on the dextran sodium sulfate (DSS)-induced mouse inflammatory bowel disease model

Experimental purpose: To test the immunosuppressive activity of YZ001052 in DSS-induced inflammatory bowel disease model in mice. PF-06651600 was used as a positive control compound.

Experimental methods: Thirty-two female C57BL/6 mice were divided randomly into 4 groups, namely, blank control group, model group, model+YZ001052 administration group, and model+PF-06651600 administration group. The mice were administered YZ001052 and PF-06651600 simultaneously at the beginning of modeling, with a dose of 50 mg/kg, once a day. 50 g of DSS was added 1000 mL of sterile water to formulate a 5% DSS solution, and the solution was filteed with a 0.22μm filter membrane. Starting from day 0, the blank group was gave drinking water without DSS, and the other experimental groups were gave drinking water containing 5% DSS. All mice were sacrificed on day 8. After the experiment, the colon was collected, photographed and measured for length.The datas were analyzed according to Statistics.

Experimental results: As shown in FIG. 78, compared with the normal control group, the colorectal length of the mice in the model group was significantly shortened, indicating that the colorectum of the mice with inflammatory bowel disease was severely damaged. Compared with the mice in the model group, the colorectal length of mice in the YZ001052-treated group was significantly increased, indicating that YZ001052 treatment can alleviate colorectal damage in mice with inflammatory bowel disease, and the results are better than PF-06651600.

### Example 20F Immunosuppressive effect of compounds on radiation-induced lung injury in mice

#### 1. Construction of mouse model of acute radiation-induced lung injury

(1) Animal grouping and drug administration

Eighteen female C57BL/6 mice were divided randomly into three groups: blank control group, simple irradiation group, and irradiation + drug YZ001052 (30 mg/kg q. d) intervention group, with 6 mice in each group.

(2) Modeling method

The mice were anesthetized with 1% sodium pentobarbital by intraperitoneal injection, and irradiated the whole lung with 22.5 Gy in a single shot using 220 KV X-rays through the Small Animal Precision Radiotherapy Research Platform (SARRP). After irradiation, the mice were housed in a normal manner.

#### 2. Index detection

Three weeks after modeling, the mice were anesthetized and dissected. The lung tissues were exposed and the left lung was ligated, an open tracheotomy was performed, the lung was lavaged with cold PBS, and the alveolar lavage fluid (BALF) was collected and centrifuged at 4°C.

The supernatant was collected to detect the content of inflammatory factor TNF-α by ELISA. As shown in FIG. 79, after irradiation induction, the TNF-α level in the bronchoalveolar lavage fluid of mice in the model group was significantly increased. The drug YZ001052 has a good inhibitory effect on the level of TNF-α.

Resuspend the bronchoalveolar lavage fluid cell pellet in 200μL PBS for white blood cell (WBC) counting. As shown in FIG. 80, after irradiation induction, the number of white blood cells in the bronchoalveolar lavage fluid of mice in the model group increased significantly. The drug YZ001052 can significantly reduce the number of inflammatory cell infiltration in the lungs of mice caused by irradiation.

## Claims

1. A crystal form of an aromatic heterocyclic compound, **characterized in that**, the structural formula of the aromatic heterocyclic compound is:

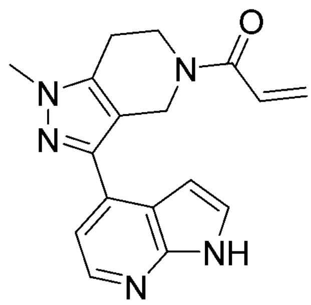

the crystal form is:

crystal form I, using X-ray diffraction, has characteristic diffraction peaks at 2θ angles of 8.4°, 10.0°, 13.6°, 16.4°, and 19.7°;
crystal form III, using X-ray diffraction, has characteristic diffraction peaks at 2θ angles of 8.2°, 9.8°, 17.2°, and 26.8°;
wherein, an error range of the 2θ angle is ±0.2°.

2. The crystal form according to claim 1, **characterized in that**:

the crystal form I, using the X-ray diffraction method, also has characteristic diffraction peaks at 2θ angles of 15.2°, 18.1°, and 24.1°;
wherein, an error range of the 2θ angle is ±0.2°.

3. A composition, **characterized in that**: the composition comprises one or both of the crystal form I and the crystal form III according to claim 1 or 2; and a percentage of the crystal form I and/or the crystal form III in the total weight of the composition is 50% or more .

4. The composition according to claim 3, **characterized in that**: the percentage of the crystal form I and/or the crystal form III in the total weight of the composition is 80% or more.

5. The composition according to claim 3, **characterized in that**: the percentage of the crystal form I and/or the crystal form III in the total weight of the composition is 90% or more.

6. A pharmaceutical composition, comprising one or both of the crystal form I and the crystal form III according to claim 1 or 2, and a pharmaceutically acceptable carrier or excipient.

7. A method for producing the crystal form I of claim 1 or 2, **characterized in that**: adding a solvent to the aromatic heterocyclic compound to form a suspension, stirring at room temperature, filtering, and drying to obtain the crystal form I; the solvent is one or a combination of two or more of acetone, methanol, ethanol, water, ethyl acetate, toluene, methyl tert-butyl ether, and n-heptane;
alternatively, adding a first organic solvent to the aromatic heterocyclic compound, heating to dissolve, and then adding a second organic solvent; stirring to crystallize; filtering, and drying to obtain the crystal form I; the first organic solvent is one or a combination of two or more of methanol, ethanol, dichloromethane, chloroform, and dimethyl sulfoxide; the second organic solvent is one or a combination of two or more of isopropyl ether, n-hexane, n-heptane, methyl tert-butyl ether, and water.

8. A method for producing the crystal form III according to claim 1, **characterized in that**: adding a first organic solvent to the aromatic heterocyclic compound, heating to dissolve, and then adding a second organic solvent; then stirring to crystallize; then filtering and drying; the first organic solvent is one or a combination of two or more of methanol, ethanol, tetrahydrofuran, 1,4-dioxane, and acetonitrile, and the second organic solvent is one or a combination of two or more of water and ethyl acetate.

9. An use of the crystal form according to claim 1 or 2 in the production of a drug for preventing or treating a disease caused by an abnormal JAK-STAT signaling pathway.

10. The use according to claim 9, **characterized in that**: the abnormal JAK-STAT signaling pathway refers to overactivation or overexpression of JAK3 kinase.

11. The use according to claim 9, **characterized in that**: the disease is selected from one or more of autoimmune diseases, cancer, and bone marrow proliferation diseases.

12. The use according to claim 11, **characterized in that**: the autoimmune disease is selected from one or more of alopecia areata, lupus, multiple sclerosis, amyotrophic lateral sclerosis, rheumatoid arthritis, rheumatoid arthritis, psoriasis, complications caused by organ transplantation, atopic dermatitis, autoimmune thyroid disease, ulcerative colitis, Crohn's disease, Sjögren's syndrome, vitiligo, autoimmune kidney damage, autoimmune liver damage, and chronic obstructive pulmonary disease; the cancer is selected from one or more of colon cancer, gastric adenocarcinoma, bladder cancer, breast cancer, kidney cancer, liver cancer, lung cancer, thyroid cancer, head and neck cancer, prostate cancer, pancreatic cancer, CNS (central nervous system) cancer, and malignant glioma; the bone marrow proliferation disease is selected from one or more of chronic myelomonocytic leukemia, atypical chronic myelocytic leukemia and juvenile myelomonocytic leukemia.

13. The use according to claim 11, **characterized in that**: the autoimmune disease is radiation lung injury, and the lung injury refers to acute radiation pneumonitis and radiation pulmonary fibrosis.

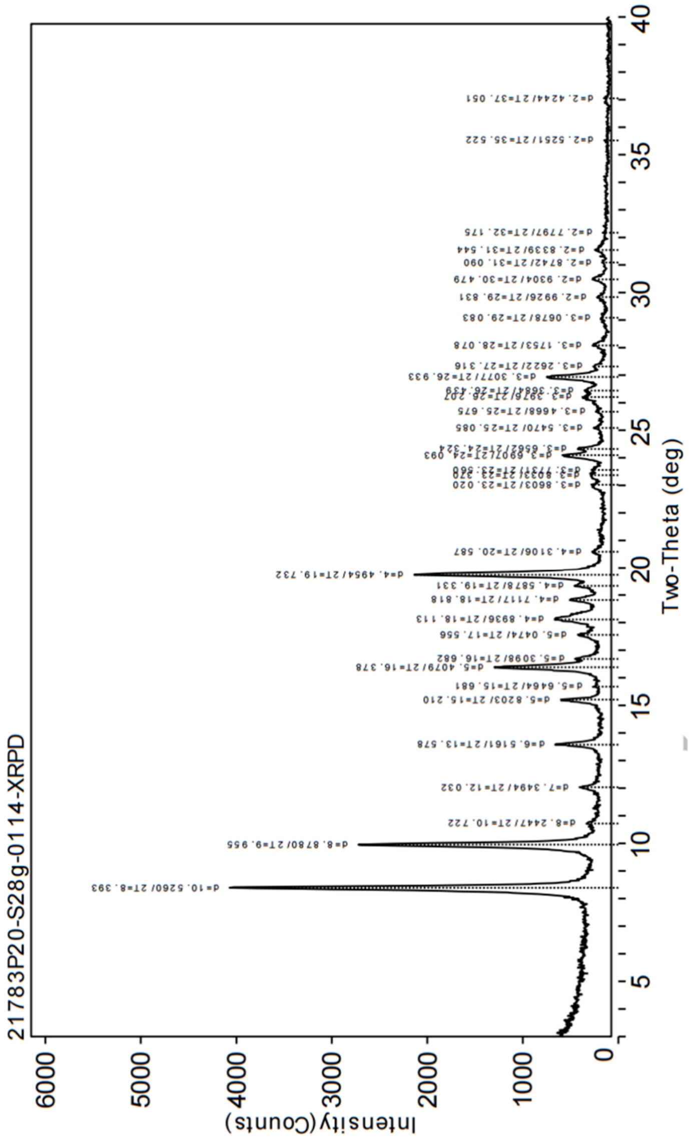


FIG.1

| 21783P20-S28g-0114-XRPD | | | | | |
|---|---|---|---|---|---|
| 2-Theta | d | Height | I% | Area | I% |
| 8.393 | 10.5260 | 3736 | 100 | 40605 | 100 |
| 9.955 | 8.8780 | 2455 | 65.7 | 27082 | 66.7 |
| 10.722 | 8.2447 | 91 | 2.4 | 817 | 2 |
| 12.032 | 7.3494 | 210 | 5.6 | 2574 | 6.3 |
| 13.578 | 6.5161 | 470 | 12.6 | 5031 | 12.4 |
| 15.210 | 5.8203 | 409 | 10.9 | 4331 | 10.7 |
| 15.681 | 5.6464 | 56 | 1.5 | 242 | 0.6 |
| 16.378 | 5.4079 | 1069 | 28.6 | 13890 | 34.2 |
| 16.682 | 5.3098 | 173 | 4.6 | 1438 | 3.5 |
| 17.556 | 5.0474 | 169 | 4.5 | 1484 | 3.7 |
| 18.113 | 4.8936 | 389 | 10.4 | 5898 | 14.5 |
| 18.818 | 4.7117 | 247 | 6.6 | 2193 | 5.4 |
| 19.331 | 4.5878 | 129 | 3.5 | 755 | 1.9 |
| 19.732 | 4.4954 | 1897 | 50.8 | 22310 | 54.9 |
| 20.587 | 4.3106 | 84 | 2.2 | 632 | 1.6 |
| 23.020 | 3.8603 | 84 | 2.2 | 865 | 2.1 |
| 23.370 | 3.8033 | 85 | 2.3 | 1645 | 4.1 |
| 23.560 | 3.7731 | 82 | 2.2 | 1639 | 4 |
| 24.093 | 3.6907 | 402 | 10.8 | 6618 | 16.3 |
| 24.324 | 3.6562 | 246 | 6.6 | 4449 | 11 |
| 25.085 | 3.5470 | 89 | 2.4 | 864 | 2.1 |
| 25.675 | 3.4668 | 48 | 1.3 | 213 | 0.5 |
| 26.207 | 3.3976 | 158 | 4.2 | 3071 | 7.6 |
| 26.439 | 3.3684 | 115 | 3.1 | 2384 | 5.9 |
| 26.933 | 3.3077 | 529 | 14.2 | 6343 | 15.6 |
| 27.316 | 3.2622 | 55 | 1.5 | 200 | 0.5 |
| 28.078 | 3.1753 | 138 | 3.7 | 1855 | 4.6 |
| 29.083 | 3.0678 | 61 | 1.6 | 1231 | 3 |
| 29.831 | 2.9926 | 95 | 2.5 | 1320 | 3.3 |
| 30.479 | 2.9304 | 141 | 3.8 | 1752 | 4.3 |
| 31.090 | 2.8742 | 42 | 1.1 | 311 | 0.8 |
| 31.544 | 2.8339 | 115 | 3.1 | 1730 | 4.3 |
| 32.175 | 2.7797 | 38 | 1 | 390 | 1 |
| 35.522 | 2.5251 | 46 | 1.2 | 563 | 1.4 |
| 37.051 | 2.4244 | 59 | 1.6 | 832 | 2 |

FIG.2

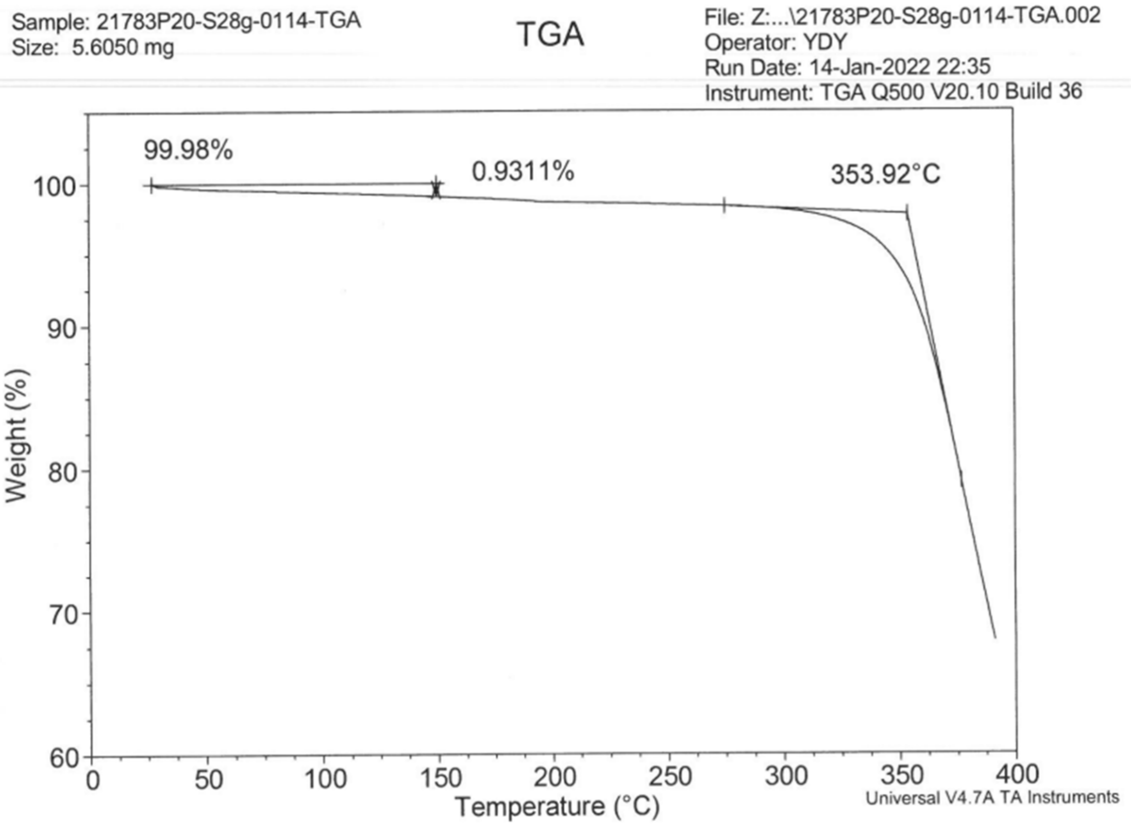
Sample: 21783P20-S28g-0114-TGA
Size: 5.6050 mg
TGA
File: Z:...\21783P20-S28g-0114-TGA.002
Operator: YDY
Run Date: 14-Jan-2022 22:35
Instrument: TGA Q500 V20.10 Build 36
99.98%
0.9311%
353.92°C
Weight (%)
Temperature (°C)
Universal V4.7A TA Instruments

FIG.3

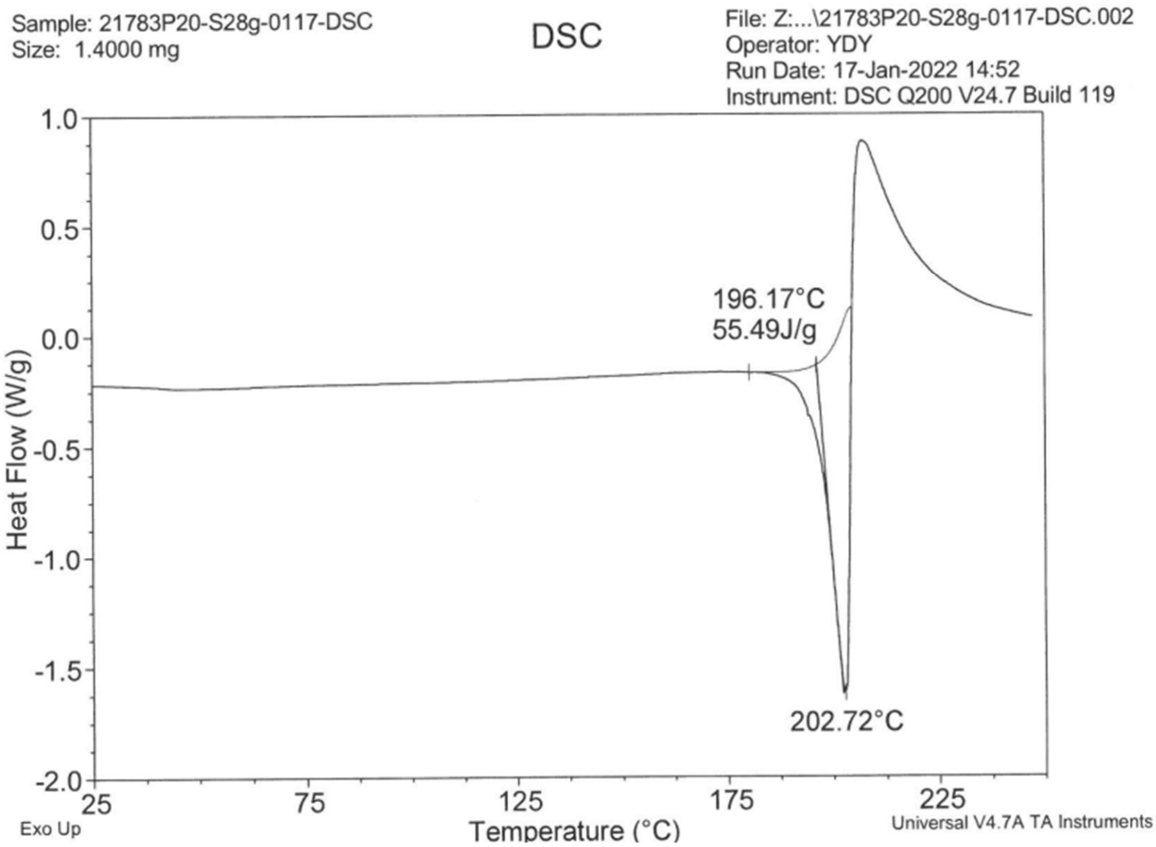
Sample: 21783P20-S28g-0117-DSC
Size: 1.4000 mg
DSC
File: Z:...\21783P20-S28g-0117-DSC.002
Operator: YDY
Run Date: 17-Jan-2022 14:52
Instrument: DSC Q200 V24.7 Build 119
196.17°C
55.49J/g
202.72°C
Heat Flow (W/g)
Exo Up
Temperature (°C)
Universal V4.7A TA Instruments

FIG.4

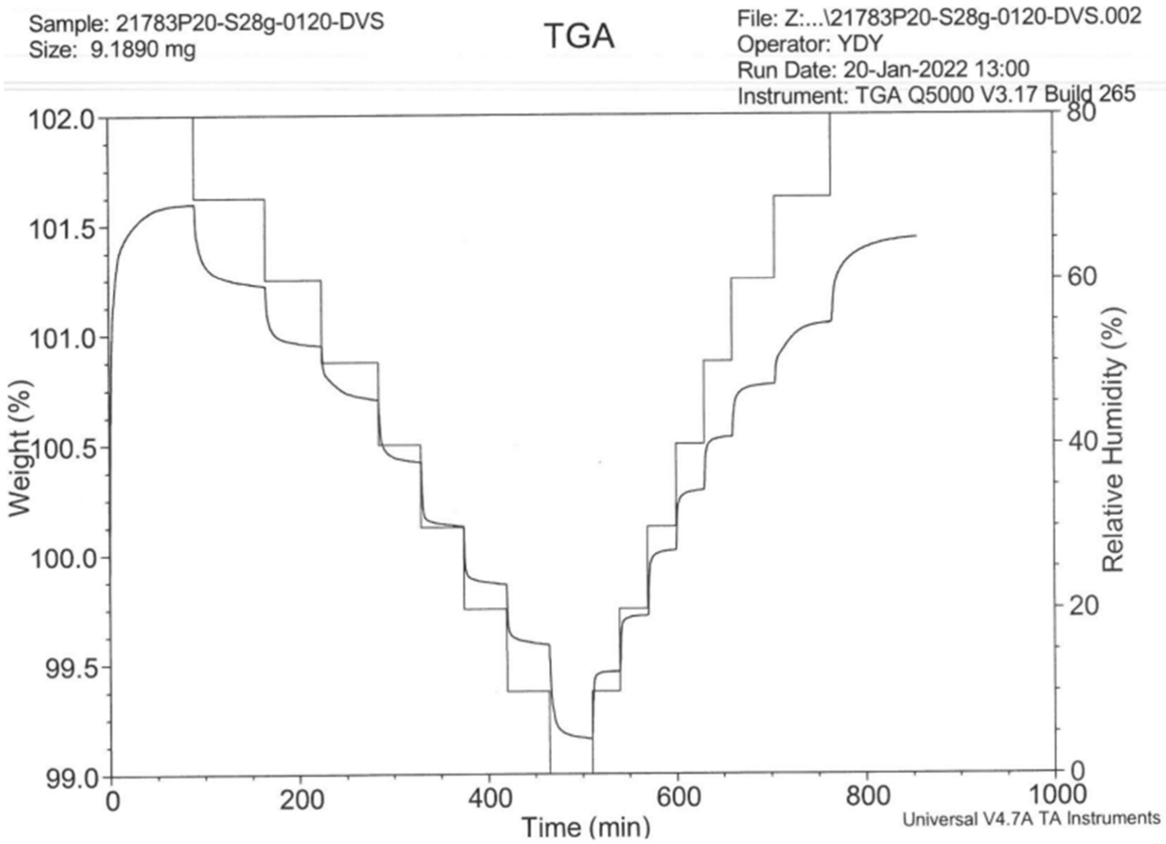
Sample: 21783P20-S28g-0120-DVS
Size: 9.1890 mg
TGA
File: Z:...\21783P20-S28g-0120-DVS.002
Operator: YDY
Run Date: 20-Jan-2022 13:00
Instrument: TGA Q5000 V3.17 Build 265
Weight (%)
Relative Humidity (%)
Time (min)
Universal V4.7A TA Instruments

FIG.5

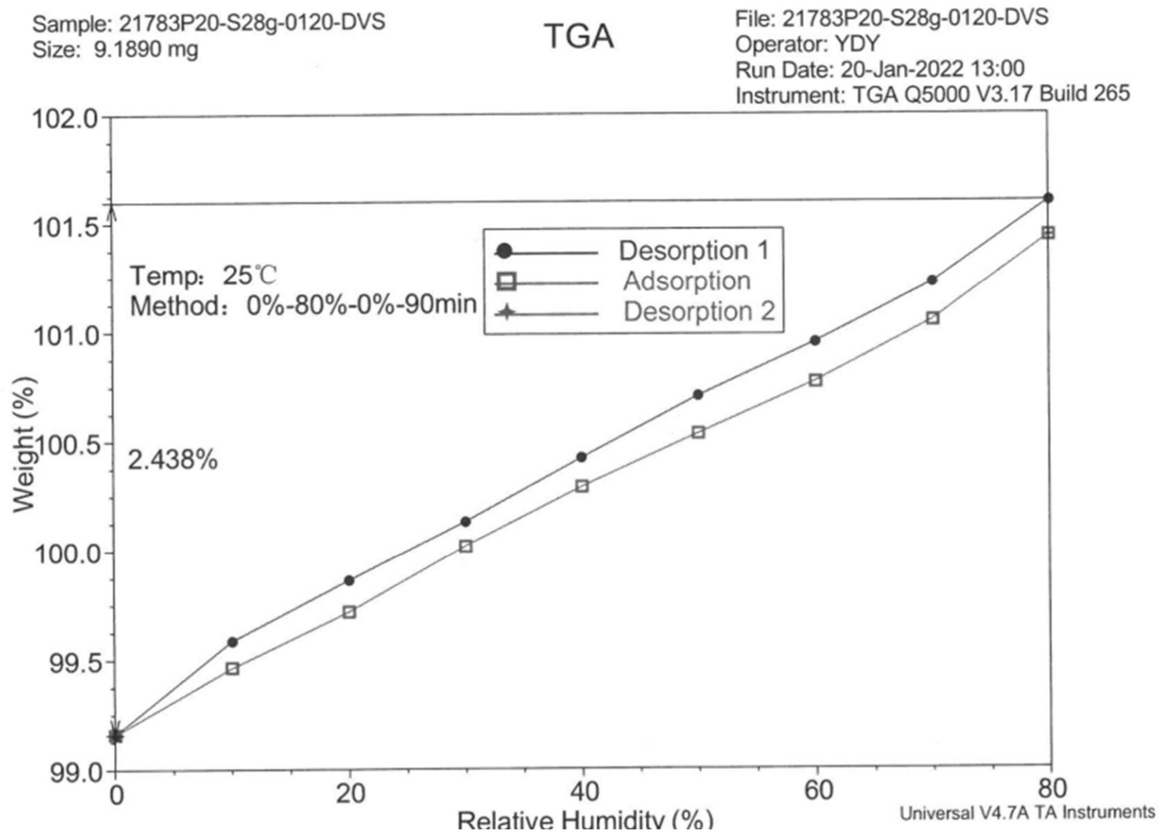
Sample: 21783P20-S28g-0120-DVS
Size: 9.1890 mg
TGA
File: 21783P20-S28g-0120-DVS
Operator: YDY
Run Date: 20-Jan-2022 13:00
Instrument: TGA Q5000 V3.17 Build 265
Desorption 1
Adsorption
Desorption 2
Temp: 25℃
Method: 0%-80%-0%-90min
2.438%
Weight (%)
Relative Humidity (%)
Universal V4.7A TA Instruments

FIG.6

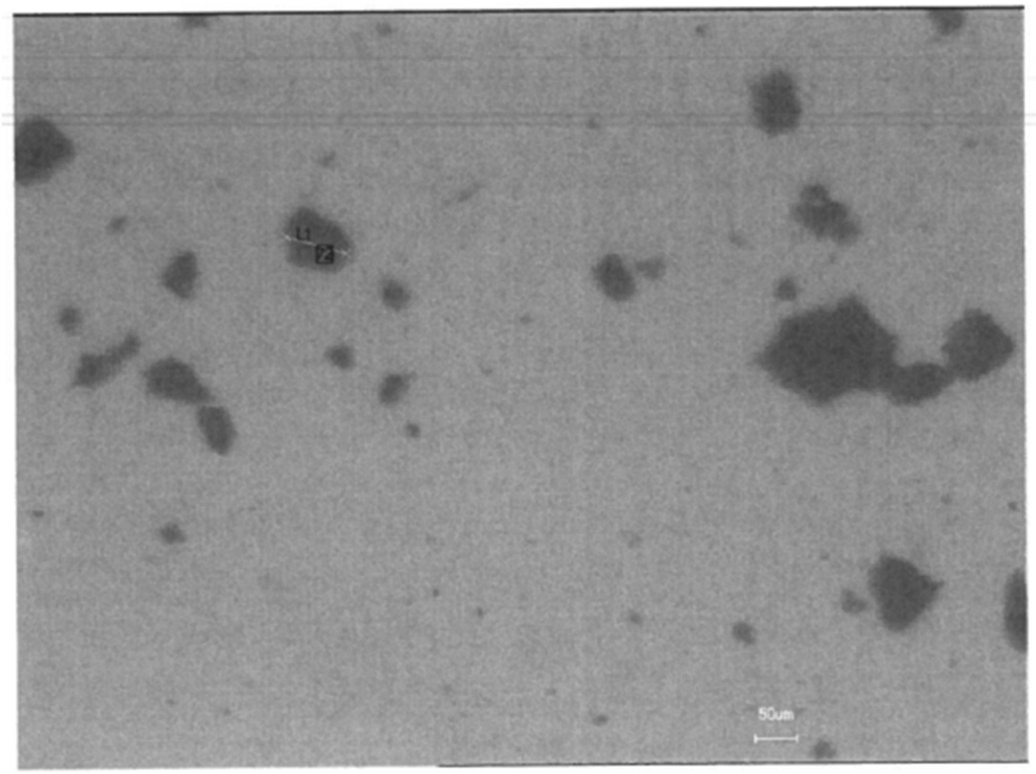


FIG.7

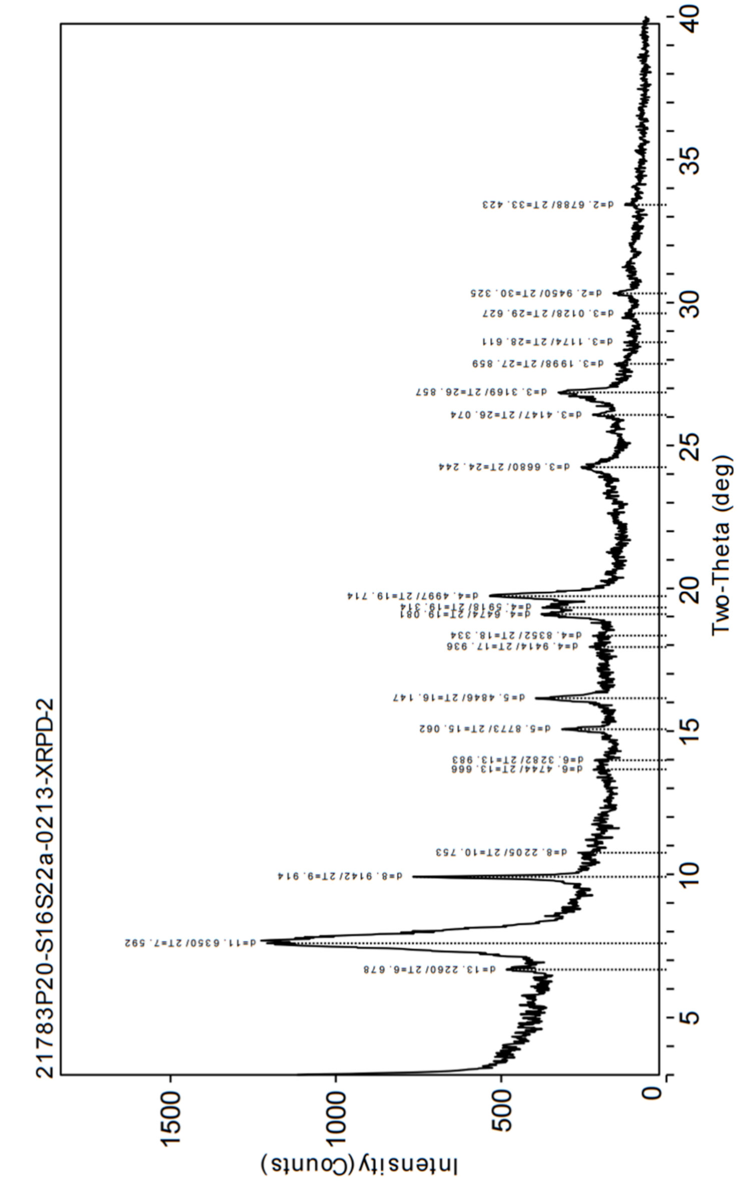
21783P20-S16S22a-0213-XRPD-2
Intensity(Counts)
Two-Theta (deg)
d=13.2260/2T=6.678
d=11.6350/2T=7.592
d=8.9142/2T=9.914
d=8.2205/2T=10.753
d=6.4744/2T=13.666
d=6.3282/2T=13.983
d=5.8773/2T=15.062
d=5.4846/2T=16.147
d=4.9414/2T=17.936
d=4.8352/2T=18.334
d=4.6474/2T=19.081
d=4.5918/2T=19.314
d=4.4997/2T=19.714
d=3.6680/2T=24.244
d=3.4147/2T=26.074
d=3.3169/2T=26.857
d=3.1998/2T=27.859
d=3.1174/2T=28.611
d=3.0128/2T=29.627
d=2.9450/2T=30.325
d=2.6788/2T=33.423

FIG.8

| 21783P20-S16S22a-0213-XRPD-2 | | | | | |
|---|---|---|---|---|---|
| 2-Theta | d | Height | I% | Area | I% |
| 6.678 | 13.2260 | 112 | 16.2 | 1018 | 5.7 |
| 7.592 | 11.6350 | 691 | 100 | 18005 | 100 |
| 9.914 | 8.9142 | 536 | 77.6 | 4579 | 25.4 |
| 10.753 | 8.2205 | 60 | 8.7 | 871 | 4.8 |
| 13.666 | 6.4744 | 52 | 7.5 | 813 | 4.5 |
| 13.983 | 6.3282 | 53 | 7.7 | 886 | 4.9 |
| 15.062 | 5.8773 | 148 | 21.4 | 1368 | 7.6 |
| 16.147 | 5.4846 | 223 | 32.3 | 2676 | 14.9 |
| 17.936 | 4.9414 | 52 | 7.5 | 535 | 3 |
| 18.334 | 4.8352 | 51 | 7.4 | 836 | 4.6 |
| 19.081 | 4.6474 | 147 | 21.3 | 2202 | 12.2 |
| 19.314 | 4.5918 | 118 | 17.1 | 2268 | 12.6 |
| 19.714 | 4.4997 | 283 | 41 | 3396 | 18.9 |
| 24.244 | 3.6680 | 102 | 14.8 | 1843 | 10.2 |
| 26.074 | 3.4147 | 65 | 9.4 | 407 | 2.3 |
| 26.857 | 3.3169 | 170 | 24.6 | 2745 | 15.2 |
| 27.859 | 3.1998 | 43 | 6.2 | 387 | 2.1 |
| 28.611 | 3.1174 | 37 | 5.4 | 183 | 1 |
| 29.627 | 3.0128 | 36 | 5.2 | 509 | 2.8 |
| 30.325 | 2.9450 | 68 | 9.8 | 668 | 3.7 |
| 33.423 | 2.6788 | 47 | 6.8 | 463 | 2.6 |

FIG.9

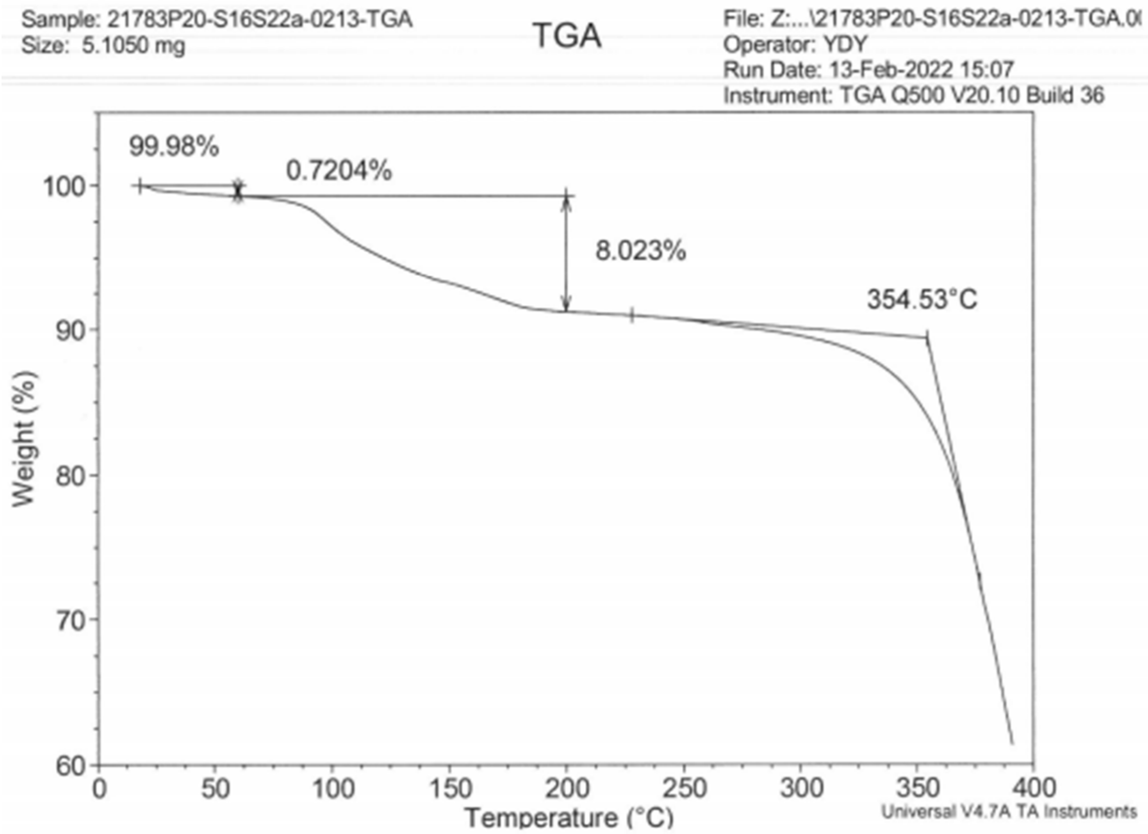
Sample: 21783P20-S16S22a-0213-TGA
Size: 5.1050 mg
TGA
File: Z:...\21783P20-S16S22a-0213-TGA.0(
Operator: YDY
Run Date: 13-Feb-2022 15:07
Instrument: TGA Q500 V20.10 Build 36
99.98%
0.7204%
8.023%
354.53°C
Weight (%)
Temperature (°C)
Universal V4.7A TA Instruments

FIG.10

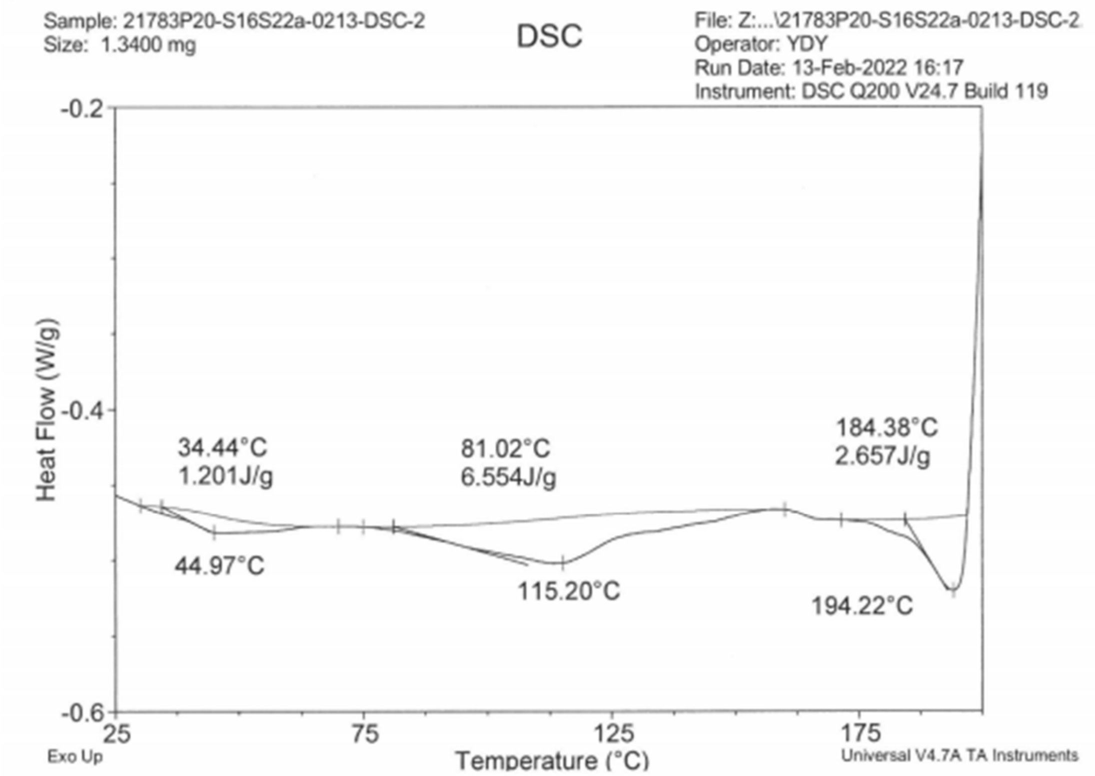
Sample: 21783P20-S16S22a-0213-DSC-2
Size: 1.3400 mg
DSC
File: Z:...\21783P20-S16S22a-0213-DSC-2.
Operator: YDY
Run Date: 13-Feb-2022 16:17
Instrument: DSC Q200 V24.7 Build 119
34.44°C
1.201J/g
44.97°C
81.02°C
6.554J/g
115.20°C
184.38°C
2.657J/g
194.22°C
Heat Flow (W/g)
Exo Up
Temperature (°C)
Universal V4.7A TA Instruments

FIG.11

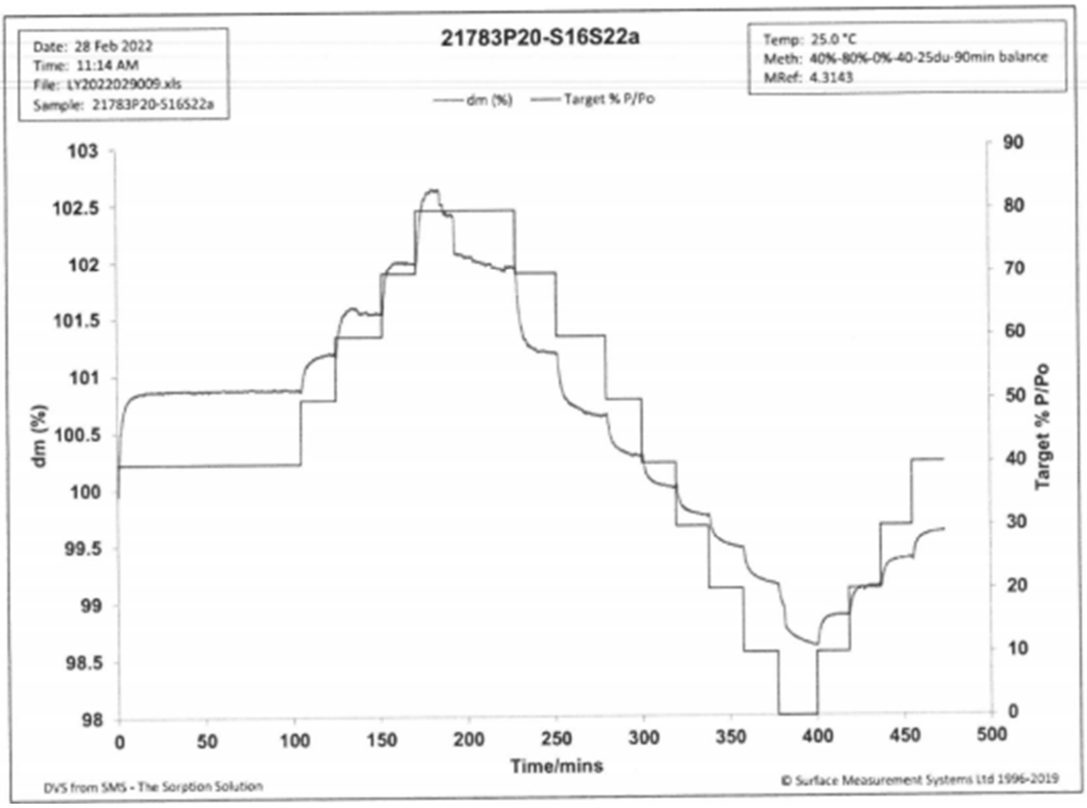
21783P20-S16S22a
Date: 28 Feb 2022
Time: 11:14 AM
File: LY2022029009.xls
Sample: 21783P20-S16S22a
Temp: 25.0 °C
Meth: 40%-80%-0%-40-25du-90min balance
MRef: 4.3143
dm (%)
Target % P/Po
Time/mins
DVS from SMS - The Sorption Solution
© Surface Measurement Systems Ltd 1996-2019

FIG.12

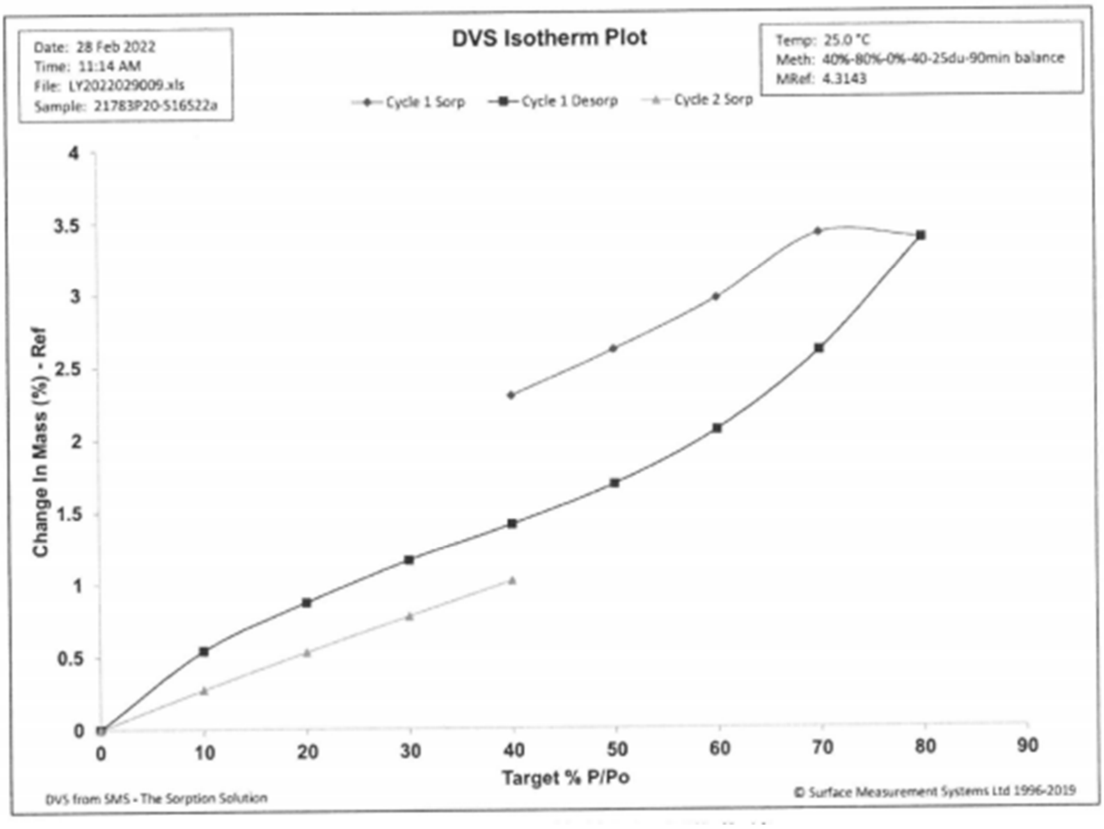
DVS Isotherm Plot
Date: 28 Feb 2022
Time: 11:14 AM
File: LY2022029009.xls
Sample: 21783P20-S16S22a
Temp: 25.0 °C
Meth: 40%-80%-0%-40-25du-90min balance
MRef: 4.3143
Cycle 1 Sorp
Cycle 1 Desorp
Cycle 2 Sorp
Change In Mass (%) - Ref
Target % P/Po
DVS from SMS - The Sorption Solution
© Surface Measurement Systems Ltd 1996-2019

FIG.13

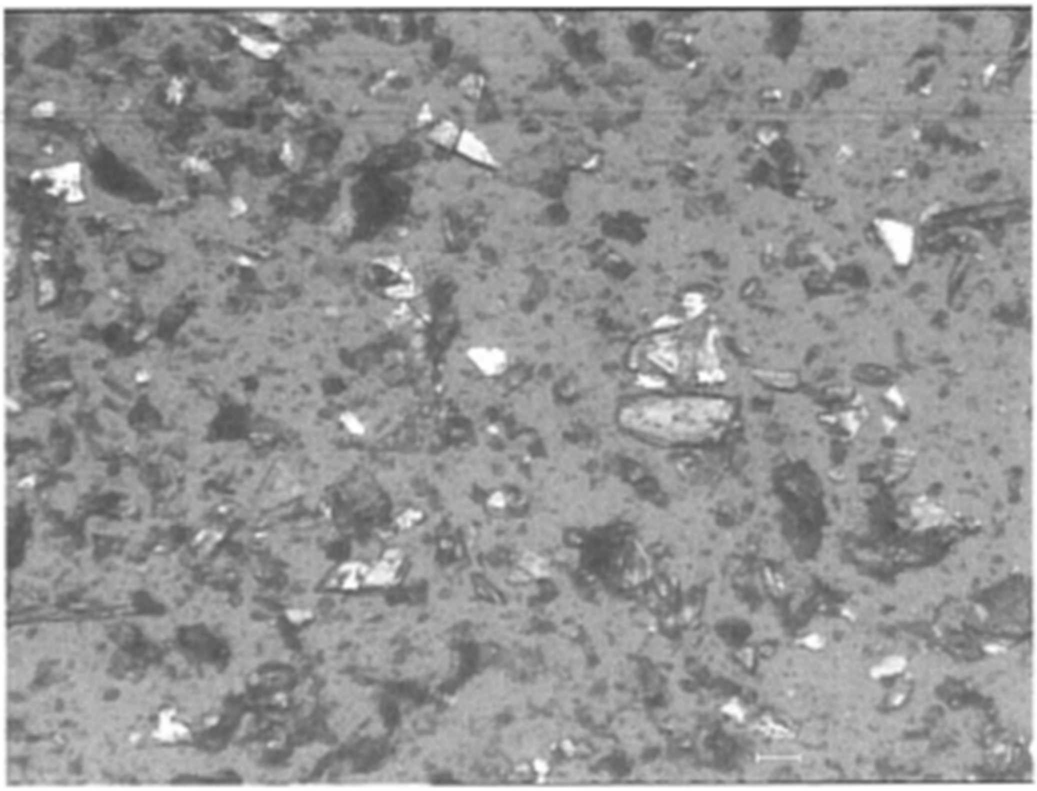

FIG.14

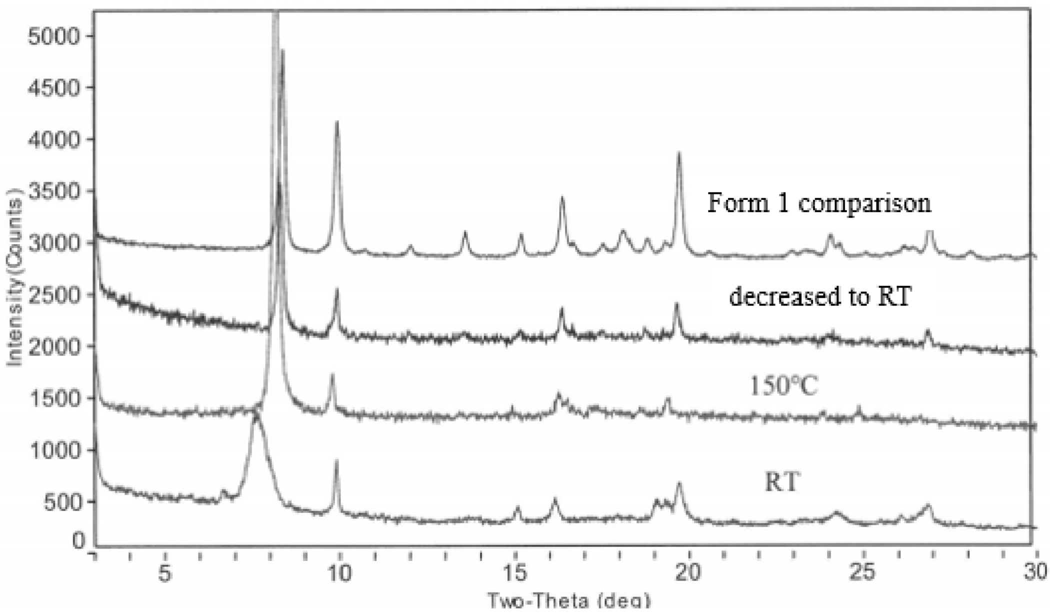
5000
4500
4000
3500
3000
2500
2000
1500
1000
500
0
Intensity(Counts)
Form 1 comparison
decreased to RT
150°C
RT
5
10
15
20
25
30
Two-Theta (deg)

FIG.15

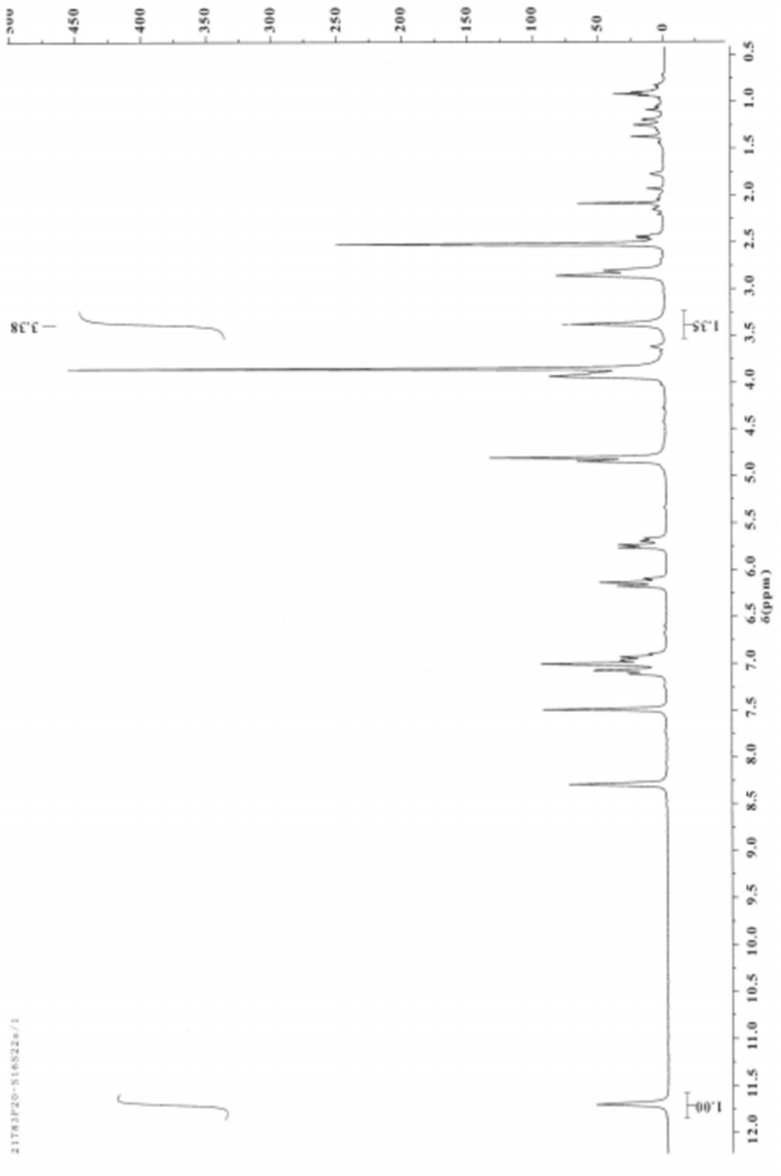


FIG.16

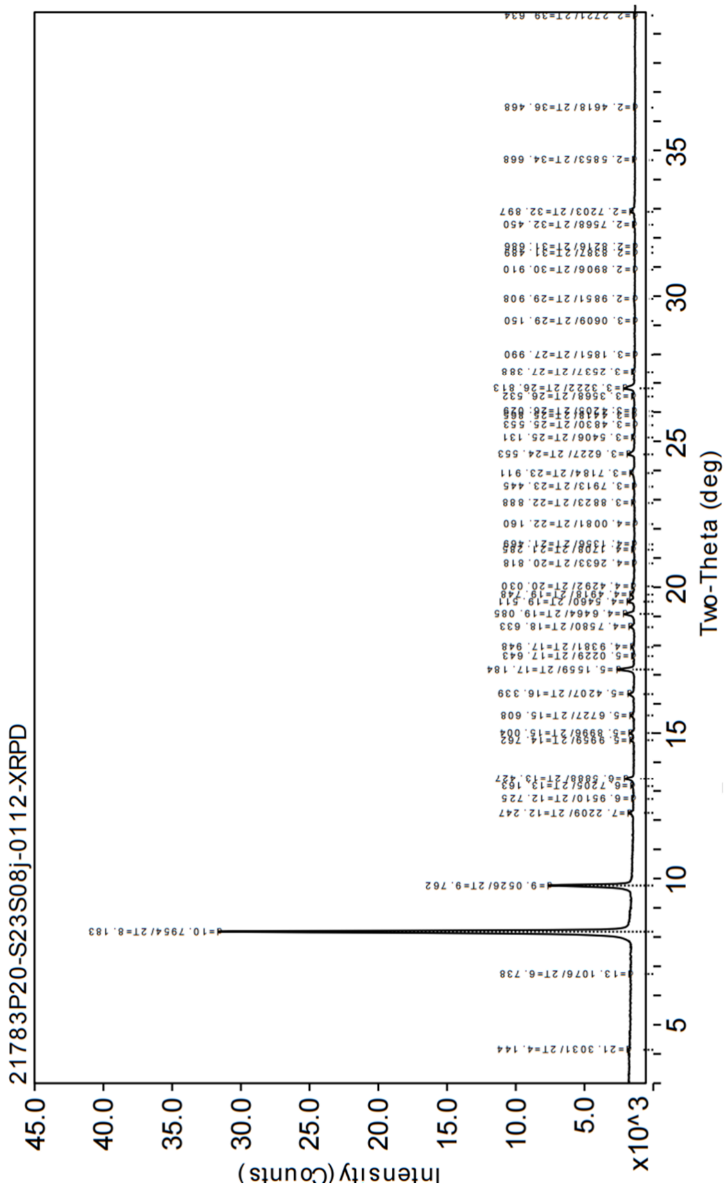

FIG.17

| 21783P20-S23S08j-0112-XRPD | | | | | |
|---|---|---|---|---|---|
| 2-Theta | d | Height | I% | Area | I% |
| 4.144 | 21.3031 | 131 | 0.4 | 318 | 0.2 |
| 6.738 | 13.1076 | 110 | 0.4 | 1418 | 0.9 |
| 8.183 | 10.7954 | 30156 | 100 | 154621 | 100 |
| 9.762 | 9.0526 | 6101 | 20.2 | 31543 | 20.4 |
| 12.247 | 7.2209 | 328 | 1.1 | 1641 | 1.1 |
| 12.725 | 6.9510 | 99 | 0.3 | 440 | 0.3 |
| 13.163 | 6.7205 | 129 | 0.4 | 820 | 0.5 |
| 13.427 | 6.5888 | 625 | 2.1 | 3595 | 2.3 |
| 14.762 | 5.9959 | 226 | 0.7 | 1759 | 1.1 |
| 15.004 | 5.8996 | 292 | 1 | 1738 | 1.1 |
| 15.608 | 5.6727 | 231 | 0.8 | 1440 | 0.9 |
| 16.339 | 5.4207 | 423 | 1.4 | 2768 | 1.8 |
| 17.184 | 5.1559 | 1236 | 4.1 | 6525 | 4.2 |
| 17.643 | 5.0229 | 124 | 0.4 | 969 | 0.6 |
| 17.948 | 4.9381 | 175 | 0.6 | 1425 | 0.9 |
| 18.633 | 4.7580 | 295 | 1 | 1543 | 1 |
| 19.085 | 4.6464 | 704 | 2.3 | 3981 | 2.6 |
| 19.511 | 4.5460 | 457 | 1.5 | 2744 | 1.8 |
| 19.748 | 4.4918 | 249 | 0.8 | 1487 | 1 |
| 20.030 | 4.4292 | 130 | 0.4 | 1033 | 0.7 |
| 20.818 | 4.2633 | 74 | 0.2 | 511 | 0.3 |
| 21.285 | 4.1708 | 148 | 0.5 | 1296 | 0.8 |
| 21.469 | 4.1356 | 71 | 0.2 | 1048 | 0.7 |
| 22.160 | 4.0081 | 53 | 0.2 | 395 | 0.3 |
| 22.888 | 3.8823 | 126 | 0.4 | 876 | 0.6 |
| 23.445 | 3.7913 | 67 | 0.2 | 433 | 0.3 |
| 23.911 | 3.7184 | 304 | 1 | 2385 | 1.5 |
| 24.553 | 3.6227 | 478 | 1.6 | 3203 | 2.1 |
| 25.131 | 3.5406 | 160 | 0.5 | 966 | 0.6 |
| 25.553 | 3.4830 | 48 | 0.2 | 252 | 0.2 |
| 25.865 | 3.4418 | 63 | 0.2 | 1194 | 0.8 |
| 26.029 | 3.4205 | 60 | 0.2 | 807 | 0.5 |
| 26.532 | 3.3568 | 150 | 0.5 | 1101 | 0.7 |
| 26.813 | 3.3222 | 763 | 2.5 | 4911 | 3.2 |
| 27.388 | 3.2537 | 176 | 0.6 | 1286 | 0.8 |
| 27.990 | 3.1851 | 47 | 0.2 | 515 | 0.3 |
| 29.150 | 3.0609 | 37 | 0.1 | 251 | 0.2 |
| 29.908 | 2.9851 | 81 | 0.3 | 821 | 0.5 |
| 30.910 | 2.8906 | 68 | 0.2 | 535 | 0.3 |
| 31.489 | 2.8387 | 41 | 0.1 | 523 | 0.3 |
| 31.686 | 2.8216 | 51 | 0.2 | 647 | 0.4 |
| 32.450 | 2.7568 | 93 | 0.3 | 596 | 0.4 |
| 32.897 | 2.7203 | 305 | 1 | 2203 | 1.4 |
| 34.668 | 2.5853 | 106 | 0.4 | 729 | 0.5 |
| 36.468 | 2.4618 | 62 | 0.2 | 601 | 0.4 |
| 39.634 | 2.2721 | 55 | 0.2 | 464 | 0.3 |

FIG.18

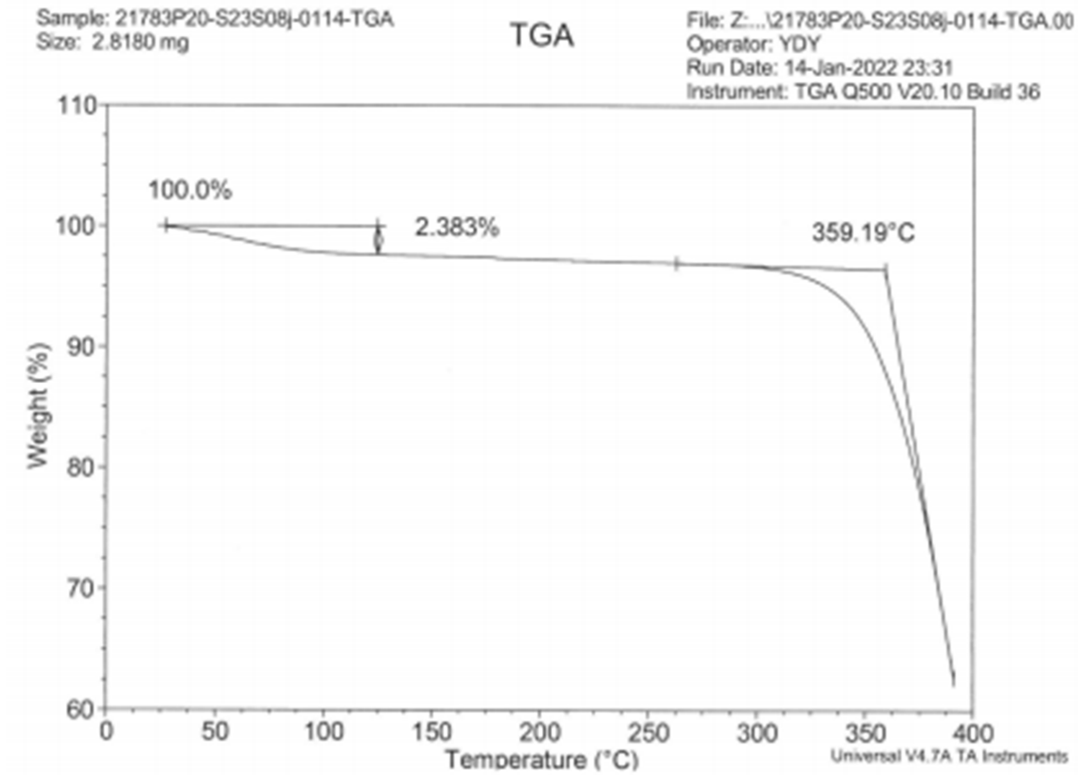
Sample: 21783P20-S23S08j-0114-TGA
Size: 2.8180 mg
TGA
File: Z:...\21783P20-S23S08j-0114-TGA.00
Operator: YDY
Run Date: 14-Jan-2022 23:31
Instrument: TGA Q500 V20.10 Build 36
100.0%
2.383%
359.19°C
Weight (%)
Temperature (°C)
Universal V4.7A TA Instruments

FIG.19

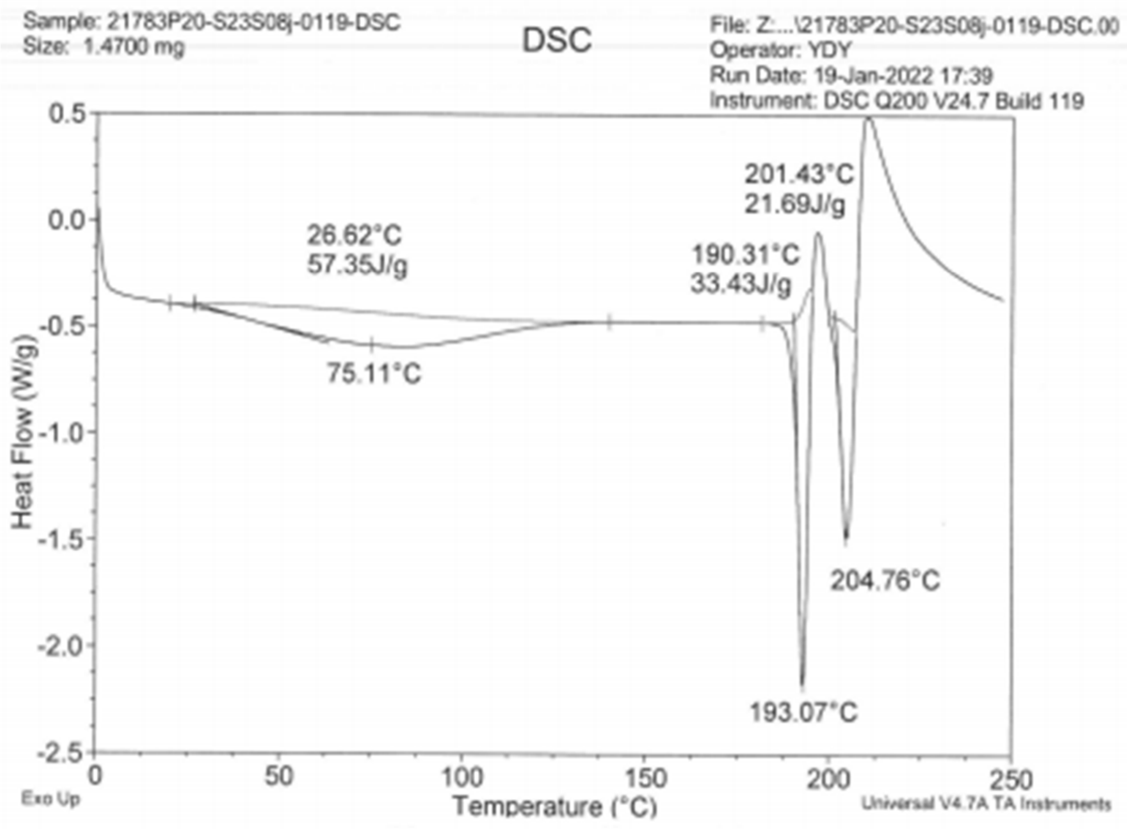
Sample: 21783P20-S23S08j-0119-DSC
Size: 1.4700 mg
DSC
File: Z:...\21783P20-S23S08j-0119-DSC.00
Operator: YDY
Run Date: 19-Jan-2022 17:39
Instrument: DSC Q200 V24.7 Build 119
26.62°C
57.35J/g
75.11°C
201.43°C
21.69J/g
190.31°C
33.43J/g
204.76°C
193.07°C
Heat Flow (W/g)
Temperature (°C)
Exo Up
Universal V4.7A TA Instruments

FIG.20

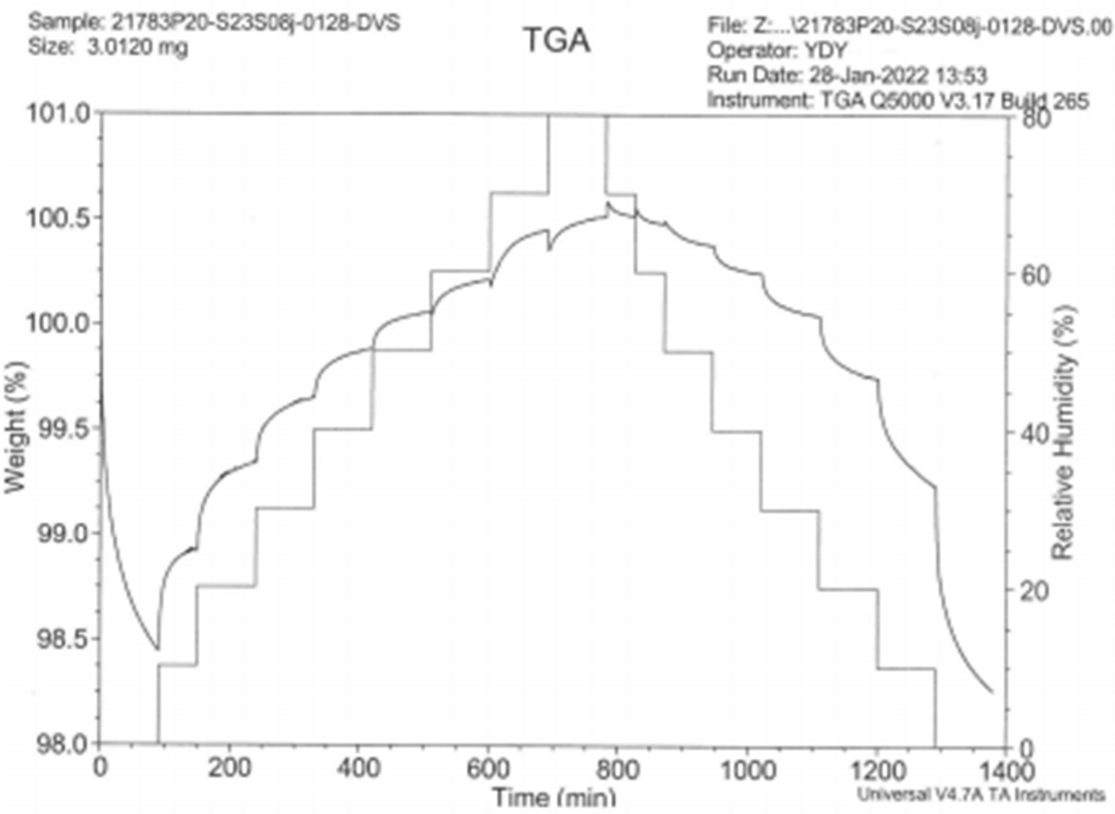
Sample: 21783P20-S23S08j-0128-DVS
Size: 3.0120 mg
TGA
File: Z:...\21783P20-S23S08j-0128-DVS.00
Operator: YDY
Run Date: 28-Jan-2022 13:53
Instrument: TGA Q5000 V3.17 Build 265
Weight (%)
Relative Humidity (%)
Time (min)
Universal V4.7A TA Instruments

FIG.21

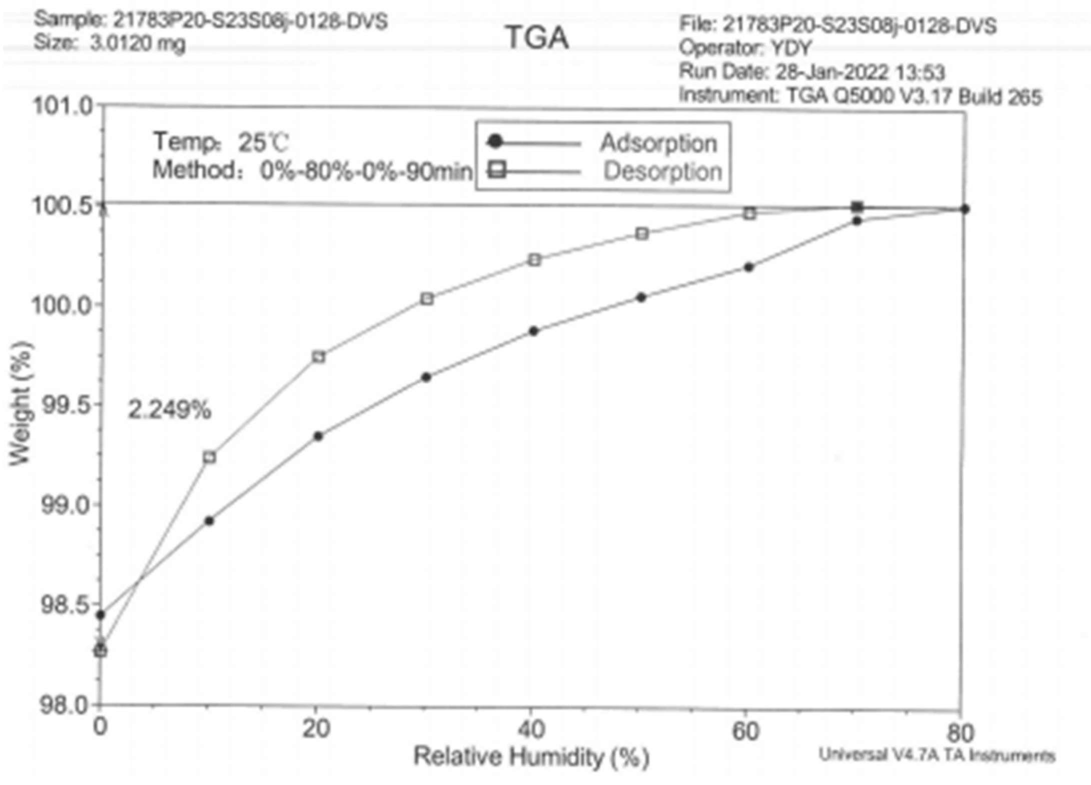
Sample: 21783P20-S23S08j-0128-DVS
Size: 3.0120 mg
TGA
File: 21783P20-S23S08j-0128-DVS
Operator: YDY
Run Date: 28-Jan-2022 13:53
Instrument: TGA Q5000 V3.17 Build 265
Temp: 25℃
Method: 0%-80%-0%-90min
Adsorption
Desorption
2.249%
Weight (%)
Relative Humidity (%)
Universal V4.7A TA Instruments

FIG.22

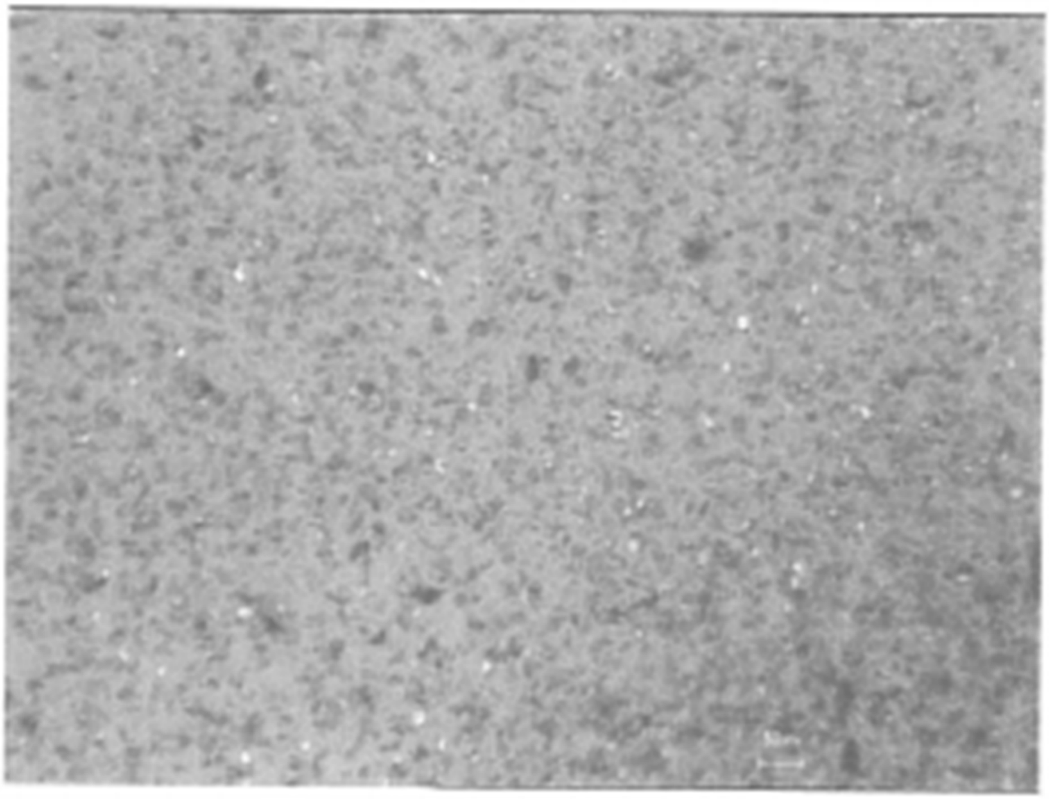

FIG.23

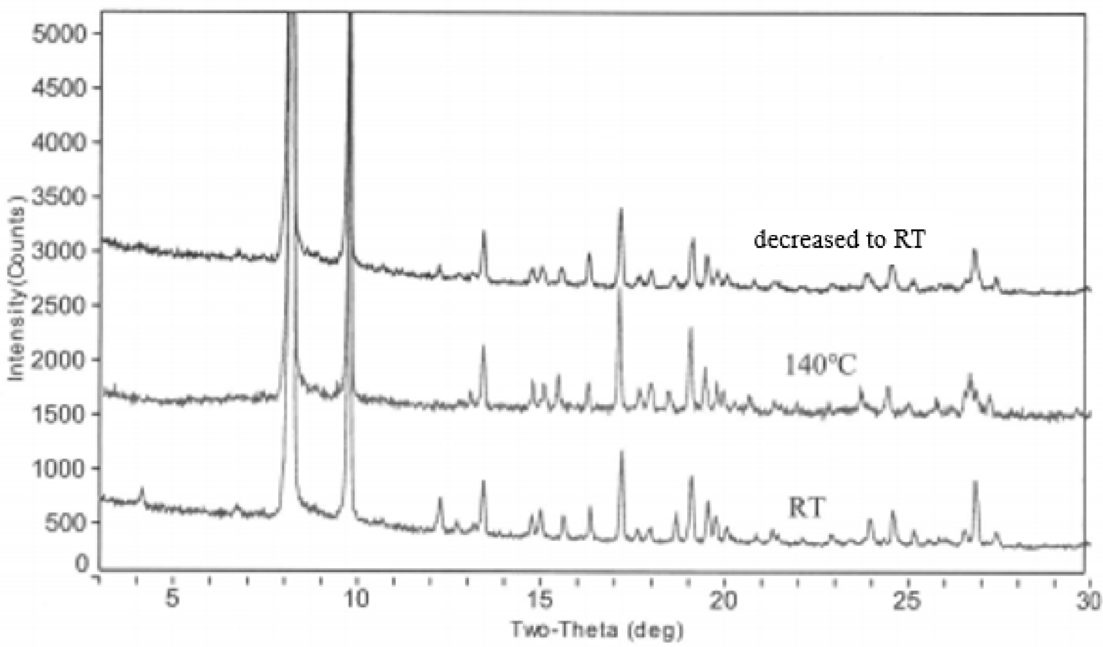
5000
4500
4000
3500
3000
2500
2000
1500
1000
500
0
Intensity(Counts)
decreased to RT
140°C
RT
5
10
15
20
25
30
Two-Theta (deg)

FIG.24

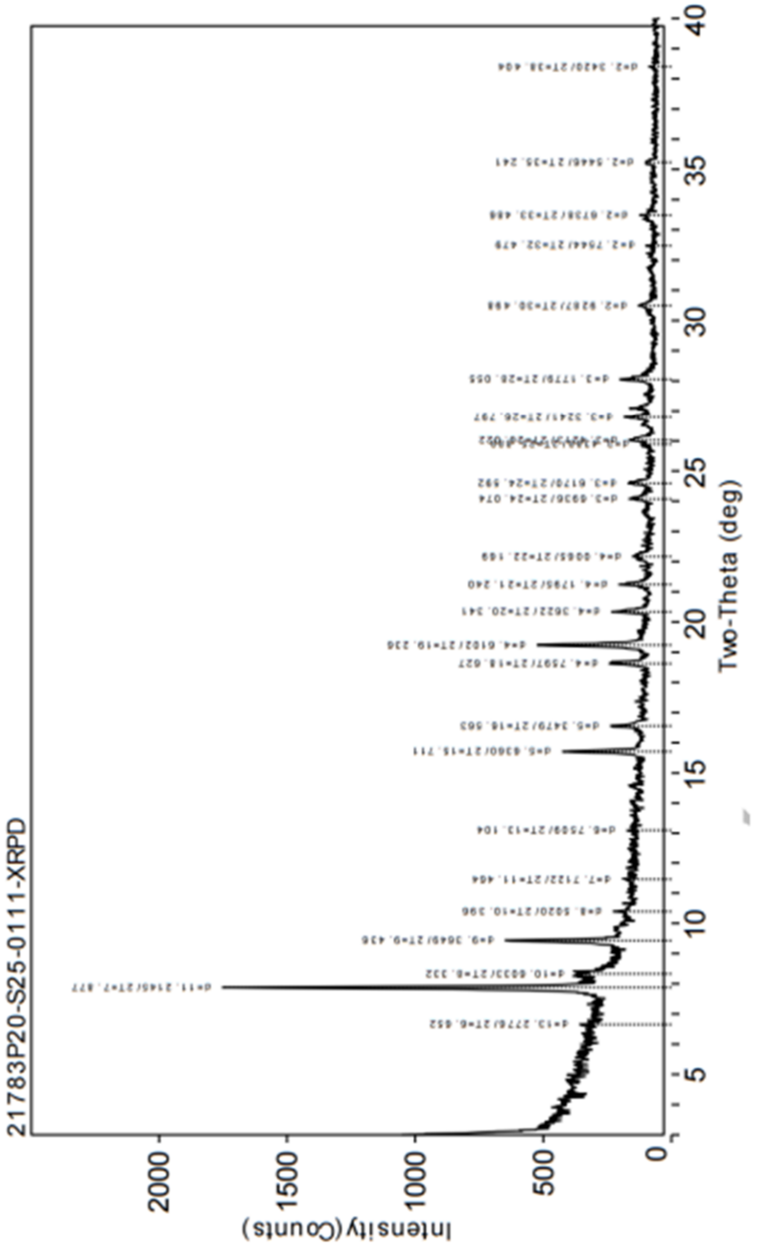


FIG.25

| 21783P20-S25-0111-XRPD | | | | | |
|---|---|---|---|---|---|
| 2-Theta | d | Height | I% | Area | I% |
| 6.652 | 13.2776 | 65 | 4.5 | 688 | 8.3 |
| 7.877 | 11.2145 | 1451 | 100 | 8259 | 100 |
| 8.332 | 10.6033 | 80 | 5.5 | 661 | 8 |
| 9.436 | 9.3649 | 457 | 31.5 | 3473 | 42.1 |
| 10.396 | 8.5020 | 70 | 4.8 | 547 | 6.6 |
| 11.464 | 7.7122 | 44 | 3 | 344 | 4.2 |
| 13.104 | 6.7509 | 42 | 2.9 | 175 | 2.1 |
| 15.711 | 5.6360 | 310 | 21.4 | 2045 | 24.8 |
| 16.563 | 5.3479 | 130 | 9 | 1084 | 13.1 |
| 18.627 | 4.7597 | 147 | 10.1 | 1171 | 14.2 |
| 19.236 | 4.6102 | 421 | 29 | 3053 | 37 |
| 20.341 | 4.3622 | 134 | 9.2 | 830 | 10 |
| 21.240 | 4.1795 | 120 | 8.3 | 962 | 11.6 |
| 22.169 | 4.0065 | 68 | 4.7 | 912 | 11 |
| 24.074 | 3.6936 | 72 | 5 | 484 | 5.9 |
| 24.592 | 3.6170 | 76 | 5.2 | 473 | 5.7 |
| 25.888 | 3.4388 | 42 | 2.9 | 469 | 5.7 |
| 26.022 | 3.4213 | 89 | 6.1 | 986 | 11.9 |
| 26.797 | 3.3241 | 106 | 7.3 | 1266 | 15.3 |
| 28.055 | 3.1779 | 124 | 8.5 | 1142 | 13.8 |
| 30.498 | 2.9287 | 59 | 4.1 | 728 | 8.8 |
| 32.479 | 2.7544 | 35 | 2.4 | 290 | 3.5 |
| 33.486 | 2.6738 | 55 | 3.8 | 587 | 7.1 |
| 35.241 | 2.5446 | 37 | 2.5 | 409 | 5 |
| 38.404 | 2.3420 | 29 | 2 | 155 | 1.9 |

FIG.26

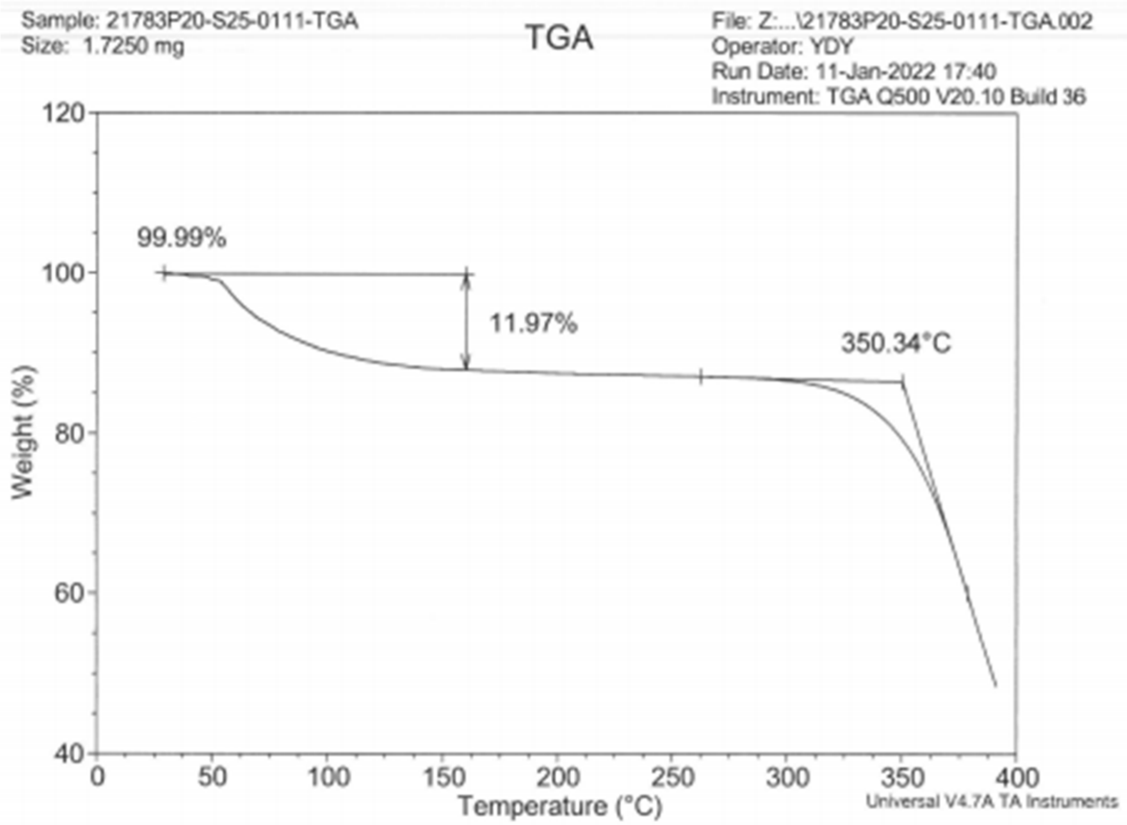
Sample: 21783P20-S25-0111-TGA
Size: 1.7250 mg
TGA
File: Z:...\21783P20-S25-0111-TGA.002
Operator: YDY
Run Date: 11-Jan-2022 17:40
Instrument: TGA Q500 V20.10 Build 36
99.99%
11.97%
350.34°C
Weight (%)
Temperature (°C)
Universal V4.7A TA Instruments

FIG.27

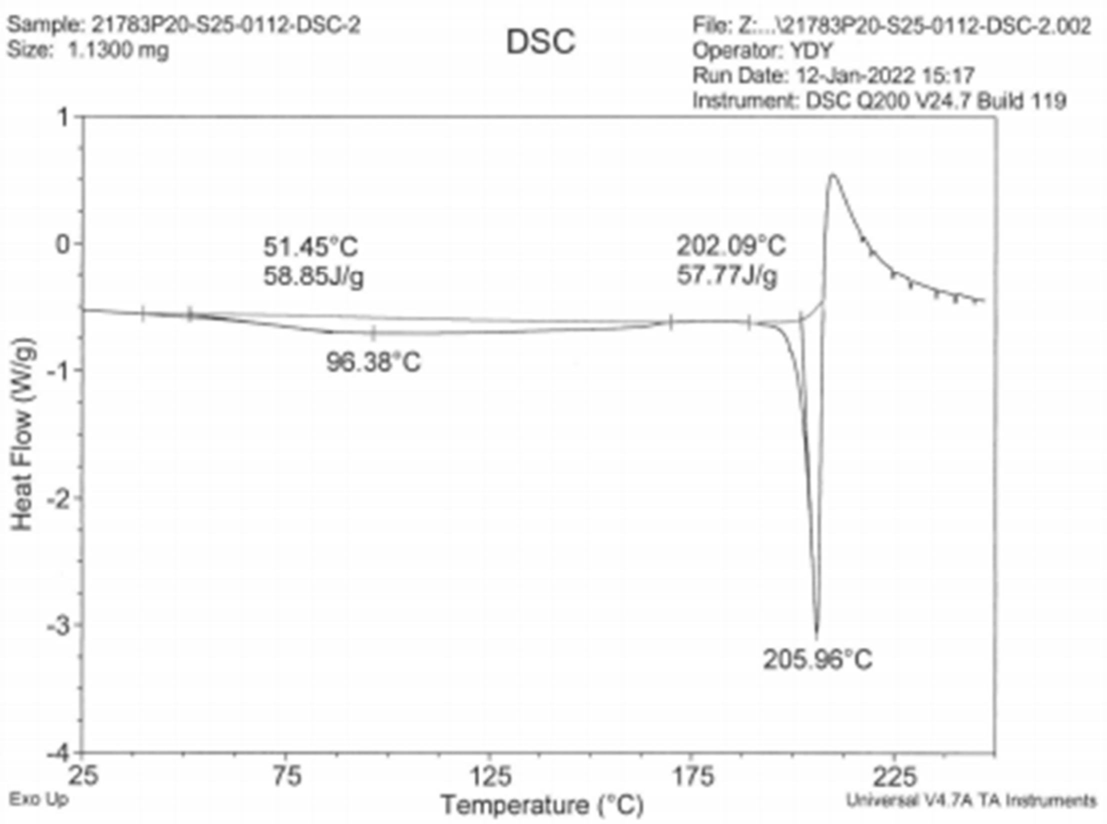
Sample: 21783P20-S25-0112-DSC-2
Size: 1.1300 mg
DSC
File: Z:...\21783P20-S25-0112-DSC-2.002
Operator: YDY
Run Date: 12-Jan-2022 15:17
Instrument: DSC Q200 V24.7 Build 119
51.45°C
58.85J/g
202.09°C
57.77J/g
96.38°C
205.96°C
Heat Flow (W/g)
Exo Up
Temperature (°C)
Universal V4.7A TA Instruments

FIG.28

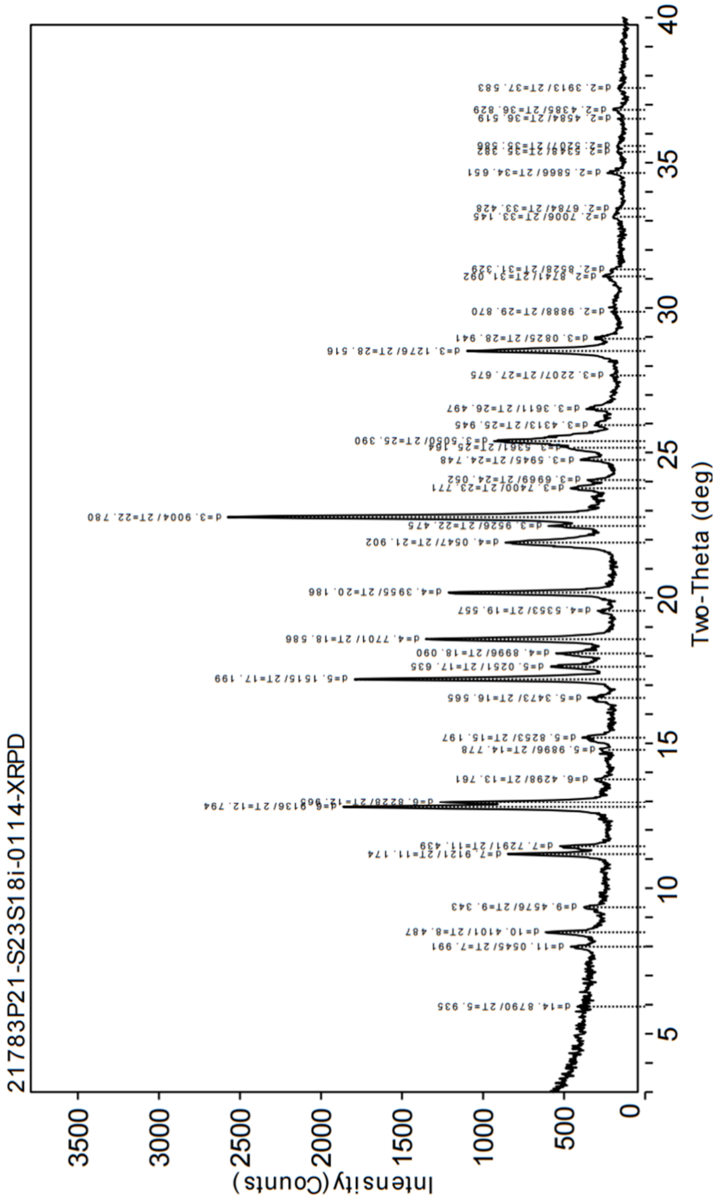
21783P21-S23S18i-0114-XRPD
Intensity(Counts)
Two-Theta (deg)
0
500
1000
1500
2000
2500
3000
3500
5
10
15
20
25
30
35
40

FIG.29

| 21783P21-S23S18i-0114-XRPD | | | | | |
|---|---|---|---|---|---|
| 2-Theta | d | Height | I% | Area | I% |
| 5.935 | 14.8790 | 63 | 2.8 | 230 | 1 |
| 7.991 | 11.0545 | 138 | 6 | 939 | 4.3 |
| 8.487 | 10.4101 | 322 | 14.1 | 2770 | 12.6 |
| 9.343 | 9.4576 | 115 | 5 | 970 | 4.4 |
| 11.174 | 7.9121 | 624 | 27.2 | 5506 | 25 |
| 11.439 | 7.7291 | 300 | 13.1 | 3349 | 15.2 |
| 12.794 | 6.9136 | 1636 | 71.4 | 15583 | 70.8 |
| 12.965 | 6.8228 | 1019 | 44.5 | 8993 | 40.8 |
| 13.761 | 6.4298 | 84 | 3.7 | 561 | 2.5 |
| 14.778 | 5.9896 | 49 | 2.1 | 340 | 1.5 |
| 15.197 | 5.8253 | 176 | 7.7 | 2215 | 10.1 |
| 16.565 | 5.3473 | 149 | 6.5 | 1525 | 6.9 |
| 17.199 | 5.1515 | 1549 | 67.6 | 11605 | 52.7 |
| 17.635 | 5.0251 | 289 | 12.6 | 2265 | 10.3 |
| 18.090 | 4.8996 | 252 | 11 | 1985 | 9 |
| 18.586 | 4.7701 | 1101 | 48.1 | 9315 | 42.3 |
| 19.557 | 4.5353 | 93 | 4.1 | 694 | 3.2 |
| 20.186 | 4.3955 | 1015 | 44.3 | 8937 | 40.6 |
| 21.902 | 4.0547 | 605 | 26.4 | 8360 | 38 |
| 22.475 | 3.9526 | 254 | 11.1 | 1828 | 8.3 |
| 22.780 | 3.9004 | 2290 | 100 | 22024 | 100 |
| 23.771 | 3.7400 | 218 | 9.5 | 3495 | 15.9 |
| 24.052 | 3.6969 | 122 | 5.3 | 737 | 3.3 |
| 24.748 | 3.5945 | 147 | 6.4 | 696 | 3.2 |
| 25.164 | 3.5361 | 174 | 7.6 | 4085 | 18.5 |
| 25.390 | 3.5050 | 677 | 29.6 | 11441 | 51.9 |
| 25.945 | 3.4313 | 76 | 3.3 | 806 | 3.7 |
| 26.497 | 3.3611 | 154 | 6.7 | 1562 | 7.1 |
| 27.675 | 3.2207 | 41 | 1.8 | 290 | 1.3 |
| 28.516 | 3.1276 | 891 | 38.9 | 8662 | 39.3 |
| 28.941 | 3.0825 | 92 | 4 | 765 | 3.5 |
| 29.870 | 2.9888 | 46 | 2 | 758 | 3.4 |
| 31.092 | 2.8741 | 110 | 4.8 | 1971 | 8.9 |
| 31.329 | 2.8528 | 49 | 2.1 | 1103 | 5 |
| 33.145 | 2.7006 | 61 | 2.7 | 1215 | 5.5 |
| 33.428 | 2.6784 | 43 | 1.9 | 891 | 4 |
| 34.651 | 2.5866 | 104 | 4.5 | 1059 | 4.8 |
| 35.382 | 2.5348 | 42 | 1.8 | 863 | 3.9 |
| 35.586 | 2.5207 | 39 | 1.7 | 797 | 3.6 |
| 36.519 | 2.4584 | 36 | 1.6 | 395 | 1.8 |
| 36.829 | 2.4385 | 72 | 3.1 | 876 | 4 |
| 37.583 | 2.3913 | 45 | 2 | 628 | 2.9 |

FIG.30

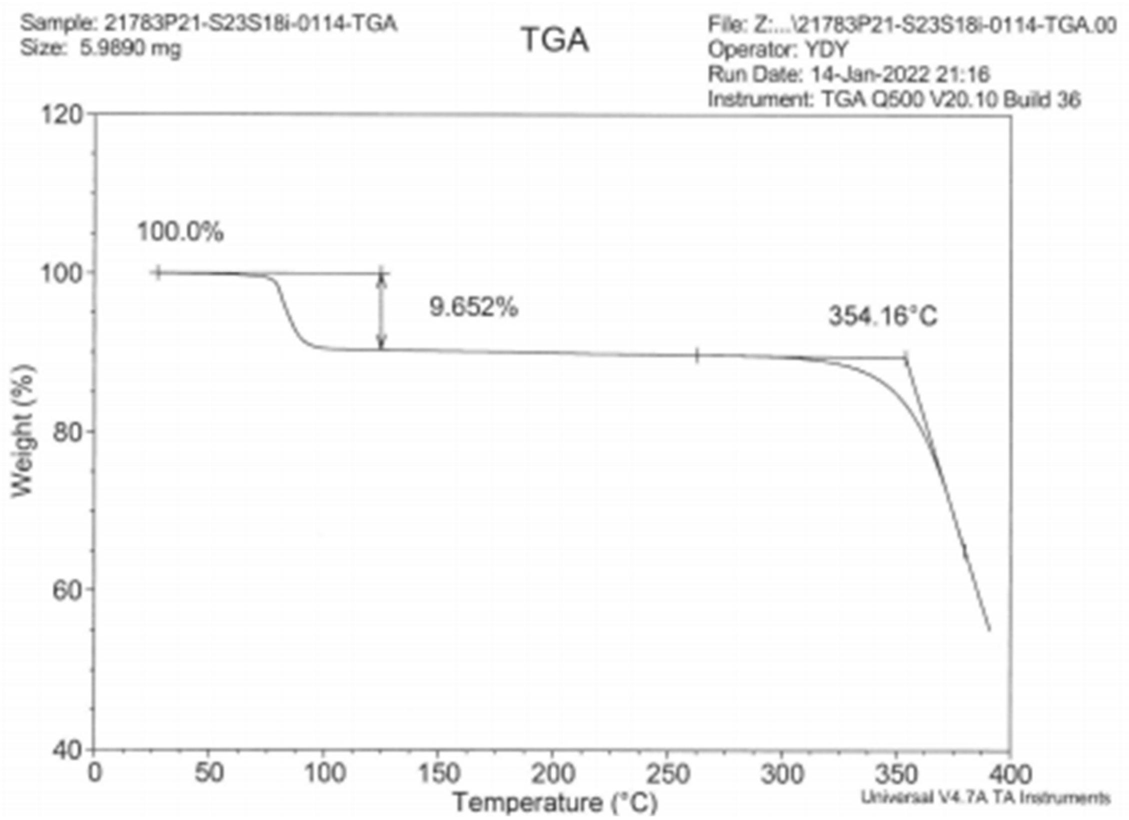
Sample: 21783P21-S23S18i-0114-TGA
Size: 5.9890 mg
TGA
File: Z:...\21783P21-S23S18i-0114-TGA.00
Operator: YDY
Run Date: 14-Jan-2022 21:16
Instrument: TGA Q500 V20.10 Build 36
100.0%
9.652%
354.16°C
Weight (%)
Temperature (°C)
Universal V4.7A TA Instruments

FIG.31

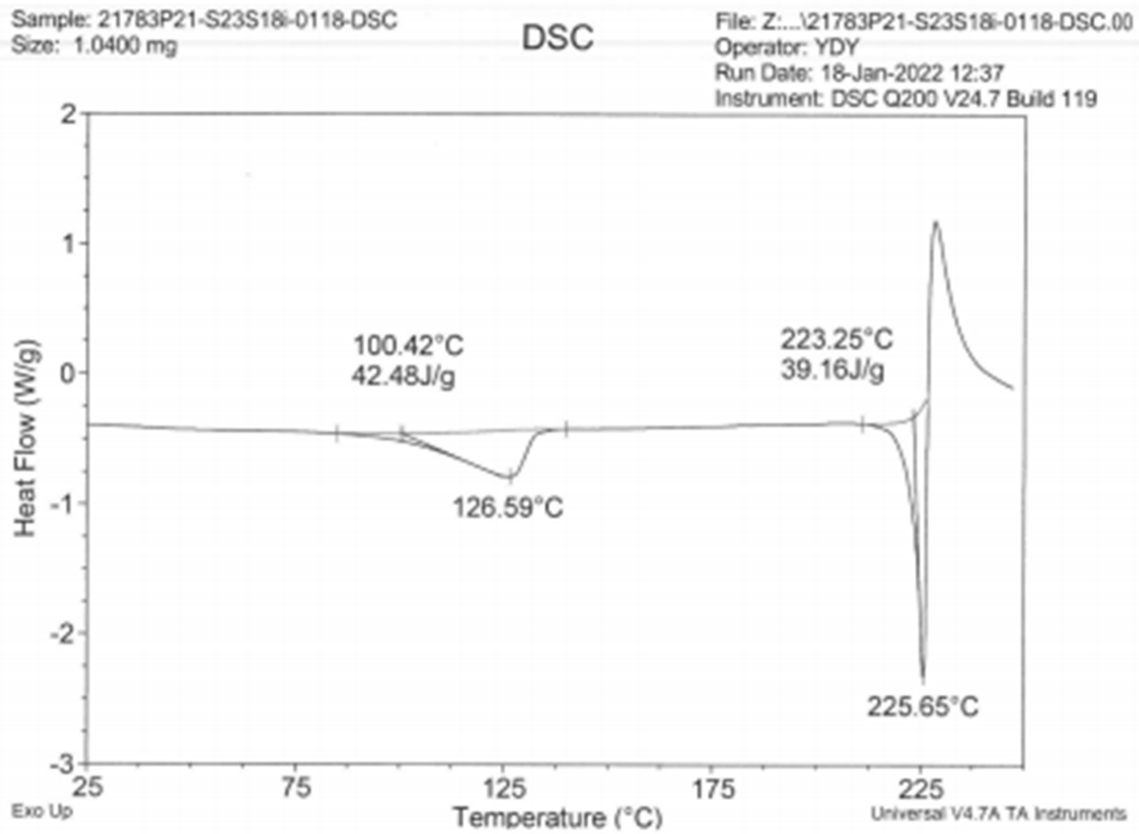
Sample: 21783P21-S23S18i-0118-DSC
Size: 1.0400 mg
DSC
File: Z:...\21783P21-S23S18i-0118-DSC.00
Operator: YDY
Run Date: 18-Jan-2022 12:37
Instrument: DSC Q200 V24.7 Build 119
100.42°C
42.48J/g
126.59°C
223.25°C
39.16J/g
225.65°C
Heat Flow (W/g)
Temperature (°C)
Exo Up
Universal V4.7A TA Instruments

FIG.32

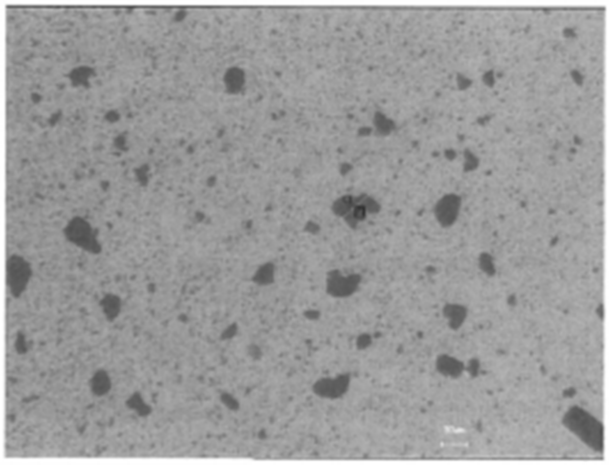

FIG.33

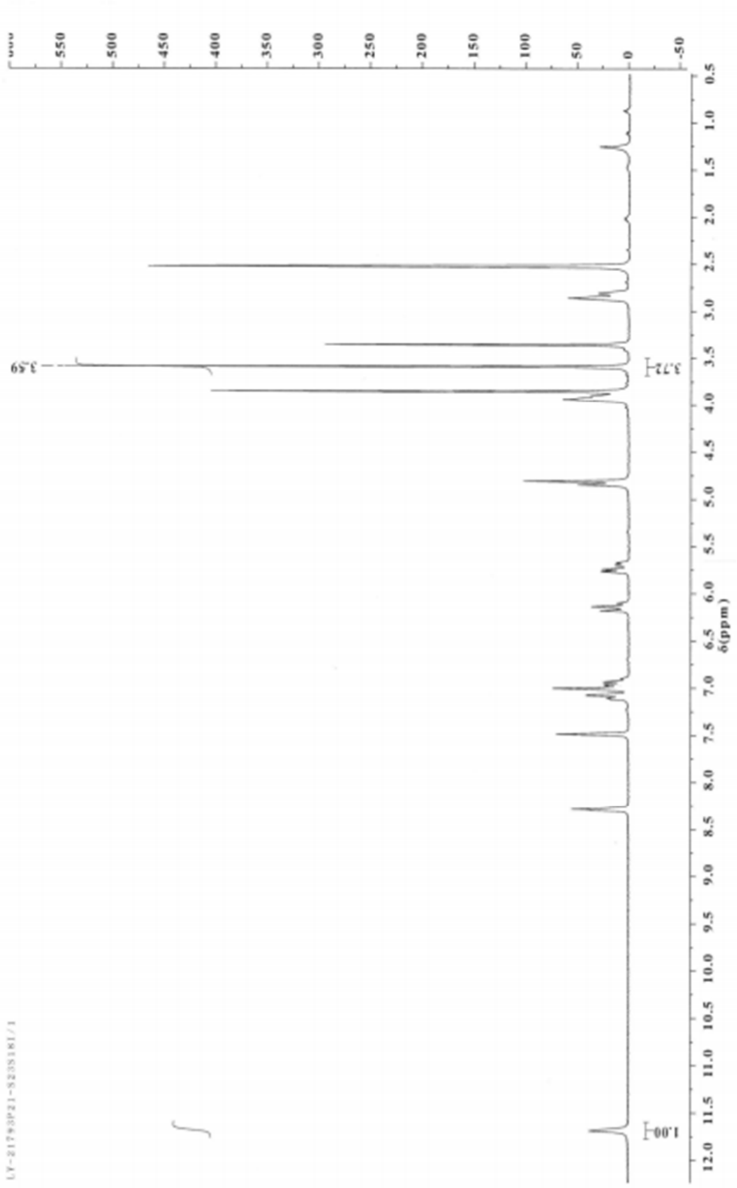

FIG.34

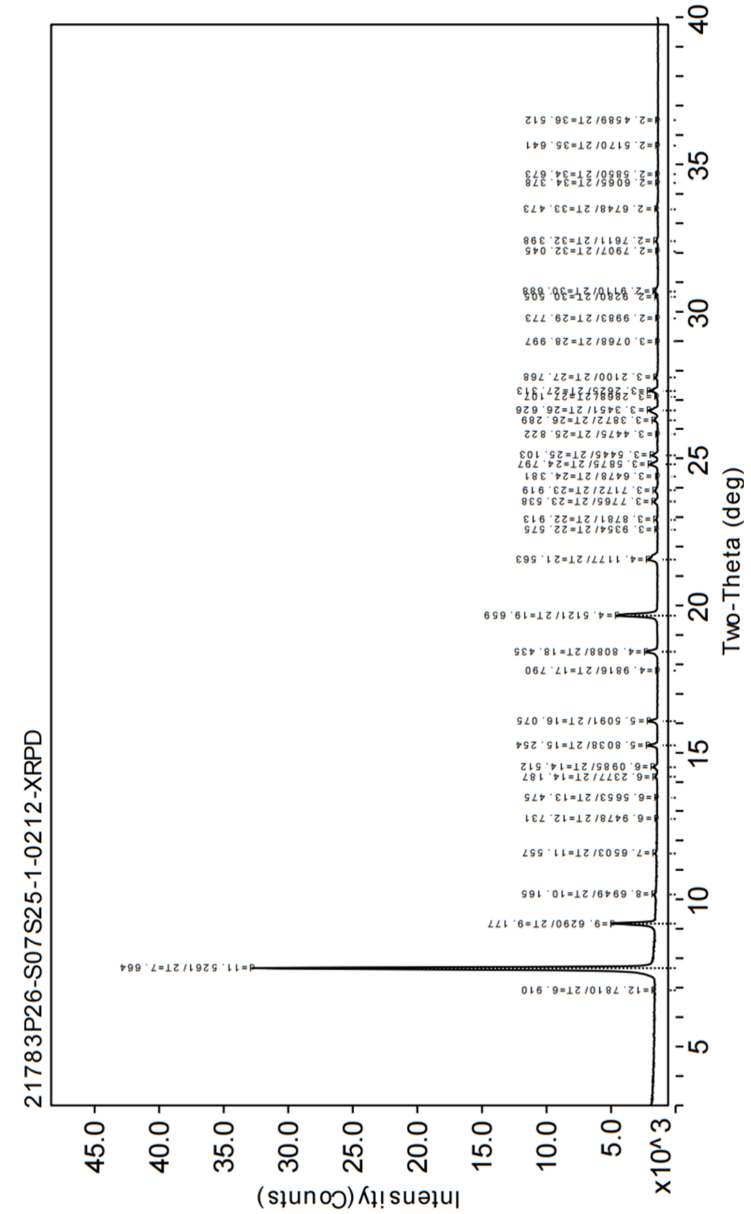
21783P26-S07S25-1-0212-XRPD
Intensity(Counts)
x10^3
Two-Theta (deg)

FIG.35

| 21783P26-S07S25-1-0212-XRPD | | | | | |
|---|---|---|---|---|---|
| 2-Theta | d | Height | I% | Area | I% |
| 6.910 | 12.7810 | 82 | 0.3 | 578 | 0.4 |
| 7.664 | 11.5261 | 31294 | 100 | 160829 | 100 |
| 9.177 | 9.6290 | 3344 | 10.7 | 18513 | 11.5 |
| 10.165 | 8.6949 | 172 | 0.5 | 890 | 0.6 |
| 11.557 | 7.6503 | 162 | 0.5 | 1073 | 0.7 |
| 12.731 | 6.9478 | 58 | 0.2 | 243 | 0.2 |
| 13.475 | 6.5653 | 51 | 0.2 | 178 | 0.1 |
| 14.187 | 6.2377 | 186 | 0.6 | 1344 | 0.8 |
| 14.512 | 6.0985 | 336 | 1.1 | 1659 | 1 |
| 15.254 | 5.8038 | 720 | 2.3 | 3895 | 2.4 |
| 16.075 | 5.5091 | 684 | 2.2 | 3582 | 2.2 |
| 17.790 | 4.9816 | 44 | 0.1 | 376 | 0.2 |
| 18.435 | 4.8088 | 896 | 2.9 | 6098 | 3.8 |
| 19.659 | 4.5121 | 3167 | 10.1 | 21905 | 13.6 |
| 21.563 | 4.1177 | 731 | 2.3 | 7635 | 4.7 |
| 22.575 | 3.9354 | 94 | 0.3 | 687 | 0.4 |
| 22.913 | 3.8781 | 128 | 0.4 | 727 | 0.5 |
| 23.538 | 3.7765 | 308 | 1 | 2040 | 1.3 |
| 23.919 | 3.7172 | 209 | 0.7 | 1612 | 1 |
| 24.381 | 3.6478 | 60 | 0.2 | 337 | 0.2 |
| 24.797 | 3.5875 | 601 | 1.9 | 4497 | 2.8 |
| 25.103 | 3.5445 | 463 | 1.5 | 2742 | 1.7 |
| 25.822 | 3.4475 | 47 | 0.2 | 190 | 0.1 |
| 26.289 | 3.3872 | 295 | 0.9 | 1988 | 1.2 |
| 26.626 | 3.3451 | 709 | 2.3 | 5536 | 3.4 |
| 27.107 | 3.2868 | 106 | 0.3 | 1859 | 1.2 |
| 27.313 | 3.2625 | 675 | 2.2 | 4414 | 2.7 |
| 27.768 | 3.2100 | 179 | 0.6 | 1108 | 0.7 |
| 28.997 | 3.0768 | 49 | 0.2 | 517 | 0.3 |
| 29.773 | 2.9983 | 71 | 0.2 | 580 | 0.4 |
| 30.505 | 2.9280 | 131 | 0.4 | 1248 | 0.8 |
| 30.688 | 2.9110 | 289 | 0.9 | 3565 | 2.2 |
| 32.045 | 2.7907 | 69 | 0.2 | 855 | 0.5 |
| 32.398 | 2.7611 | 160 | 0.5 | 944 | 0.6 |
| 33.473 | 2.6748 | 140 | 0.4 | 1085 | 0.7 |
| 34.378 | 2.6065 | 48 | 0.2 | 297 | 0.2 |
| 34.673 | 2.5850 | 81 | 0.3 | 752 | 0.5 |
| 35.641 | 2.5170 | 39 | 0.1 | 503 | 0.3 |
| 36.512 | 2.4589 | 52 | 0.2 | 317 | 0.2 |

FIG.36

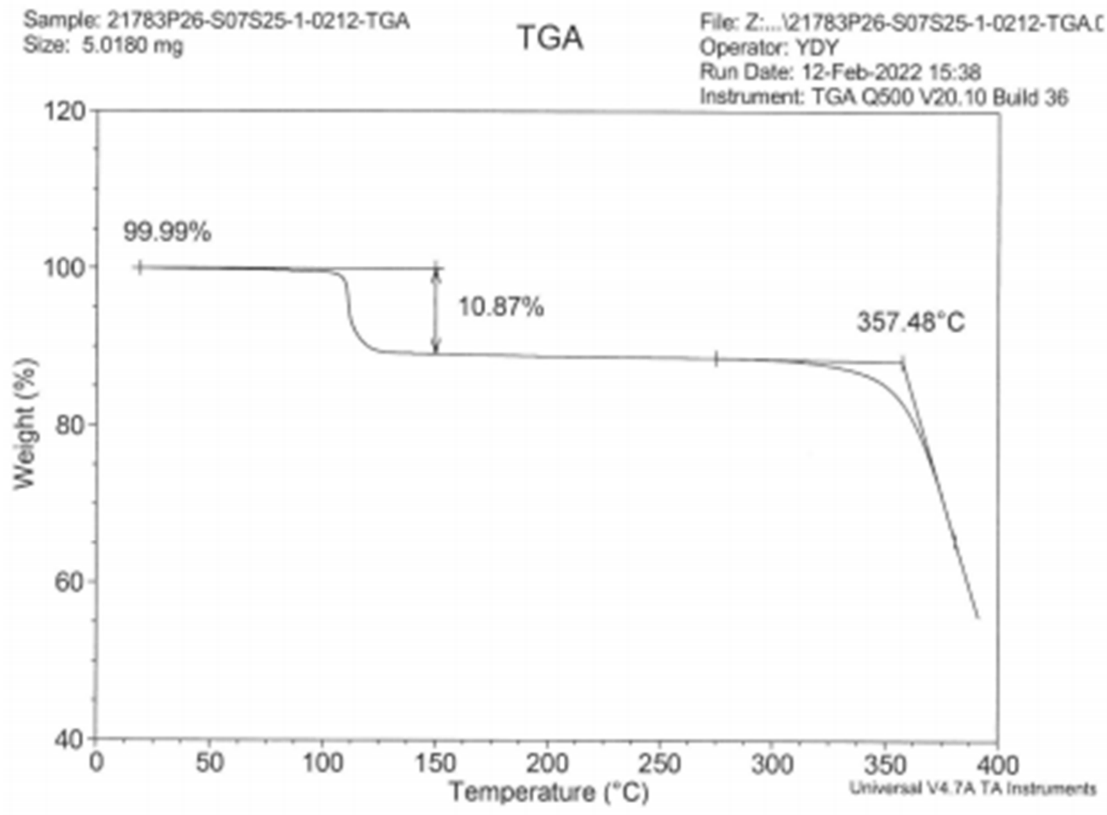
Sample: 21783P26-S07S25-1-0212-TGA
Size: 5.0180 mg
TGA
File: Z:...\21783P26-S07S25-1-0212-TGA.0
Operator: YDY
Run Date: 12-Feb-2022 15:38
Instrument: TGA Q500 V20.10 Build 36
99.99%
10.87%
357.48°C
Weight (%)
Temperature (°C)
Universal V4.7A TA Instruments

FIG.37

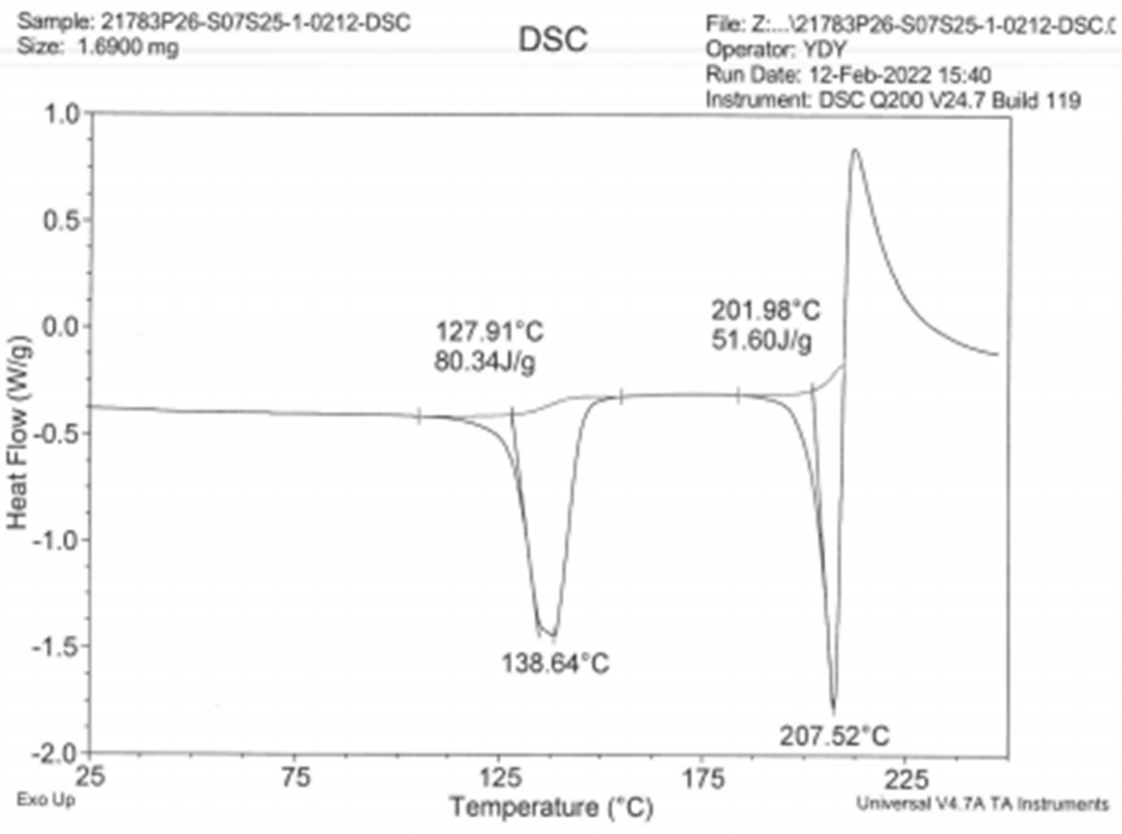
Sample: 21783P26-S07S25-1-0212-DSC
Size: 1.6900 mg
DSC
File: Z:...\21783P26-S07S25-1-0212-DSC.0
Operator: YDY
Run Date: 12-Feb-2022 15:40
Instrument: DSC Q200 V24.7 Build 119
127.91°C
80.34J/g
201.98°C
51.60J/g
138.64°C
207.52°C
Heat Flow (W/g)
Exo Up
Temperature (°C)
Universal V4.7A TA Instruments

FIG.38

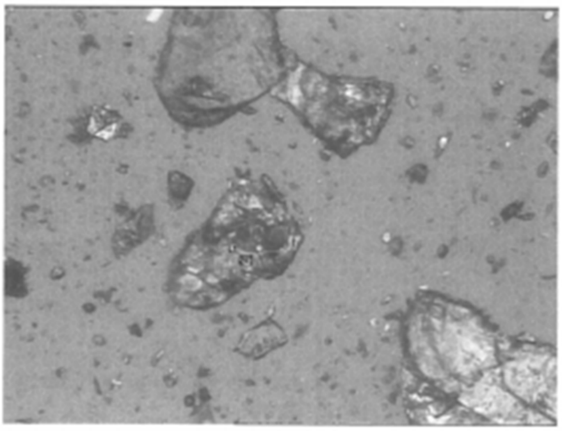

FIG.39

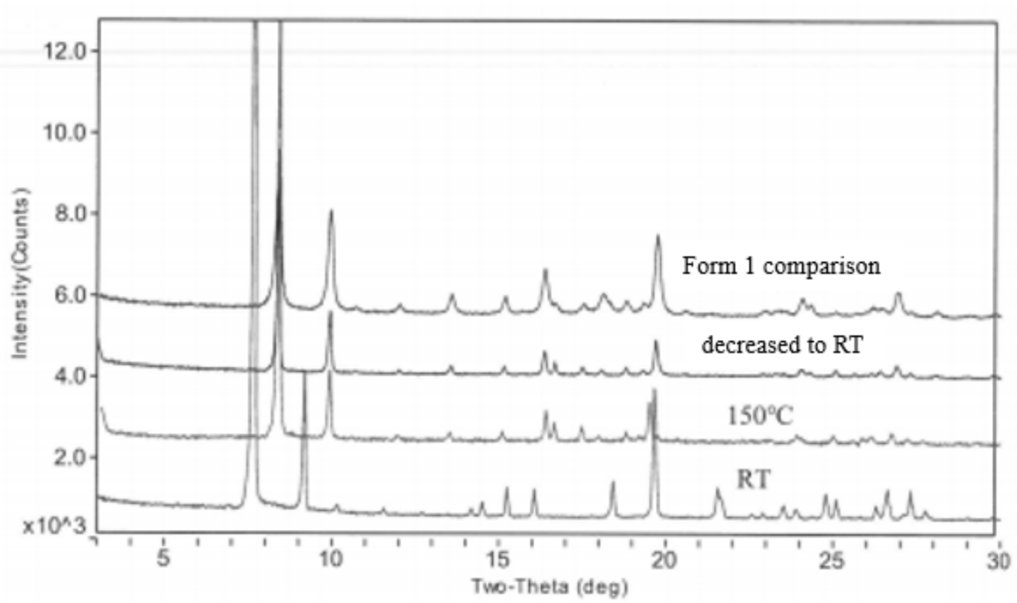
12.0
10.0
8.0
6.0
4.0
2.0
x10^3
Intensity(Counts)
Form 1 comparison
decreased to RT
150°C
RT
5
10
15
20
25
30
Two-Theta (deg)

FIG.40

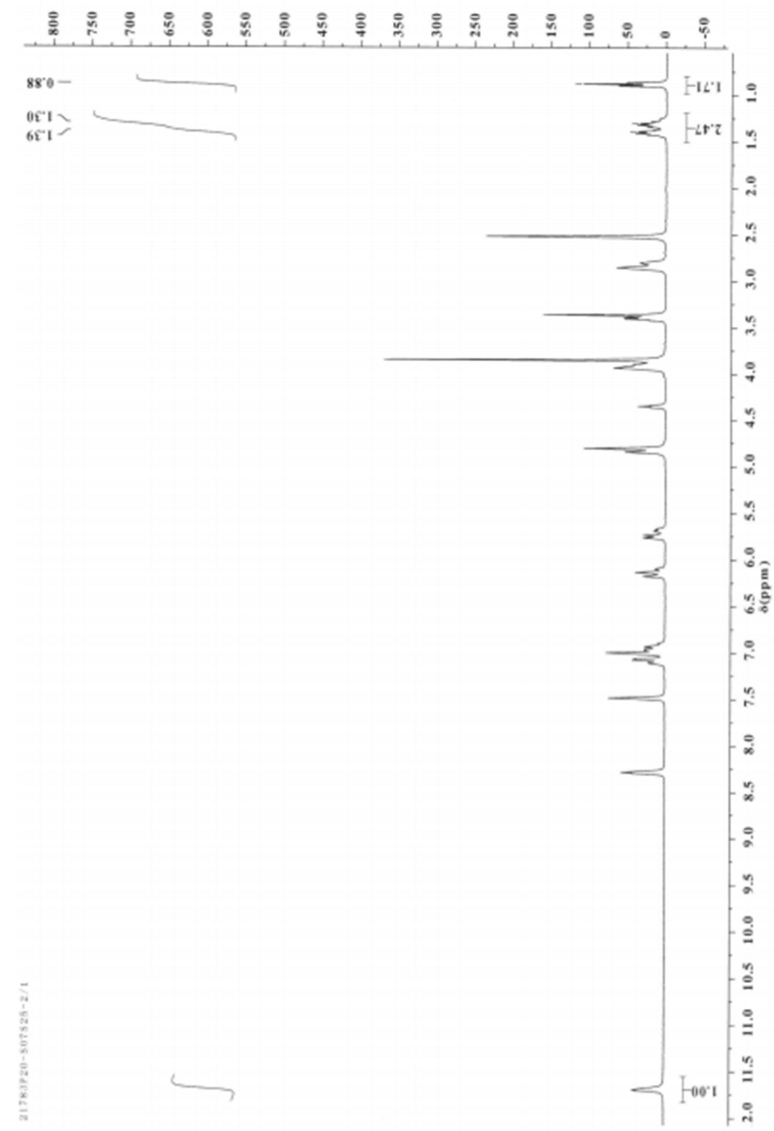

FIG.41

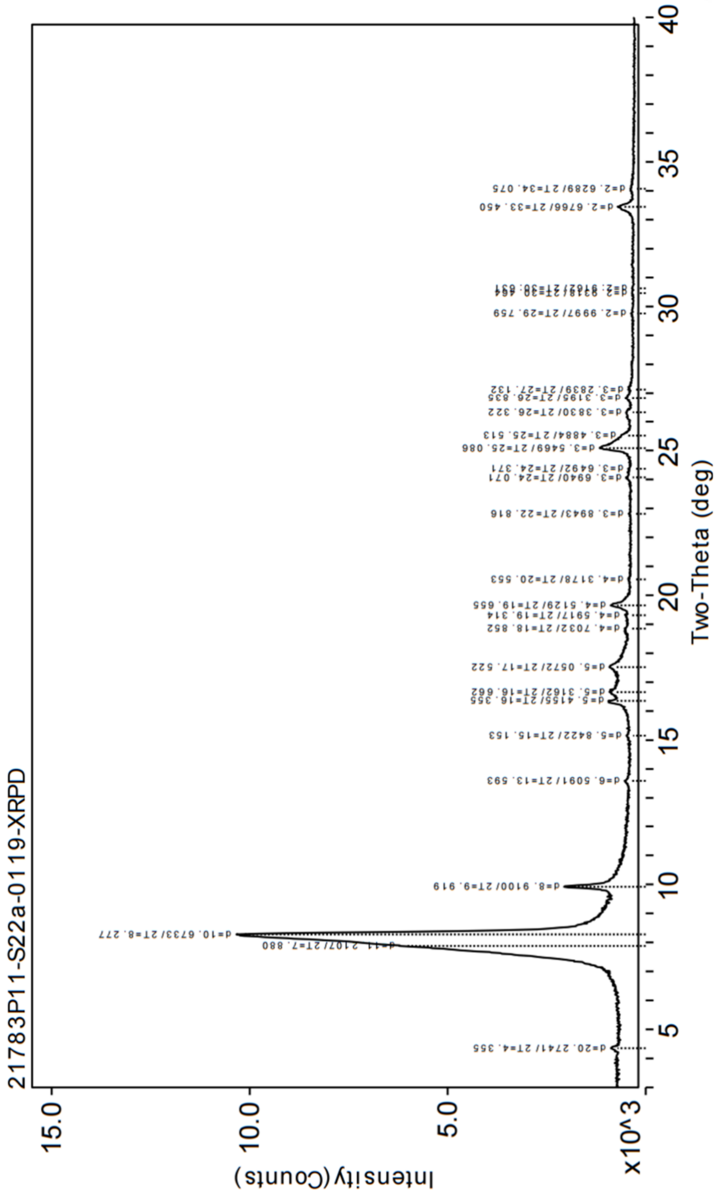
21783P11-S22a-0119-XRPD
Two-Theta (deg)
Intensity(Counts)
x10^3

FIG.42

| 21783P11-S22a-0119-XRPD | | | | | |
|---|---|---|---|---|---|
| **2-Theta** | **d** | **Height** | **I%** | **Area** | **I%** |
| 4.355 | 20.2741 | 188 | 2.1 | 1518 | 0.7 |
| 7.880 | 11.2107 | 4394 | 48.5 | 105355 | 46.2 |
| 8.277 | 10.6733 | 9069 | 100 | 227875 | 100 |
| 9.919 | 8.9100 | 1241 | 13.7 | 11818 | 5.2 |
| 13.593 | 6.5091 | 114 | 1.3 | 1160 | 0.5 |
| 15.153 | 5.8422 | 68 | 0.7 | 409 | 0.2 |
| 16.355 | 5.4155 | 482 | 5.3 | 5699 | 2.5 |
| 16.662 | 5.3162 | 275 | 3 | 5661 | 2.5 |
| 17.522 | 5.0572 | 275 | 3 | 4627 | 2 |
| 18.852 | 4.7032 | 77 | 0.8 | 697 | 0.3 |
| 19.314 | 4.5917 | 70 | 0.8 | 302 | 0.1 |
| 19.655 | 4.5129 | 450 | 5 | 5932 | 2.6 |
| 20.553 | 4.3178 | 51 | 0.6 | 435 | 0.2 |
| 22.816 | 3.8943 | 51 | 0.6 | 256 | 0.1 |
| 24.071 | 3.6940 | 114 | 1.3 | 1611 | 0.7 |
| 24.371 | 3.6492 | 57 | 0.6 | 801 | 0.4 |
| 25.086 | 3.5469 | 714 | 7.9 | 12500 | 5.5 |
| 25.513 | 3.4884 | 233 | 2.6 | 1844 | 0.8 |
| 26.322 | 3.3830 | 111 | 1.2 | 1826 | 0.8 |
| 26.835 | 3.3195 | 132 | 1.5 | 1694 | 0.7 |
| 27.132 | 3.2839 | 77 | 0.8 | 1251 | 0.5 |
| 29.759 | 2.9997 | 57 | 0.6 | 581 | 0.3 |
| 30.464 | 2.9318 | 39 | 0.4 | 553 | 0.2 |
| 30.631 | 2.9162 | 42 | 0.5 | 548 | 0.2 |
| 33.450 | 2.6766 | 378 | 4.2 | 4992 | 2.2 |
| 34.075 | 2.6289 | 63 | 0.7 | 927 | 0.4 |

FIG.43

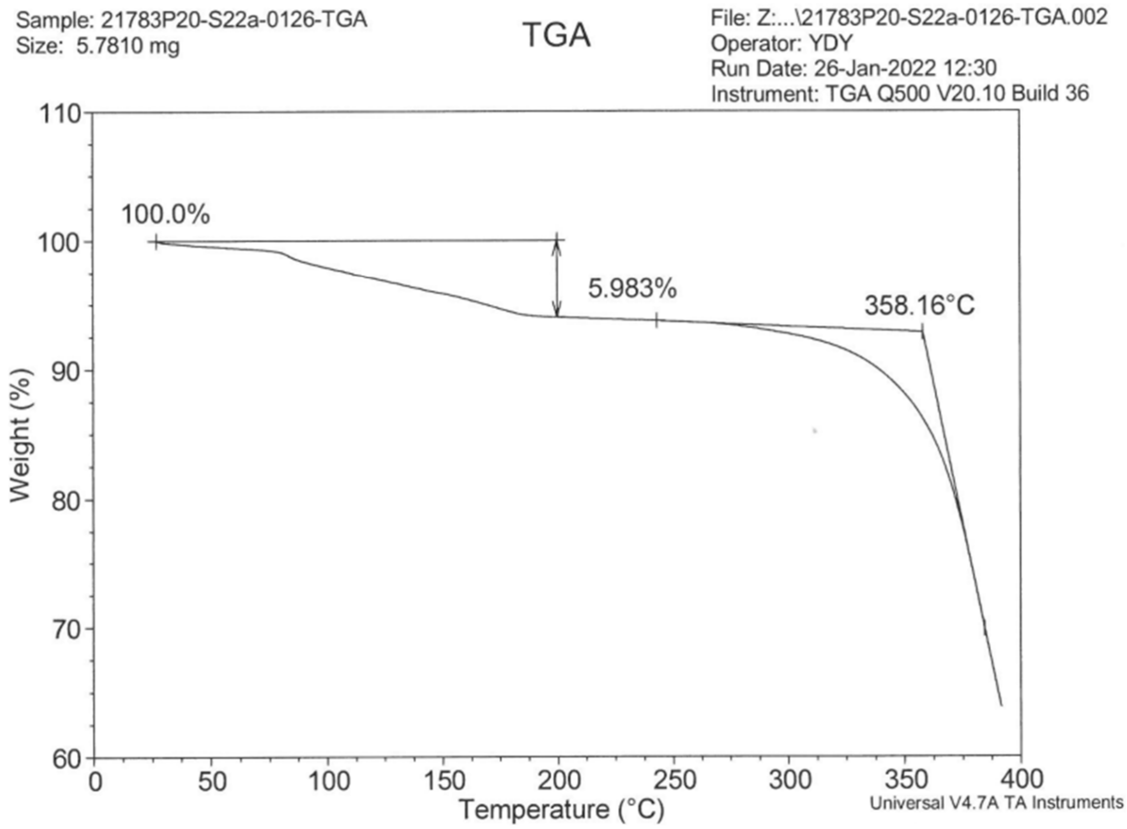
Sample: 21783P20-S22a-0126-TGA
Size: 5.7810 mg
TGA
File: Z:...\21783P20-S22a-0126-TGA.002
Operator: YDY
Run Date: 26-Jan-2022 12:30
Instrument: TGA Q500 V20.10 Build 36
100.0%
5.983%
358.16°C
Weight (%)
Temperature (°C)
Universal V4.7A TA Instruments

FIG.44

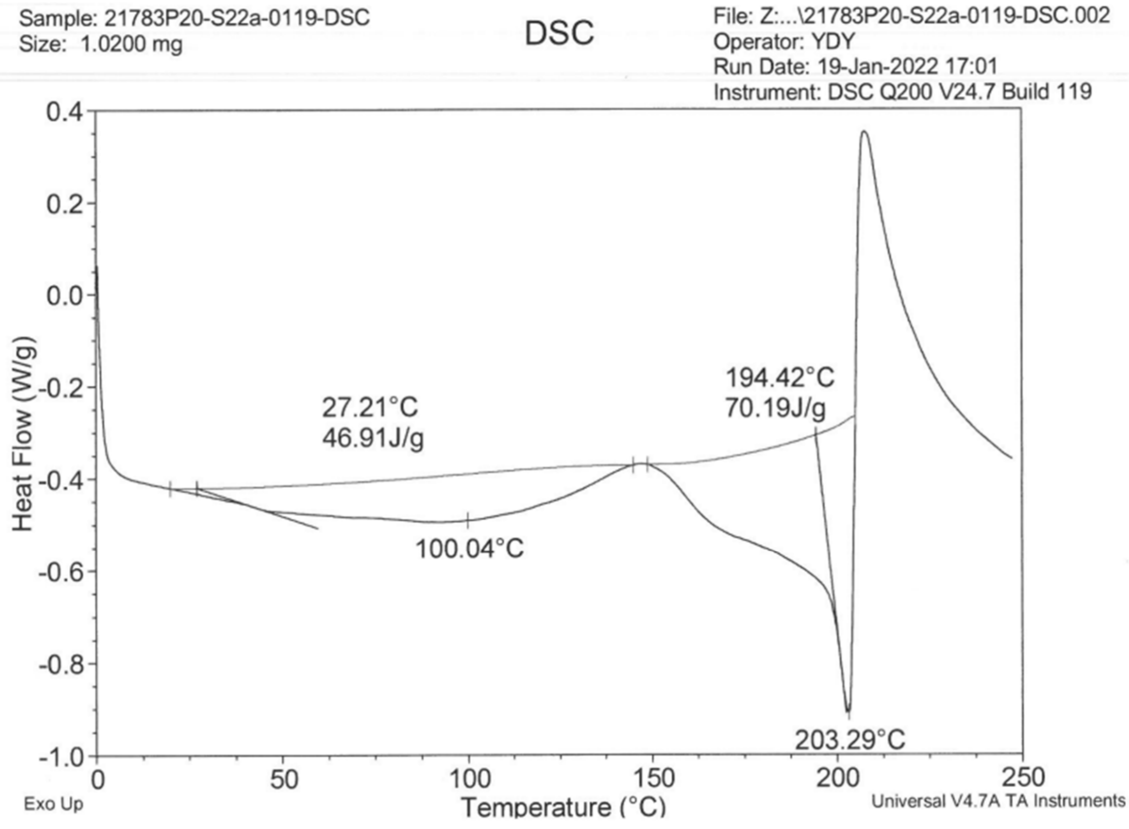
Sample: 21783P20-S22a-0119-DSC
Size: 1.0200 mg
DSC
File: Z:...\21783P20-S22a-0119-DSC.002
Operator: YDY
Run Date: 19-Jan-2022 17:01
Instrument: DSC Q200 V24.7 Build 119
27.21°C
46.91J/g
100.04°C
194.42°C
70.19J/g
203.29°C
Heat Flow (W/g)
Exo Up
Temperature (°C)
Universal V4.7A TA Instruments

FIG.45

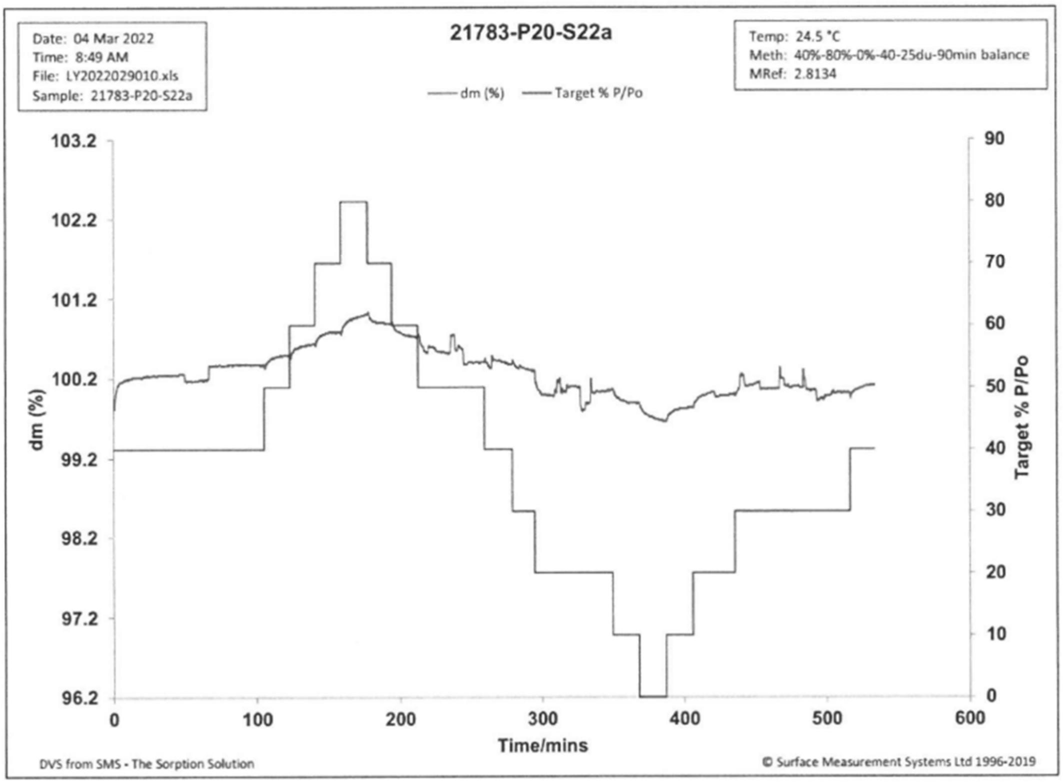
Date: 04 Mar 2022
Time: 8:49 AM
File: LY2022029010.xls
Sample: 21783-P20-S22a
21783-P20-S22a
Temp: 24.5 °C
Meth: 40%-80%-0%-40-25du-90min balance
MRef: 2.8134
dm (%)
Target % P/Po
dm (%)
Target % P/Po
Time/mins
DVS from SMS - The Sorption Solution
© Surface Measurement Systems Ltd 1996-2019

FIG.46

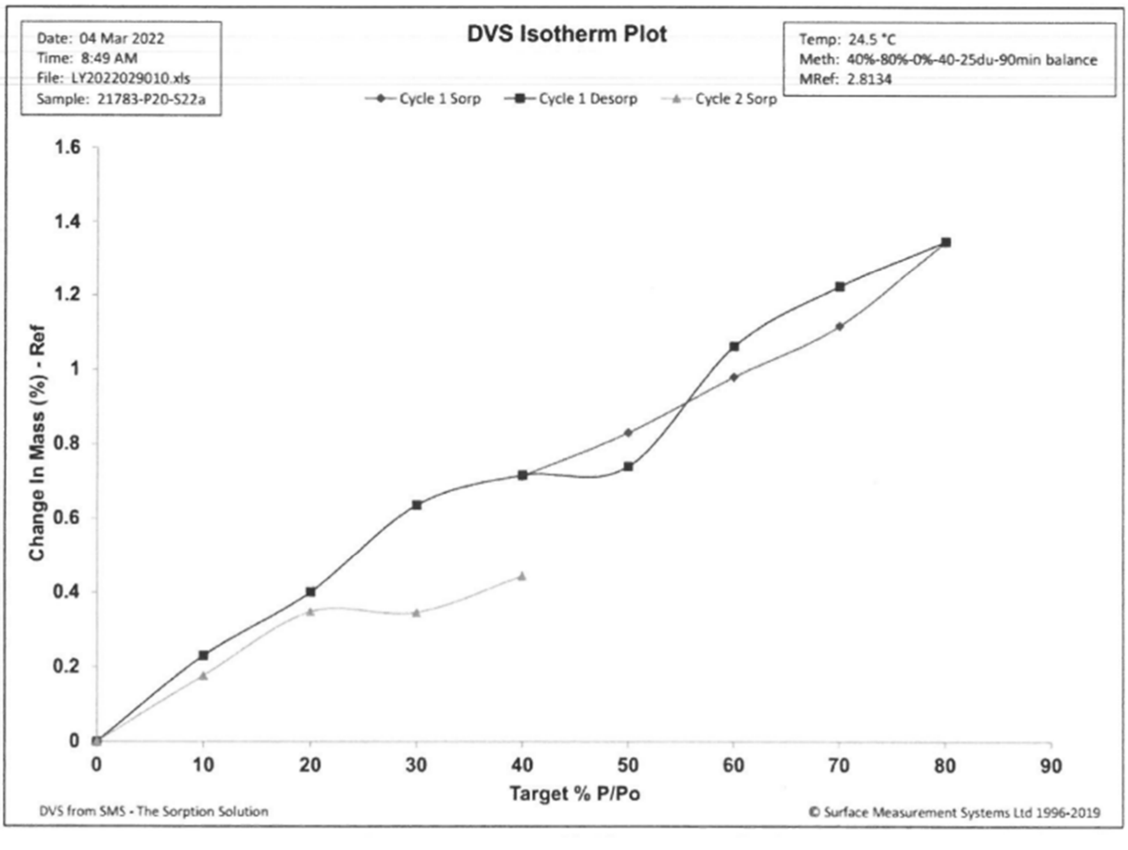
Date: 04 Mar 2022
Time: 8:49 AM
File: LY2022029010.xls
Sample: 21783-P20-S22a
DVS Isotherm Plot
Temp: 24.5 °C
Meth: 40%-80%-0%-40-25du-90min balance
MRef: 2.8134
Cycle 1 Sorp
Cycle 1 Desorp
Cycle 2 Sorp
Change In Mass (%) - Ref
Target % P/Po
DVS from SMS - The Sorption Solution
© Surface Measurement Systems Ltd 1996-2019

FIG.47

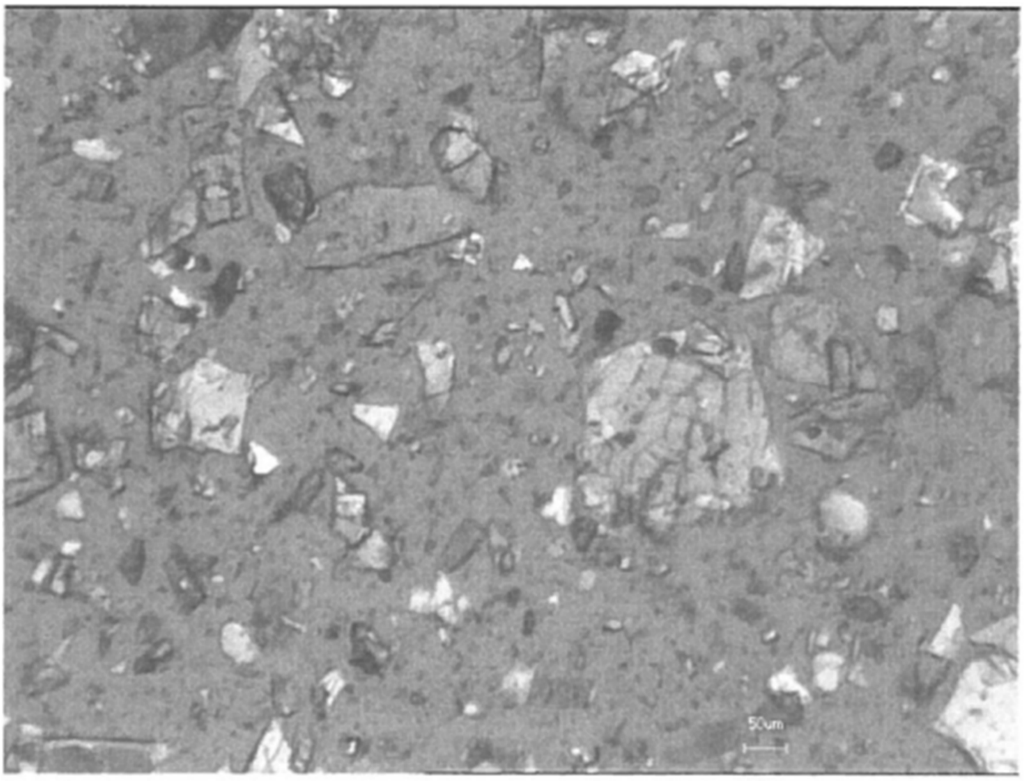
50um

FIG.48

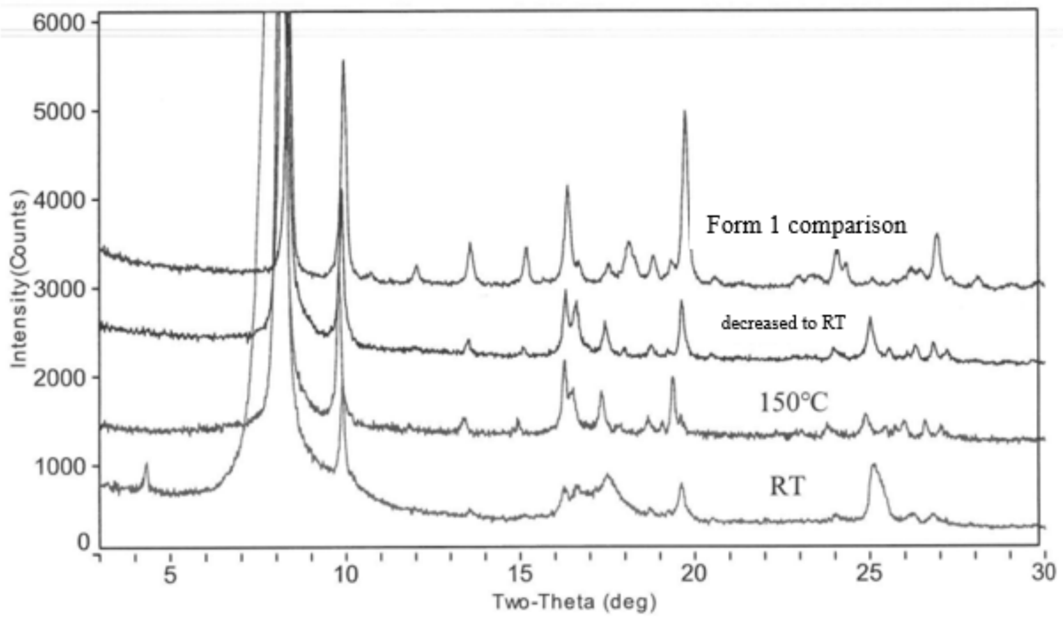
6000
5000
4000
3000
2000
1000
0
Intensity(Counts)
5
10
15
20
25
30
Two-Theta (deg)
Form 1 comparison
decreased to RT
150°C
RT

FIG.49

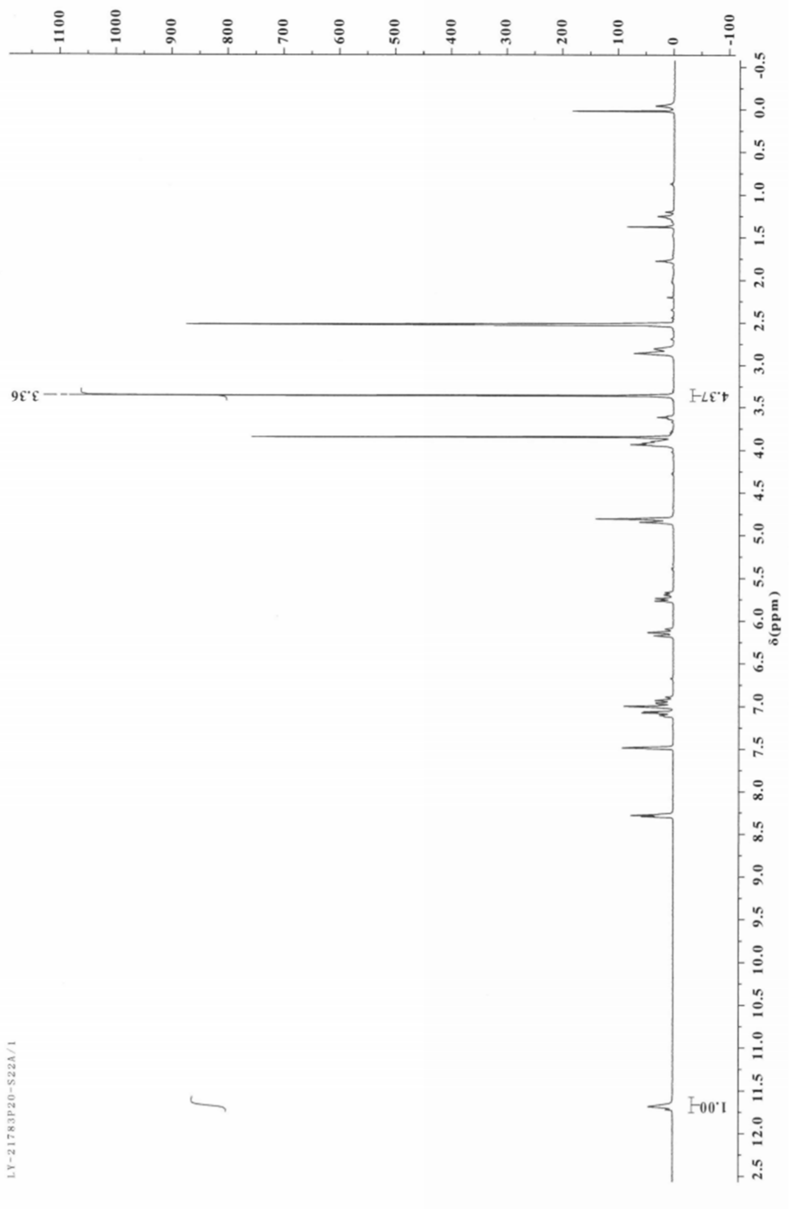

FIG.50

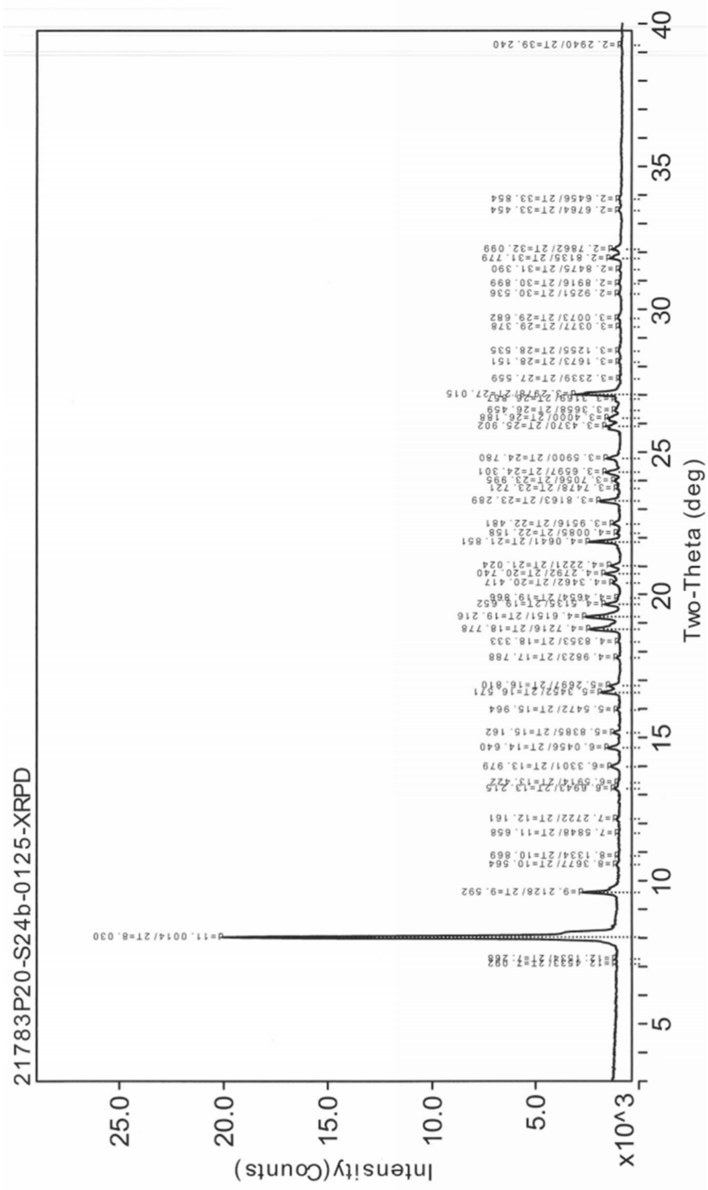

FIG.51

| 21783P20-S24b-0125-XRPD | | | | | |
|---|---|---|---|---|---|
| 2-Theta | d | Height | I% | Area | I% |
| 7.092 | 12.4533 | 72 | 0.4 | 730 | 0.5 |
| 7.268 | 12.1534 | 76 | 0.4 | 729 | 0.5 |
| 8.030 | 11.0014 | 19113 | 100 | 134644 | 100 |
| 9.592 | 9.2128 | 1737 | 9.1 | 13017 | 9.7 |
| 10.564 | 8.3677 | 123 | 0.6 | 1371 | 1 |
| 10.869 | 8.1334 | 56 | 0.3 | 360 | 0.3 |
| 11.658 | 7.5848 | 52 | 0.3 | 466 | 0.3 |
| 12.161 | 7.2722 | 147 | 0.8 | 1516 | 1.1 |
| 13.215 | 6.6943 | 245 | 1.3 | 1985 | 1.5 |
| 13.422 | 6.5914 | 74 | 0.4 | 1475 | 1.1 |
| 13.979 | 6.3301 | 434 | 2.3 | 3309 | 2.5 |
| 14.640 | 6.0456 | 580 | 3 | 4056 | 3 |
| 15.162 | 5.8385 | 298 | 1.6 | 1487 | 1.1 |
| 15.964 | 5.5472 | 139 | 0.7 | 896 | 0.7 |
| 16.571 | 5.3452 | 902 | 4.7 | 7954 | 5.9 |
| 16.810 | 5.2697 | 515 | 2.7 | 5946 | 4.4 |
| 17.788 | 4.9823 | 177 | 0.9 | 1252 | 0.9 |
| 18.333 | 4.8353 | 89 | 0.5 | 466 | 0.3 |
| 18.778 | 4.7216 | 1492 | 7.8 | 14599 | 10.8 |
| 19.216 | 4.6151 | 1511 | 7.9 | 11468 | 8.5 |
| 19.652 | 4.5135 | 686 | 3.6 | 4770 | 3.5 |
| 19.866 | 4.4654 | 90 | 0.5 | 1006 | 0.7 |
| 20.417 | 4.3462 | 327 | 1.7 | 3830 | 2.8 |
| 20.740 | 4.2792 | 742 | 3.9 | 7208 | 5.4 |
| 21.024 | 4.2221 | 413 | 2.2 | 1900 | 1.4 |
| 21.851 | 4.0641 | 1517 | 7.9 | 10530 | 7.8 |
| 22.158 | 4.0085 | 112 | 0.6 | 505 | 0.4 |
| 22.481 | 3.9516 | 313 | 1.6 | 2165 | 1.6 |
| 23.289 | 3.8163 | 977 | 5.1 | 7258 | 5.4 |
| 23.721 | 3.7478 | 106 | 0.6 | 840 | 0.6 |
| 23.995 | 3.7056 | 228 | 1.2 | 2863 | 2.1 |
| 24.301 | 3.6597 | 620 | 3.2 | 4479 | 3.3 |
| 24.780 | 3.5900 | 596 | 3.1 | 5045 | 3.7 |
| 25.902 | 3.4370 | 656 | 3.4 | 5939 | 4.4 |
| 26.188 | 3.4000 | 551 | 2.9 | 6257 | 4.6 |
| 26.459 | 3.3658 | 145 | 0.8 | 1077 | 0.8 |
| 26.857 | 3.3169 | 189 | 1 | 3415 | 2.5 |
| 27.015 | 3.2978 | 2181 | 11.4 | 16401 | 12.2 |
| 27.559 | 3.2339 | 47 | 0.2 | 229 | 0.2 |
| 28.151 | 3.1673 | 86 | 0.4 | 556 | 0.4 |
| 28.535 | 3.1255 | 107 | 0.6 | 1415 | 1.1 |
| 29.378 | 3.0377 | 83 | 0.4 | 798 | 0.6 |
| 29.682 | 3.0073 | 153 | 0.8 | 2131 | 1.6 |
| 30.536 | 2.9251 | 141 | 0.7 | 871 | 0.6 |
| 30.899 | 2.8916 | 130 | 0.7 | 1359 | 1 |
| 31.390 | 2.8475 | 82 | 0.4 | 449 | 0.3 |
| 31.779 | 2.8135 | 532 | 2.8 | 4597 | 3.4 |
| 32.099 | 2.7862 | 440 | 2.3 | 3520 | 2.6 |
| 33.454 | 2.6764 | 132 | 0.7 | 1018 | 0.8 |
| 33.854 | 2.6456 | 106 | 0.6 | 1059 | 0.8 |
| 39.240 | 2.2940 | 68 | 0.4 | 791 | 0.6 |

FIG.52

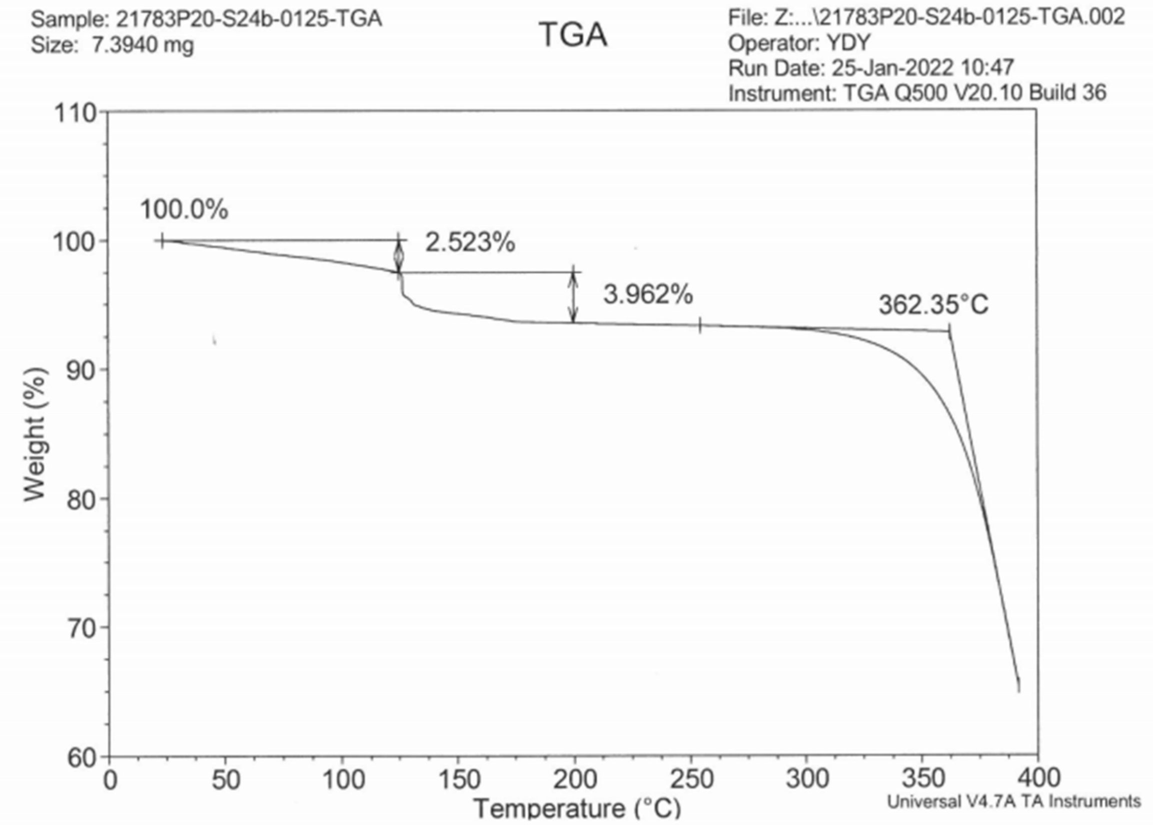
Sample: 21783P20-S24b-0125-TGA
Size: 7.3940 mg
TGA
File: Z:...\21783P20-S24b-0125-TGA.002
Operator: YDY
Run Date: 25-Jan-2022 10:47
Instrument: TGA Q500 V20.10 Build 36
100.0%
2.523%
3.962%
362.35°C
Weight (%)
Temperature (°C)
Universal V4.7A TA Instruments

FIG.53

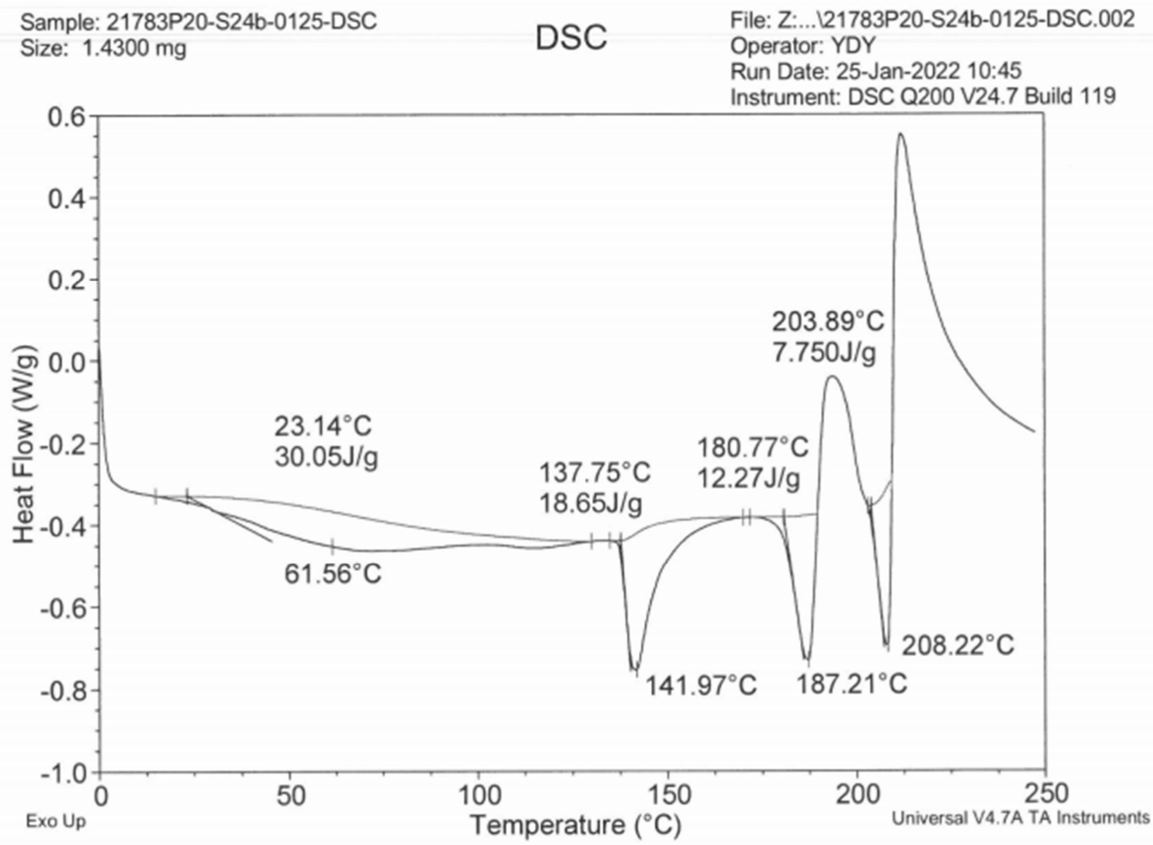
Sample: 21783P20-S24b-0125-DSC
Size: 1.4300 mg
DSC
File: Z:...\21783P20-S24b-0125-DSC.002
Operator: YDY
Run Date: 25-Jan-2022 10:45
Instrument: DSC Q200 V24.7 Build 119
203.89°C
7.750J/g
23.14°C
30.05J/g
180.77°C
12.27J/g
137.75°C
18.65J/g
61.56°C
208.22°C
141.97°C
187.21°C
Heat Flow (W/g)
Exo Up
Temperature (°C)
Universal V4.7A TA Instruments

FIG.54

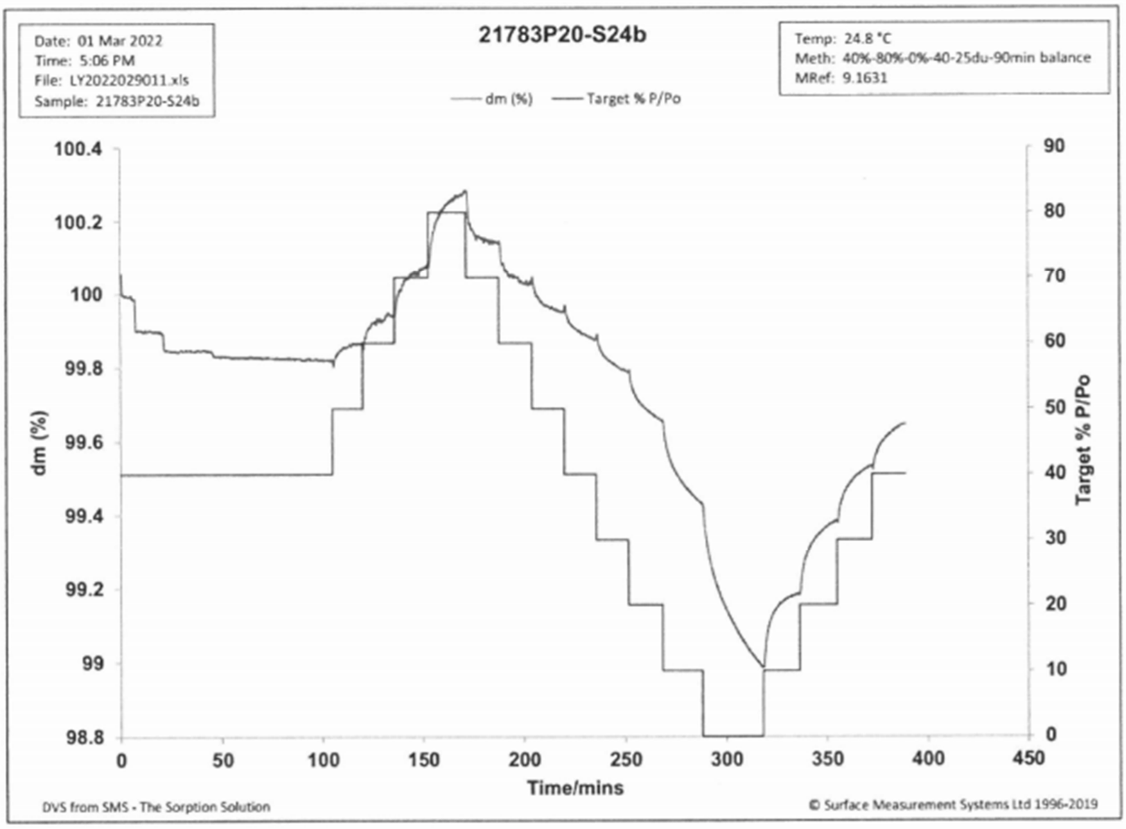
Date: 01 Mar 2022
Time: 5:06 PM
File: LY2022029011.xls
Sample: 21783P20-S24b
21783P20-S24b
Temp: 24.8 °C
Meth: 40%-80%-0%-40-25du-90min balance
MRef: 9.1631
dm (%)
Target % P/Po
dm (%)
Target % P/Po
Time/mins
DVS from SMS - The Sorption Solution
© Surface Measurement Systems Ltd 1996-2019

FIG.55

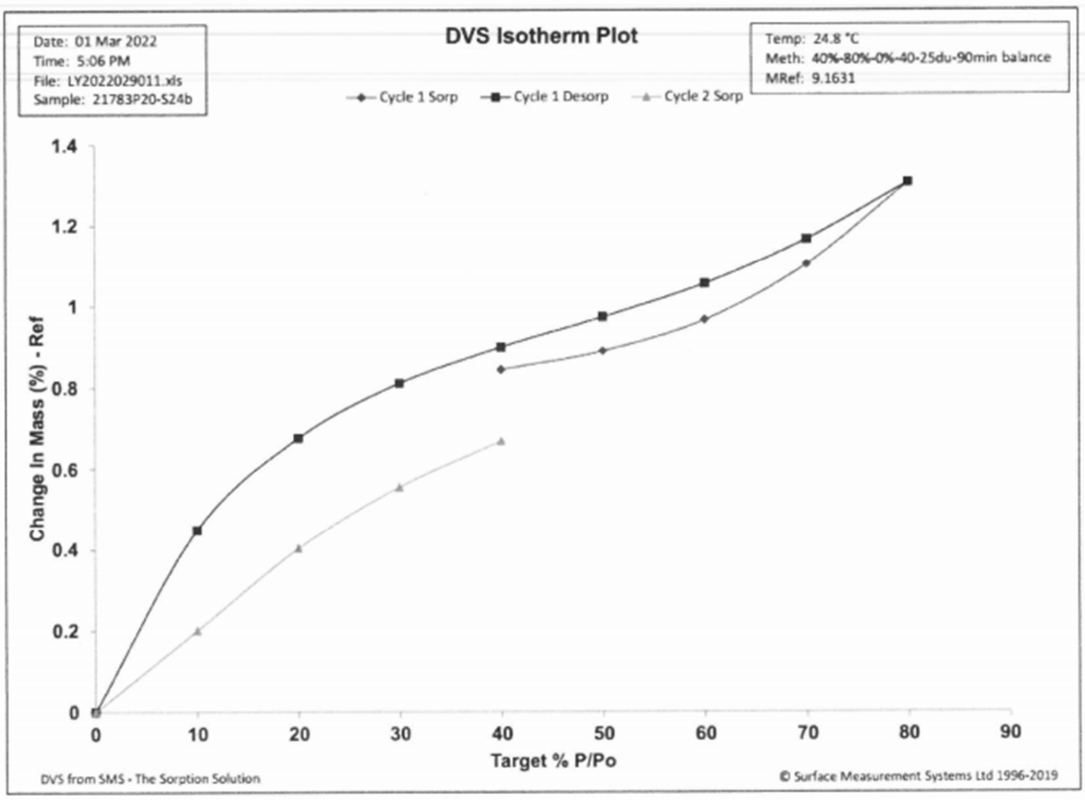
Date: 01 Mar 2022
Time: 5:06 PM
File: LY2022029011.xls
Sample: 21783P20-S24b
DVS Isotherm Plot
Temp: 24.8 °C
Meth: 40%-80%-0%-40-25du-90min balance
MRef: 9.1631
Cycle 1 Sorp
Cycle 1 Desorp
Cycle 2 Sorp
Change In Mass (%) - Ref
Target % P/Po
DVS from SMS - The Sorption Solution
© Surface Measurement Systems Ltd 1996-2019

FIG.56

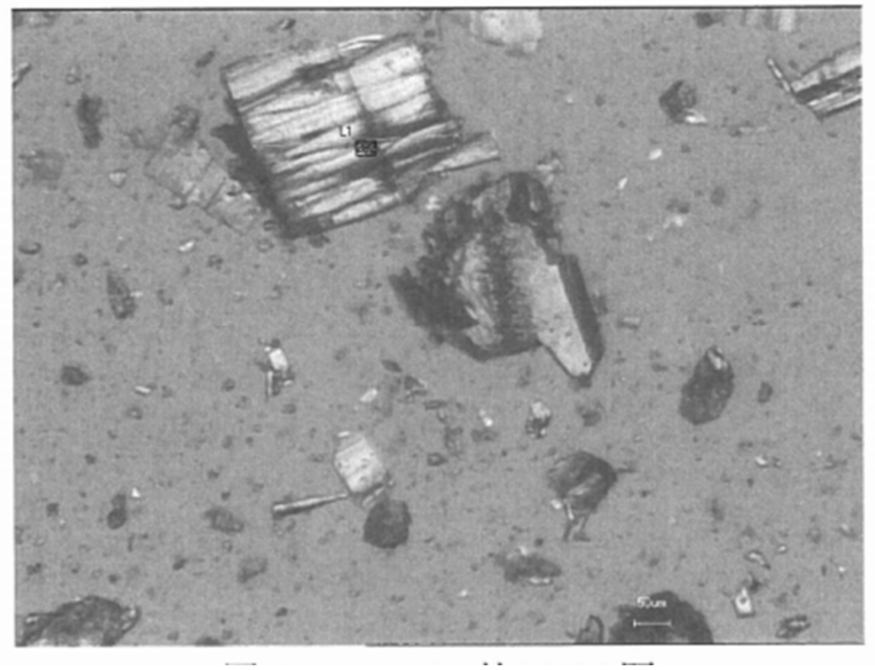

FIG.57

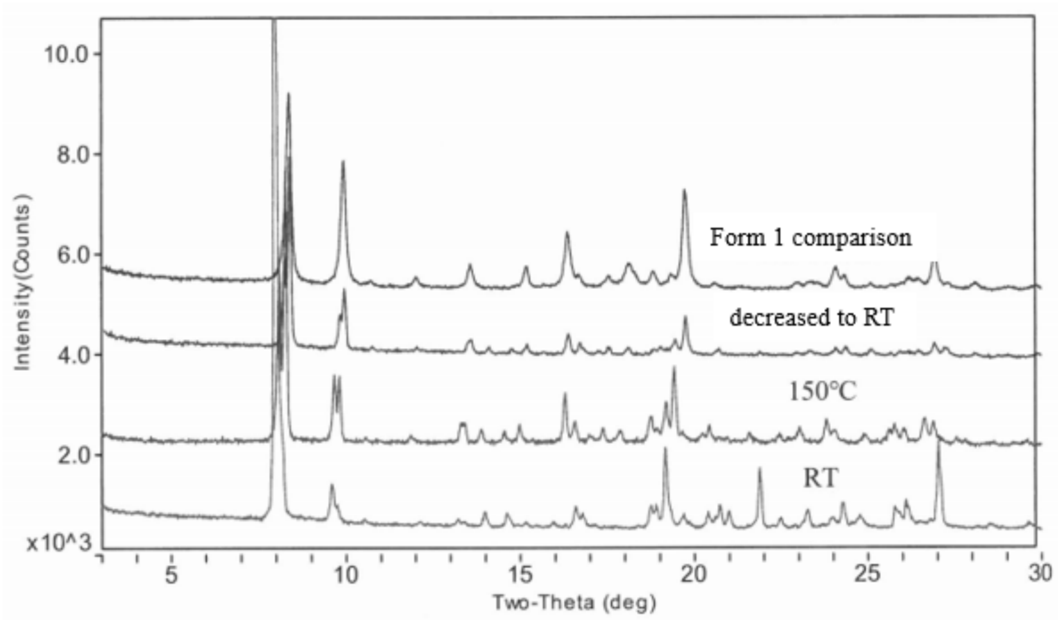

Intensity(Counts)
x10^3
10.0
8.0
6.0
4.0
2.0
Two-Theta (deg)
5
10
15
20
25
30
Form 1 comparison
decreased to RT
150°C
RT


FIG.58

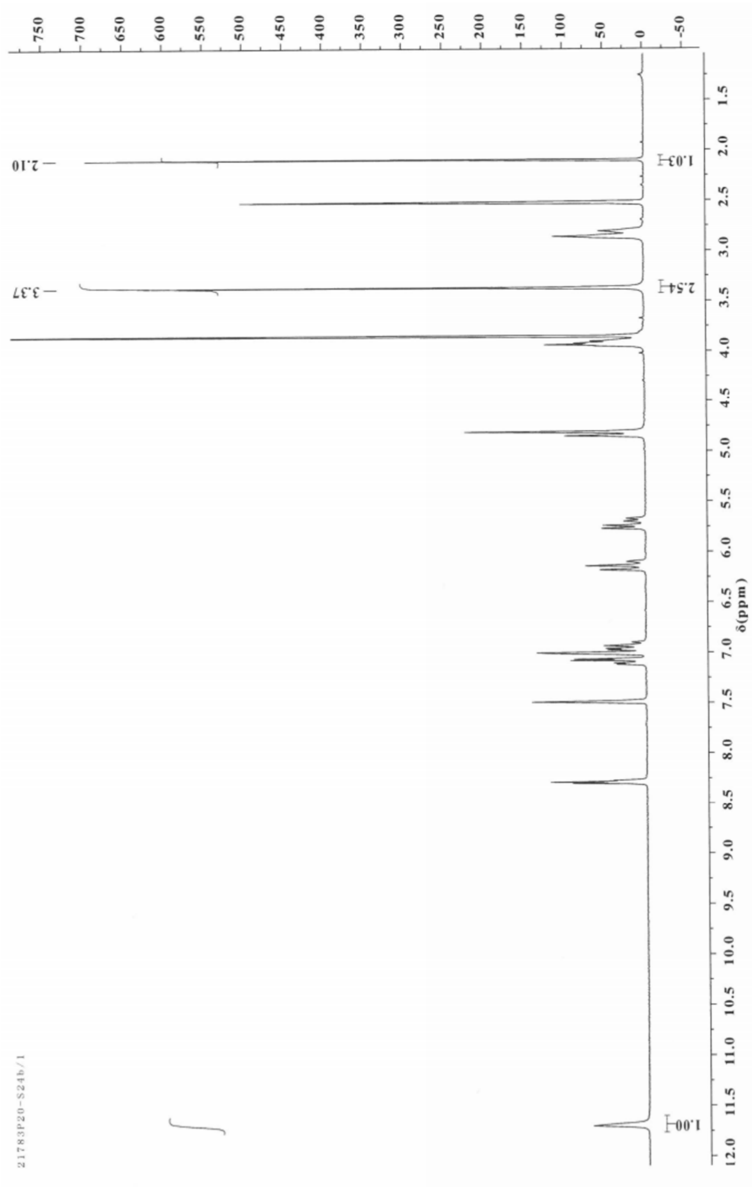

FIG.59

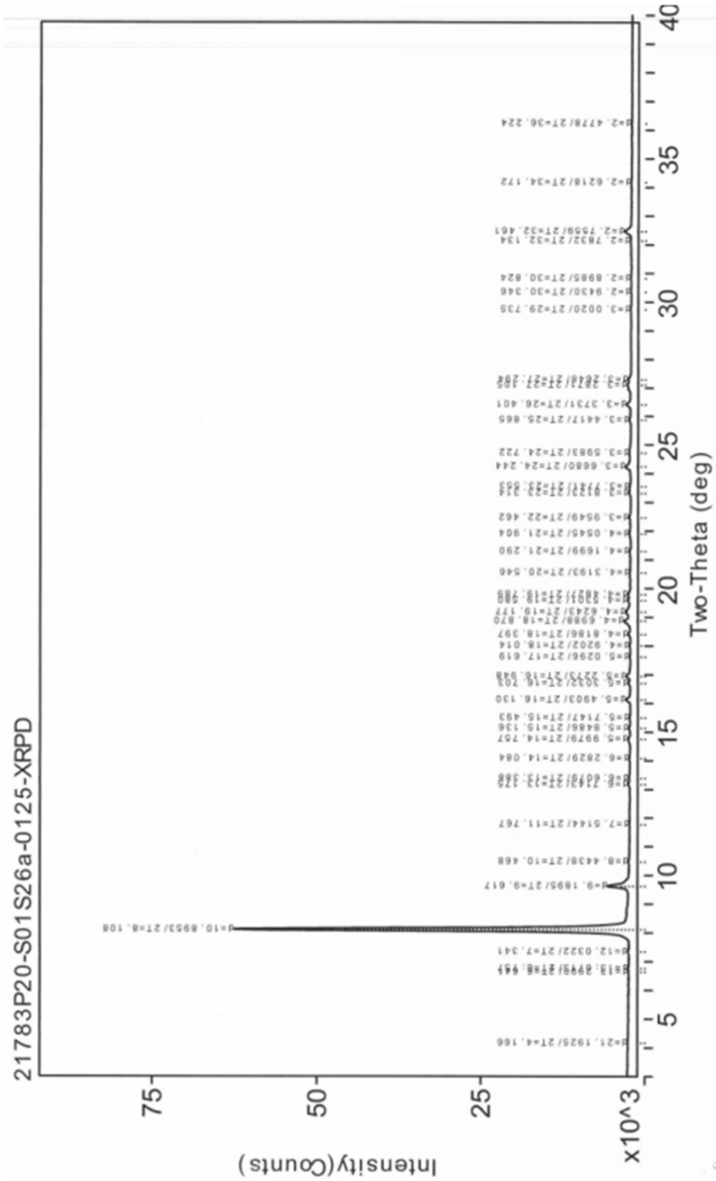
21783P20-S01S26a-0125-XRPD
Intensity(Counts)
x10^3
75
50
25
Two-Theta (deg)
5
10
15
20
25
30
35
40

FIG.60

| 21783P20-S01S26a-0125-XRPD | | | | | |
|---|---|---|---|---|---|
| 2-Theta | d | Height | I% | Area | I% |
| 4.166 | 21.1925 | 84 | 0.1 | 975 | 0.2 |
| 6.641 | 13.2990 | 101 | 0.2 | 695 | 0.2 |
| 6.757 | 13.0713 | 111 | 0.2 | 1030 | 0.2 |
| 7.341 | 12.0322 | 165 | 0.3 | 894 | 0.2 |
| 8.108 | 10.8953 | 60455 | 100 | 449607 | 100 |
| 9.617 | 9.1895 | 3194 | 5.3 | 27368 | 6.1 |
| 10.468 | 8.4438 | 115 | 0.2 | 752 | 0.2 |
| 11.767 | 7.5144 | 58 | 0.1 | 559 | 0.1 |
| 13.175 | 6.7143 | 244 | 0.4 | 2968 | 0.7 |
| 13.388 | 6.6079 | 160 | 0.3 | 1754 | 0.4 |
| 14.084 | 6.2829 | 109 | 0.2 | 805 | 0.2 |
| 14.757 | 5.9979 | 290 | 0.5 | 2665 | 0.6 |
| 15.136 | 5.8486 | 205 | 0.3 | 1524 | 0.3 |
| 15.493 | 5.7147 | 131 | 0.2 | 776 | 0.2 |
| 16.130 | 5.4903 | 646 | 1.1 | 4983 | 1.1 |
| 16.703 | 5.3032 | 260 | 0.4 | 1640 | 0.4 |
| 16.948 | 5.2273 | 578 | 1 | 6263 | 1.4 |
| 17.619 | 5.0296 | 107 | 0.2 | 695 | 0.2 |
| 18.014 | 4.9202 | 122 | 0.2 | 601 | 0.1 |
| 18.397 | 4.8186 | 276 | 0.5 | 1526 | 0.3 |
| 18.870 | 4.6988 | 855 | 1.4 | 8399 | 1.9 |
| 19.177 | 4.6243 | 553 | 0.9 | 7452 | 1.7 |
| 19.580 | 4.5301 | 255 | 0.4 | 2600 | 0.6 |
| 19.789 | 4.4827 | 314 | 0.5 | 3218 | 0.7 |
| 20.546 | 4.3193 | 84 | 0.1 | 1723 | 0.4 |
| 21.290 | 4.1699 | 182 | 0.3 | 1638 | 0.4 |
| 21.904 | 4.0545 | 212 | 0.4 | 2213 | 0.5 |
| 22.462 | 3.9549 | 234 | 0.4 | 2108 | 0.5 |
| 23.314 | 3.8123 | 230 | 0.4 | 3260 | 0.7 |
| 23.553 | 3.7741 | 155 | 0.3 | 3644 | 0.8 |
| 24.244 | 3.6680 | 698 | 1.2 | 6259 | 1.4 |
| 24.722 | 3.5983 | 145 | 0.2 | 2190 | 0.5 |
| 25.865 | 3.4417 | 203 | 0.3 | 1803 | 0.4 |
| 26.401 | 3.3731 | 568 | 0.9 | 4555 | 1 |
| 27.105 | 3.2871 | 491 | 0.8 | 8840 | 2 |
| 27.294 | 3.2648 | 386 | 0.6 | 4494 | 1 |
| 29.735 | 3.0020 | 105 | 0.2 | 2027 | 0.5 |
| 30.346 | 2.9430 | 62 | 0.1 | 501 | 0.1 |
| 30.824 | 2.8985 | 79 | 0.1 | 848 | 0.2 |
| 32.134 | 2.7832 | 71 | 0.1 | 718 | 0.2 |
| 32.461 | 2.7559 | 1002 | 1.7 | 10067 | 2.2 |
| 34.172 | 2.6218 | 77 | 0.1 | 816 | 0.2 |
| 36.224 | 2.4778 | 72 | 0.1 | 521 | 0.1 |

FIG.61

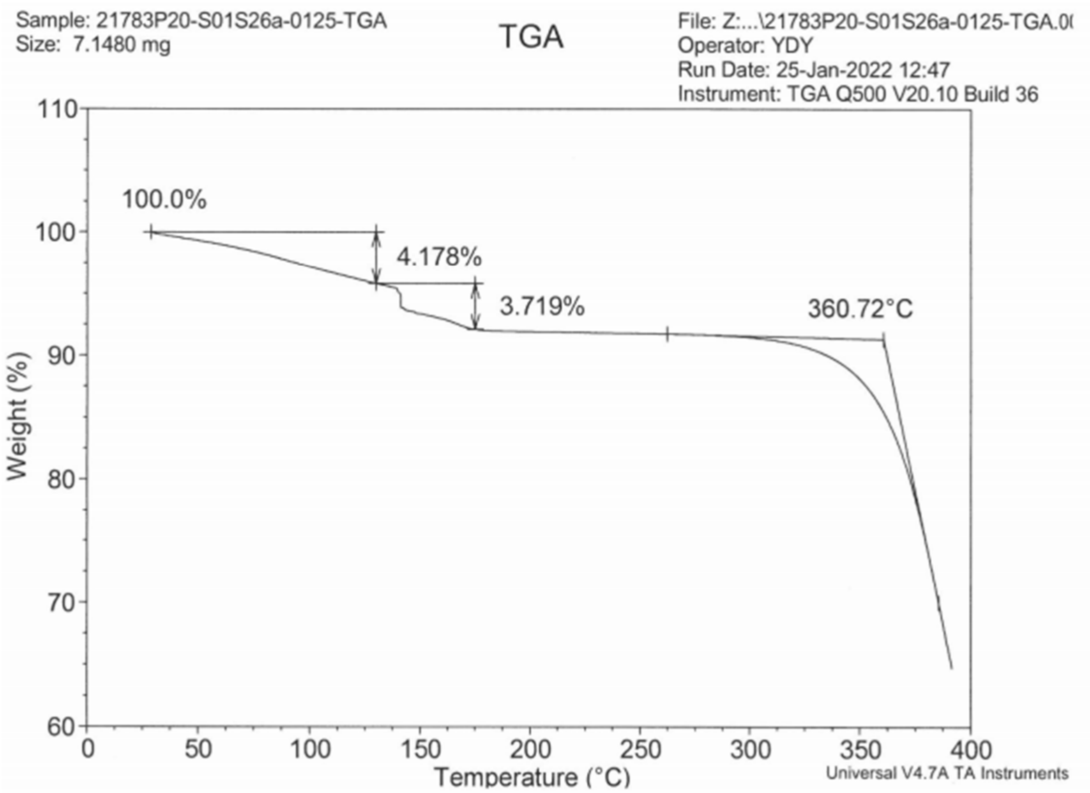
Sample: 21783P20-S01S26a-0125-TGA
Size: 7.1480 mg
TGA
File: Z:...\21783P20-S01S26a-0125-TGA.0
Operator: YDY
Run Date: 25-Jan-2022 12:47
Instrument: TGA Q500 V20.10 Build 36
100.0%
4.178%
3.719%
360.72°C
Weight (%)
Temperature (°C)
Universal V4.7A TA Instruments

FIG.62

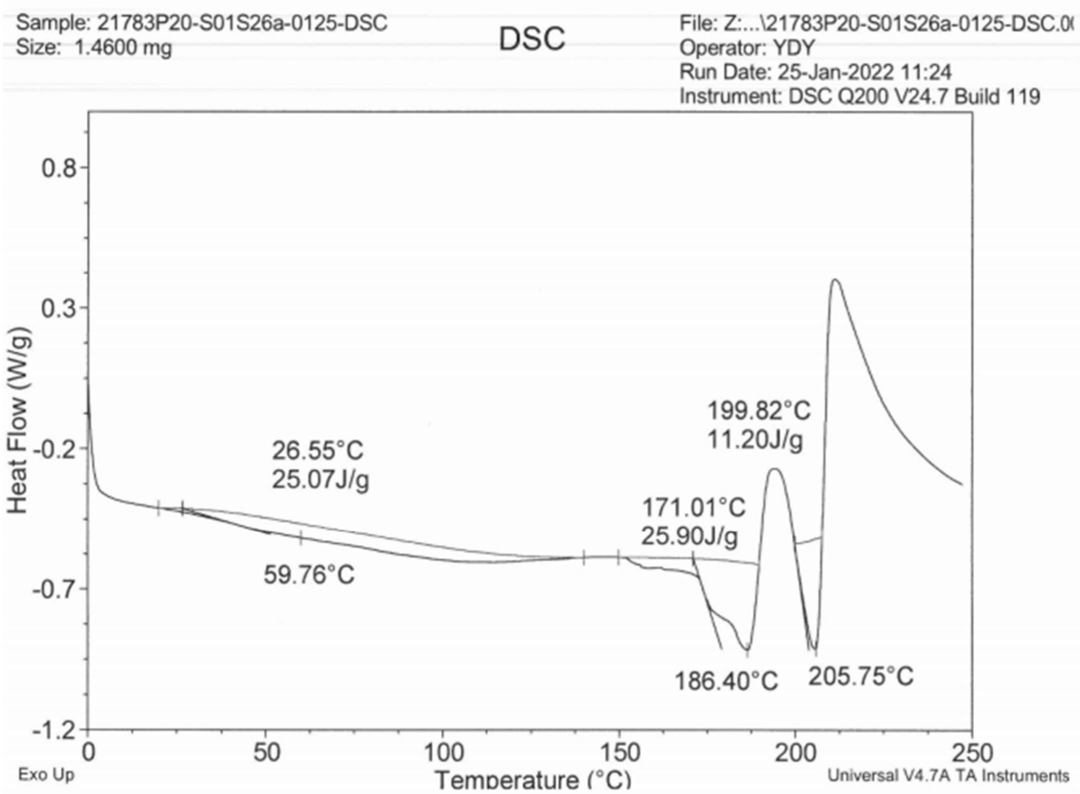
Sample: 21783P20-S01S26a-0125-DSC
Size: 1.4600 mg
DSC
File: Z:...\21783P20-S01S26a-0125-DSC.0
Operator: YDY
Run Date: 25-Jan-2022 11:24
Instrument: DSC Q200 V24.7 Build 119
26.55°C
25.07J/g
59.76°C
171.01°C
25.90J/g
199.82°C
11.20J/g
186.40°C
205.75°C
Heat Flow (W/g)
Exo Up
Temperature (°C)
Universal V4.7A TA Instruments

FIG.63

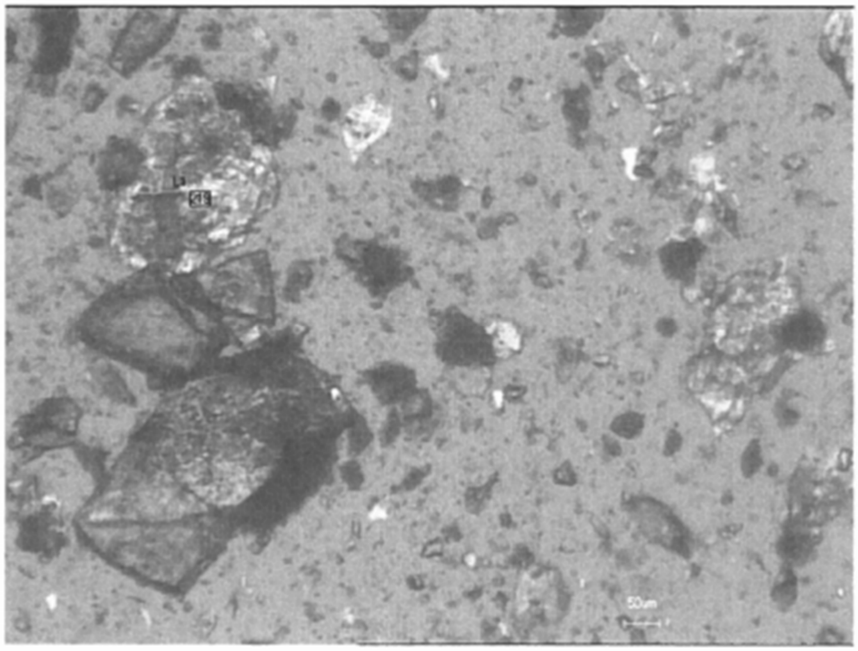

FIG.64

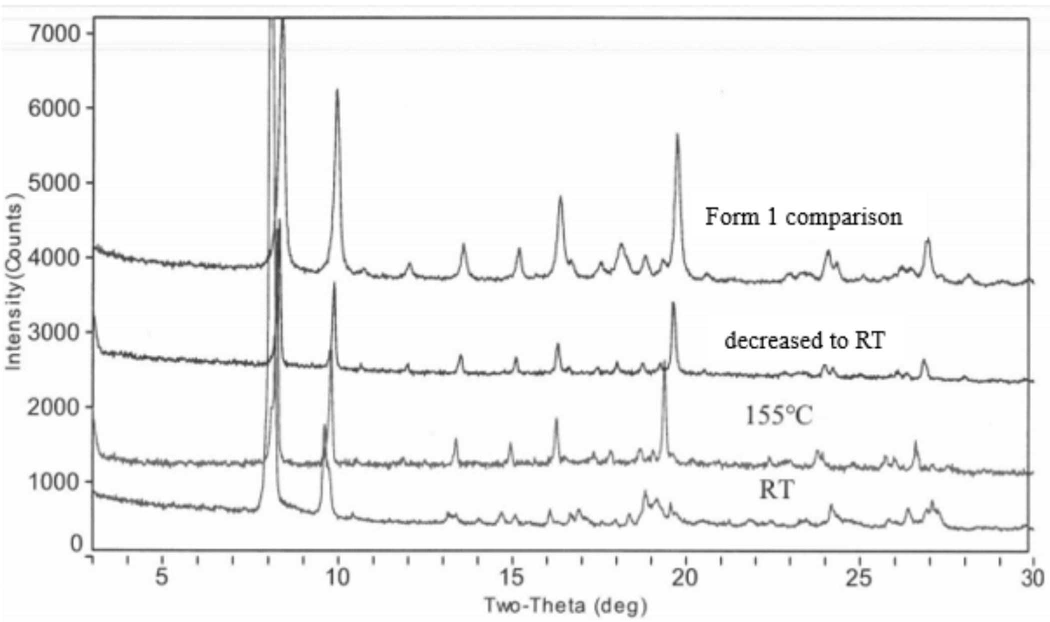
Intensity(Counts)
Two-Theta (deg)
Form 1 comparison
decreased to RT
155°C
RT

FIG.65

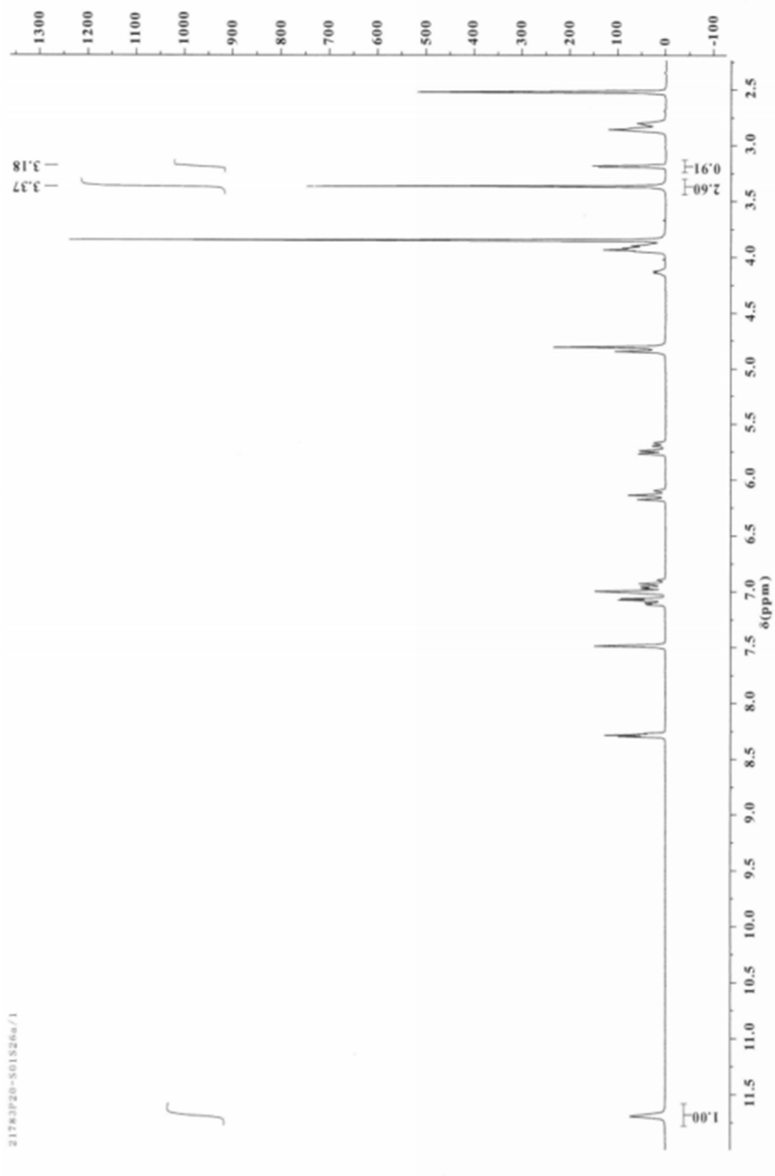

FIG.66

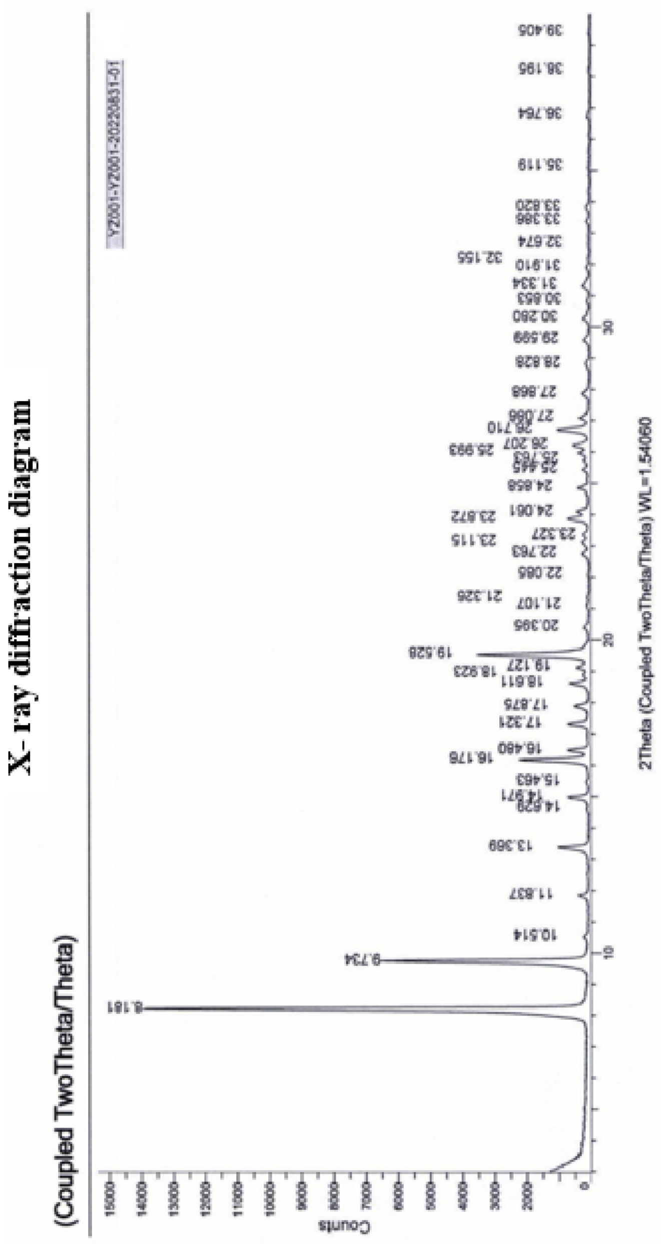
X- ray diffraction diagram
(Coupled TwoTheta/Theta)
Y2001-Y2001-20220831-01
Counts
2Theta (Coupled TwoTheta/Theta) WL=1.54060
8.181
9.734
10.514
11.837
13.369
14.629
14.971
15.463
16.176
16.480
17.321
17.875
18.611
18.923
19.127
19.528
20.395
21.107
21.326
22.085
22.763
23.115
23.327
23.872
24.061
24.858
25.445
25.763
25.993
26.207
26.710
27.086
27.868
28.828
29.599
30.280
30.853
31.334
31.910
32.155
32.674
33.386
33.820
35.119
36.764
38.195
39.405

FIG.67

## Peak List #1

| Index | Caption (display) | d Value | Net Intensity | Gross Intensity | Rel. Intensity | h,k,l |
|---|---|---|---|---|---|---|
| 1 | 8.181 | 10.79873 | 12018 | 12175 | 100.0 % | n.a. |
| 2 | 9.734 | 9.07898 | 5619 | 5753 | 46.8 % | n.a. |
| 3 | 10.514 | 8.40742 | 121 | 237 | 1.0 % | n.a. |
| 4 | 11.837 | 7.47030 | 272 | 368 | 2.3 % | n.a. |
| 5 | 13.369 | 6.61762 | 913 | 999 | 7.6 % | n.a. |
| 6 | 14.629 | 6.05039 | 48.5 | 131 | 0.4 % | n.a. |
| 7 | 14.971 | 5.91290 | 584 | 670 | 4.9 % | n.a. |
| 8 | 15.463 | 5.72580 | 55.6 | 143 | 0.5 % | n.a. |
| 9 | 16.176 | 5.47516 | 2117 | 2214 | 17.6 % | n.a. |
| 10 | 16.480 | 5.37460 | 602 | 703 | 5.0 % | n.a. |
| 11 | 17.321 | 5.11557 | 618 | 717 | 5.1 % | n.a. |
| 12 | 17.875 | 4.95821 | 427 | 518 | 3.6 % | n.a. |
| 13 | 18.611 | 4.76383 | 571 | 659 | 4.8 % | n.a. |
| 14 | 18.923 | 4.68600 | 127 | 218 | 1.1 % | n.a. |
| 15 | 19.127 | 4.63645 | 342 | 434 | 2.8 % | n.a. |
| 16 | 19.528 | 4.54219 | 3387 | 3478 | 28.2 % | n.a. |
| 17 | 20.395 | 4.35089 | 121 | 200 | 1.0 % | n.a. |
| 18 | 21.107 | 4.20573 | 61.5 | 127 | 0.5 % | n.a. |
| 19 | 21.326 | 4.16299 | 31.8 | 93.6 | 0.3 % | n.a. |
| 20 | 22.085 | 4.02175 | 23.9 | 78.7 | 0.2 % | n.a. |
| 21 | 22.763 | 3.90347 | 204 | 272 | 1.7 % | n.a. |
| 22 | 23.115 | 3.84468 | 192 | 269 | 1.6 % | n.a. |
| 23 | 23.327 | 3.81026 | 150 | 230 | 1.2 % | n.a. |
| 24 | 23.872 | 3.72451 | 634 | 719 | 5.3 % | n.a. |
| 25 | 24.061 | 3.69564 | 290 | 375 | 2.4 % | n.a. |
| 26 | 24.858 | 3.57903 | 300 | 395 | 2.5 % | n.a. |
| 27 | 25.445 | 3.49772 | 139 | 244 | 1.2 % | n.a. |
| 28 | 25.763 | 3.45531 | 99.4 | 207 | 0.8 % | n.a. |
| 29 | 25.993 | 3.42520 | 229 | 337 | 1.9 % | n.a. |
| 30 | 26.207 | 3.39767 | 437 | 545 | 3.6 % | n.a. |
| 31 | 26.710 | 3.33483 | 928 | 1029 | 7.7 % | n.a. |
| 32 | 27.086 | 3.28939 | 269 | 362 | 2.2 % | n.a. |
| 33 | 27.868 | 3.19890 | 200 | 271 | 1.7 % | n.a. |
| 34 | 28.828 | 3.09452 | 93.5 | 148 | 0.8 % | n.a. |
| 35 | 29.599 | 3.01557 | 155 | 211 | 1.3 % | n.a. |
| 36 | 30.280 | 2.94932 | 174 | 234 | 1.5 % | n.a. |
| 37 | 30.853 | 2.89580 | 41.8 | 108 | 0.3 % | n.a. |
| 38 | 31.334 | 2.85246 | 186 | 253 | 1.5 % | n.a. |
| 39 | 31.910 | 2.80233 | 78.3 | 139 | 0.7 % | n.a. |
| 40 | 32.155 | 2.78148 | 30.8 | 86.6 | 0.3 % | n.a. |
| 41 | 32.674 | 2.73851 | 31.2 | 84.2 | 0.3 % | n.a. |
| 42 | 33.386 | 2.68167 | 82.4 | 140 | 0.7 % | n.a. |
| 43 | 33.820 | 2.64824 | 87.1 | 142 | 0.7 % | n.a. |
| 44 | 35.119 | 2.55321 | 35.9 | 76.8 | 0.3 % | n.a. |
| 45 | 36.764 | 2.44269 | 52.1 | 88.9 | 0.4 % | n.a. |
| 46 | 38.195 | 2.35437 | 19.1 | 57.1 | 0.2 % | n.a. |
| 47 | 39.405 | 2.28485 | 26.5 | 65.9 | 0.2 % | n.a. |

FIG.68

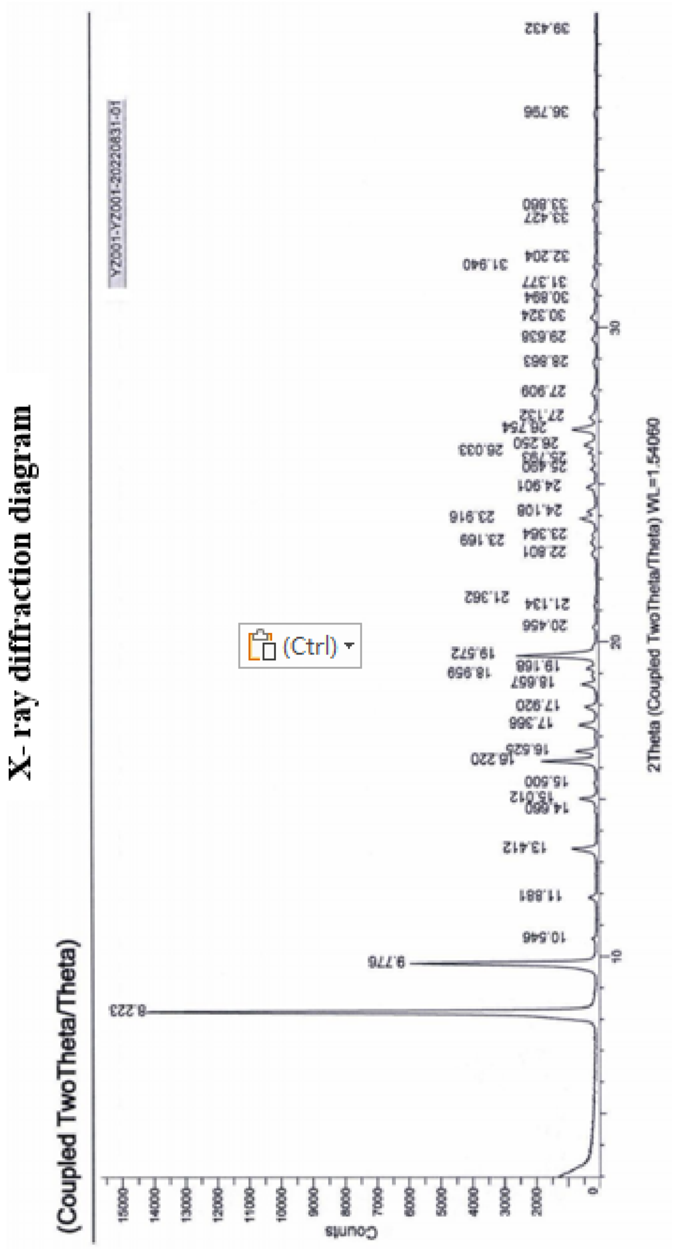
X- ray diffraction diagram
(Coupled TwoTheta/Theta)
YZ001-YZ001-20220831-01
Counts
2Theta (Coupled TwoTheta/Theta) WL=1.54060
(Ctrl)
8.223
9.776
10.546
11.881
13.412
14.660
15.012
15.500
16.220
16.525
17.366
17.920
18.657
18.959
19.168
19.572
20.456
21.134
21.362
22.801
23.169
23.364
23.916
24.108
24.901
25.490
25.793
26.033
26.250
26.754
27.132
27.909
28.863
29.638
30.324
30.884
31.377
31.940
32.204
33.427
33.860
36.796
39.432

FIG.69

## Peak List #1

| Index | Caption (display) | d Value | Net Intensity | Gross Intensity | Rel. Intensity | h,k,l |
|---|---|---|---|---|---|---|
| 1 | 8.223 | 10.74354 | 12299 | 12417 | 100.0 % | n.a. |
| 2 | 9.776 | 9.03999 | 5200 | 5305 | 42.3 % | n.a. |
| 3 | 10.546 | 8.38150 | 104 | 196 | 0.8 % | n.a. |
| 4 | 11.881 | 7.44274 | 246 | 326 | 2.0 % | n.a. |
| 5 | 13.412 | 6.59670 | 723 | 797 | 5.9 % | n.a. |
| 6 | 14.660 | 6.03746 | 35.4 | 103 | 0.3 % | n.a. |
| 7 | 15.012 | 5.89688 | 511 | 581 | 4.2 % | n.a. |
| 8 | 15.500 | 5.71238 | 40.2 | 112 | 0.3 % | n.a. |
| 9 | 16.220 | 5.46018 | 1721 | 1799 | 14.0 % | n.a. |
| 10 | 16.525 | 5.36014 | 623 | 702 | 5.1 % | n.a. |
| 11 | 17.366 | 5.10231 | 520 | 595 | 4.2 % | n.a. |
| 12 | 17.920 | 4.94595 | 301 | 366 | 2.4 % | n.a. |
| 13 | 18.657 | 4.75222 | 469 | 534 | 3.8 % | n.a. |
| 14 | 18.959 | 4.67722 | 108 | 177 | 0.9 % | n.a. |
| 15 | 19.168 | 4.62661 | 286 | 356 | 2.3 % | n.a. |
| 16 | 19.572 | 4.53199 | 2341 | 2412 | 19.0 % | n.a. |
| 17 | 20.456 | 4.33819 | 109 | 166 | 0.9 % | n.a. |
| 18 | 21.134 | 4.20038 | 33.9 | 85.8 | 0.3 % | n.a. |
| 19 | 21.362 | 4.15618 | 29.4 | 78.6 | 0.2 % | n.a. |
| 20 | 22.801 | 3.89692 | 127 | 183 | 1.0 % | n.a. |
| 21 | 23.169 | 3.83592 | 154 | 216 | 1.2 % | n.a. |
| 22 | 23.364 | 3.80428 | 102 | 167 | 0.8 % | n.a. |
| 23 | 23.916 | 3.71769 | 471 | 538 | 3.8 % | n.a. |
| 24 | 24.108 | 3.68857 | 248 | 314 | 2.0 % | n.a. |
| 25 | 24.901 | 3.57293 | 280 | 346 | 2.3 % | n.a. |
| 26 | 25.490 | 3.49167 | 97.8 | 167 | 0.8 % | n.a. |
| 27 | 25.793 | 3.45133 | 101 | 175 | 0.8 % | n.a. |
| 28 | 26.033 | 3.42009 | 174 | 250 | 1.4 % | n.a. |
| 29 | 26.250 | 3.39223 | 349 | 426 | 2.8 % | n.a. |
| 30 | 26.754 | 3.32948 | 720 | 796 | 5.9 % | n.a. |
| 31 | 27.132 | 3.28393 | 174 | 244 | 1.4 % | n.a. |
| 32 | 27.909 | 3.19428 | 135 | 188 | 1.1 % | n.a. |
| 33 | 28.863 | 3.09077 | 77.0 | 116 | 0.6 % | n.a. |
| 34 | 29.638 | 3.01177 | 138 | 180 | 1.1 % | n.a. |
| 35 | 30.324 | 2.94512 | 138 | 187 | 1.1 % | n.a. |
| 36 | 30.894 | 2.89211 | 39.5 | 91.3 | 0.3 % | n.a. |
| 37 | 31.377 | 2.84867 | 104 | 156 | 0.8 % | n.a. |
| 38 | 31.940 | 2.79976 | 58.9 | 106 | 0.5 % | n.a. |
| 39 | 32.204 | 2.77741 | 27.0 | 69.9 | 0.2 % | n.a. |
| 40 | 33.427 | 2.67848 | 41.1 | 88.8 | 0.3 % | n.a. |
| 41 | 33.860 | 2.64520 | 88.2 | 132 | 0.7 % | n.a. |
| 42 | 36.796 | 2.44062 | 54.4 | 83.5 | 0.4 % | n.a. |
| 43 | 39.432 | 2.28333 | 20.0 | 50.9 | 0.2 % | n.a. |

FIG.70

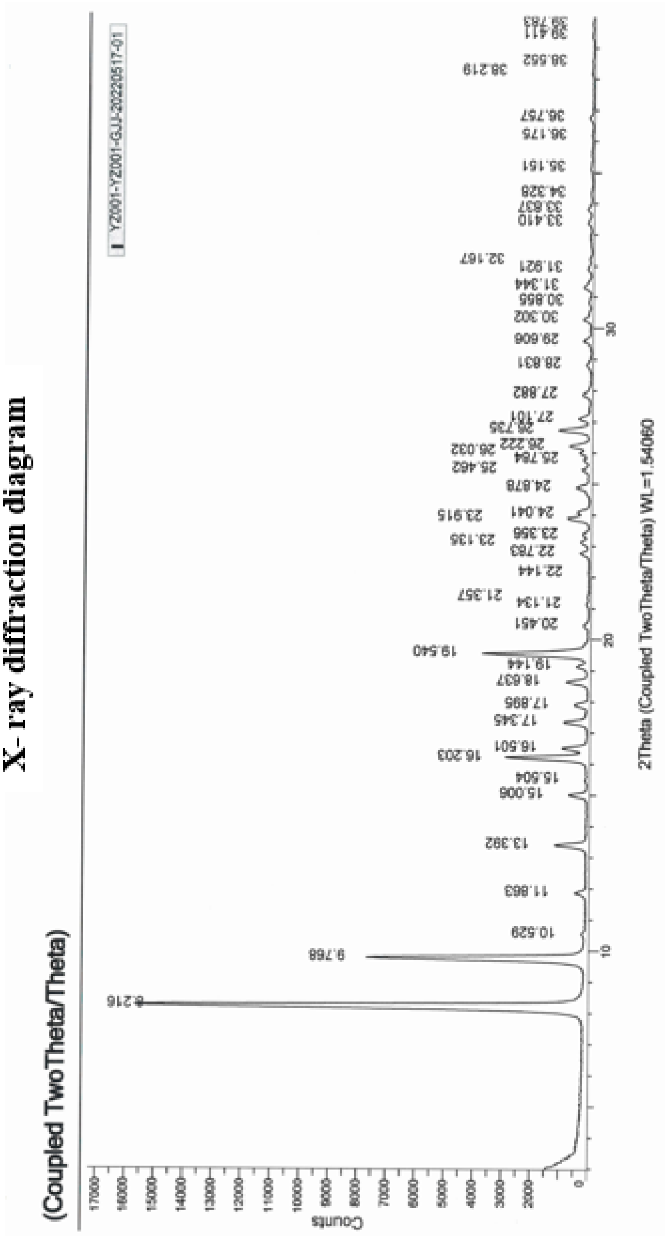
X- ray diffraction diagram
(Coupled TwoTheta/Theta)
YZ001-YZ001-GJJ-20220517-01
Counts
2Theta (Coupled TwoTheta/Theta) WL=1.54060
8.216
9.768
10.529
11.863
13.382
15.006
15.504
16.203
16.501
17.345
17.885
18.637
19.144
19.540
20.451
21.134
21.357
22.144
22.783
23.135
23.356
23.915
24.041
24.878
25.462
25.784
26.032
26.222
26.735
27.101
27.882
28.831
29.606
30.302
30.855
31.344
31.921
32.167
33.410
33.837
34.328
35.151
36.175
36.757
38.219
38.552
39.411
39.783

FIG.71

## Peak List #1

| Index | Caption (display) | d Value | Net Intensity | Gross Intensity | Rel. Intensity | h,k,l |
|---|---|---|---|---|---|---|
| 1 | 8.216 | 10.75283 | 15150 | 15332 | 100.0 % | n.a. |
| 2 | 9.768 | 9.04750 | 7377 | 7530 | 48.7 % | n.a. |
| 3 | 10.529 | 8.39520 | 95.8 | 229 | 0.6 % | n.a. |
| 4 | 11.863 | 7.45400 | 367 | 476 | 2.4 % | n.a. |
| 5 | 13.392 | 6.60621 | 1070 | 1176 | 7.1 % | n.a. |
| 6 | 15.006 | 5.89918 | 586 | 682 | 3.9 % | n.a. |
| 7 | 15.504 | 5.71088 | 27.1 | 126 | 0.2 % | n.a. |
| 8 | 16.203 | 5.46603 | 2739 | 2844 | 18.1 % | n.a. |
| 9 | 16.501 | 5.36787 | 823 | 927 | 5.4 % | n.a. |
| 10 | 17.345 | 5.10867 | 802 | 902 | 5.3 % | n.a. |
| 11 | 17.895 | 4.95269 | 425 | 524 | 2.8 % | n.a. |
| 12 | 18.637 | 4.75722 | 726 | 823 | 4.8 % | n.a. |
| 13 | 19.144 | 4.63240 | 336 | 437 | 2.2 % | n.a. |
| 14 | 19.540 | 4.53941 | 3617 | 3717 | 23.9 % | n.a. |
| 15 | 20.451 | 4.33915 | 160 | 241 | 1.1 % | n.a. |
| 16 | 21.134 | 4.20045 | 80.1 | 150 | 0.5 % | n.a. |
| 17 | 21.357 | 4.15718 | 31.5 | 99.5 | 0.2 % | n.a. |
| 18 | 22.144 | 4.01102 | 25.3 | 95.0 | 0.2 % | n.a. |
| 19 | 22.783 | 3.89999 | 295 | 376 | 1.9 % | n.a. |
| 20 | 23.135 | 3.84153 | 279 | 365 | 1.8 % | n.a. |
| 21 | 23.356 | 3.80561 | 188 | 275 | 1.2 % | n.a. |
| 22 | 23.915 | 3.71786 | 730 | 816 | 4.8 % | n.a. |
| 23 | 24.041 | 3.69869 | 331 | 414 | 2.2 % | n.a. |
| 24 | 24.878 | 3.57610 | 437 | 528 | 2.9 % | n.a. |
| 25 | 25.462 | 3.49546 | 229 | 332 | 1.5 % | n.a. |
| 26 | 25.784 | 3.45245 | 169 | 274 | 1.1 % | n.a. |
| 27 | 26.032 | 3.42014 | 267 | 373 | 1.8 % | n.a. |
| 28 | 26.222 | 3.39583 | 657 | 763 | 4.3 % | n.a. |
| 29 | 26.735 | 3.33176 | 1033 | 1133 | 6.8 % | n.a. |
| 30 | 27.101 | 3.28760 | 366 | 457 | 2.4 % | n.a. |
| 31 | 27.882 | 3.19734 | 255 | 327 | 1.7 % | n.a. |
| 32 | 28.831 | 3.09414 | 147 | 208 | 1.0 % | n.a. |
| 33 | 29.606 | 3.01491 | 224 | 292 | 1.5 % | n.a. |
| 34 | 30.302 | 2.94719 | 239 | 315 | 1.6 % | n.a. |
| 35 | 30.855 | 2.89569 | 57.5 | 137 | 0.4 % | n.a. |
| 36 | 31.344 | 2.85156 | 229 | 305 | 1.5 % | n.a. |
| 37 | 31.921 | 2.80139 | 118 | 185 | 0.8 % | n.a. |
| 38 | 32.167 | 2.78046 | 50.2 | 111 | 0.3 % | n.a. |
| 39 | 33.410 | 2.67983 | 125 | 190 | 0.8 % | n.a. |
| 40 | 33.837 | 2.64695 | 148 | 210 | 1.0 % | n.a. |
| 41 | 34.328 | 2.61023 | 39.0 | 92.9 | 0.3 % | n.a. |
| 42 | 35.151 | 2.55095 | 48.5 | 103 | 0.3 % | n.a. |
| 43 | 36.175 | 2.48111 | 18.0 | 66.4 | 0.1 % | n.a. |
| 44 | 36.757 | 2.44312 | 105 | 148 | 0.7 % | n.a. |
| 45 | 38.219 | 2.35298 | 17.6 | 64.4 | 0.1 % | n.a. |
| 46 | 38.552 | 2.33342 | 29.0 | 73.0 | 0.2 % | n.a. |
| 47 | 39.411 | 2.28448 | 28.3 | 71.8 | 0.2 % | n.a. |
| 48 | 39.783 | 2.26400 | 21.2 | 66.8 | 0.1 % | n.a. |

FIG.72

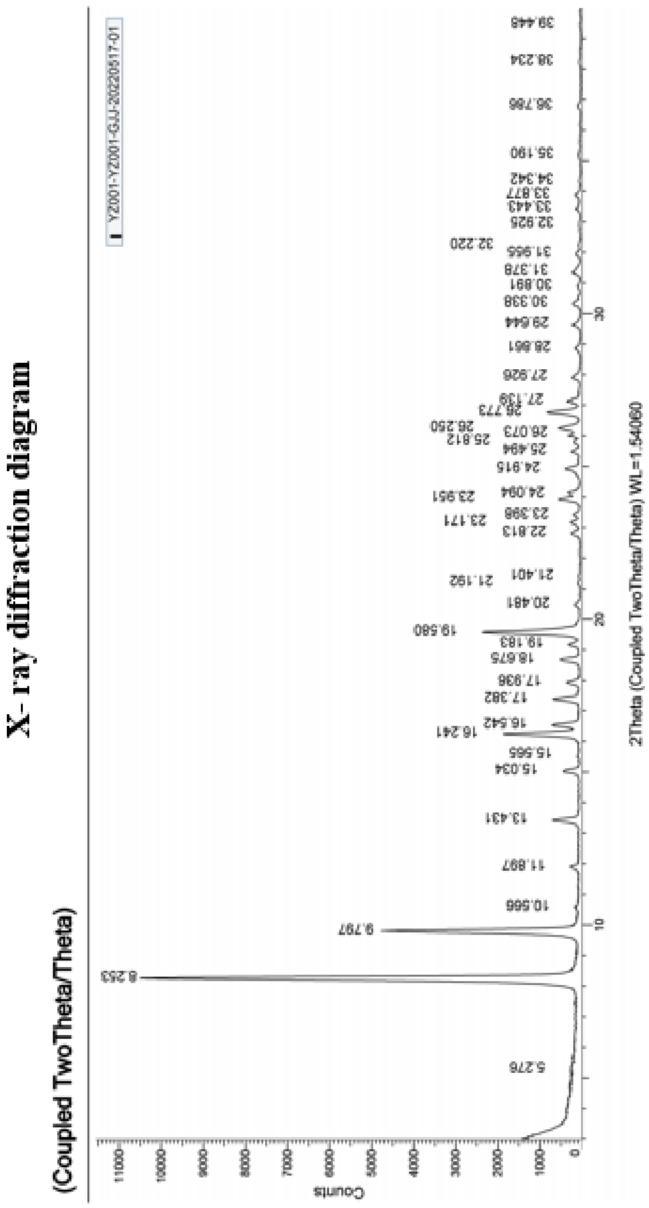
X- ray diffraction diagram
(Coupled TwoTheta/Theta)
YZ001-YZ001-GJJ-20220517-01
Counts
2Theta (Coupled TwoTheta/Theta) WL=1.54060

FIG.73

## Peak List #1

| Index | Caption (display) | d Value | Net Intensity | Gross Intensity | Rel. Intensity | h,k,l |
|---|---|---|---|---|---|---|
| 1 | 5.276 | 16.73500 | 45.4 | 273 | 0.4 % | n.a. |
| 2 | 8.253 | 10.70455 | 10130 | 10275 | 100.0 % | n.a. |
| 3 | 9.797 | 9.02052 | 4235 | 4349 | 41.8 % | n.a. |
| 4 | 10.566 | 8.36596 | 63.8 | 161 | 0.6 % | n.a. |
| 5 | 11.897 | 7.43256 | 184 | 266 | 1.8 % | n.a. |
| 6 | 13.431 | 6.58718 | 612 | 687 | 6.0 % | n.a. |
| 7 | 15.034 | 5.88827 | 364 | 436 | 3.6 % | n.a. |
| 8 | 15.565 | 5.68841 | 22.2 | 94.2 | 0.2 % | n.a. |
| 9 | 16.241 | 5.45318 | 1776 | 1854 | 17.5 % | n.a. |
| 10 | 16.542 | 5.35472 | 620 | 698 | 6.1 % | n.a. |
| 11 | 17.382 | 5.09783 | 605 | 678 | 6.0 % | n.a. |
| 12 | 17.936 | 4.94149 | 287 | 354 | 2.8 % | n.a. |
| 13 | 18.675 | 4.74750 | 450 | 519 | 4.4 % | n.a. |
| 14 | 19.183 | 4.62313 | 238 | 309 | 2.4 % | n.a. |
| 15 | 19.580 | 4.53023 | 2289 | 2358 | 22.6 % | n.a. |
| 16 | 20.481 | 4.33297 | 109 | 166 | 1.1 % | n.a. |
| 17 | 21.192 | 4.18906 | 48.1 | 98.8 | 0.5 % | n.a. |
| 18 | 21.401 | 4.14873 | 22.8 | 72.1 | 0.2 % | n.a. |
| 19 | 22.813 | 3.89491 | 197 | 250 | 1.9 % | n.a. |
| 20 | 23.171 | 3.83560 | 201 | 260 | 2.0 % | n.a. |
| 21 | 23.398 | 3.79895 | 131 | 192 | 1.3 % | n.a. |
| 22 | 23.951 | 3.71241 | 475 | 537 | 4.7 % | n.a. |
| 23 | 24.094 | 3.69063 | 226 | 287 | 2.2 % | n.a. |
| 24 | 24.915 | 3.57084 | 322 | 390 | 3.2 % | n.a. |
| 25 | 25.494 | 3.49106 | 167 | 246 | 1.7 % | n.a. |
| 26 | 25.812 | 3.44876 | 90.3 | 171 | 0.9 % | n.a. |
| 27 | 26.073 | 3.41493 | 136 | 218 | 1.3 % | n.a. |
| 28 | 26.250 | 3.39228 | 474 | 555 | 4.7 % | n.a. |
| 29 | 26.773 | 3.32715 | 745 | 823 | 7.4 % | n.a. |
| 30 | 27.139 | 3.28308 | 249 | 320 | 2.5 % | n.a. |
| 31 | 27.926 | 3.19239 | 172 | 226 | 1.7 % | n.a. |
| 32 | 28.861 | 3.09098 | 98.2 | 140 | 1.0 % | n.a. |
| 33 | 29.644 | 3.01112 | 144 | 186 | 1.4 % | n.a. |
| 34 | 30.338 | 2.94383 | 169 | 214 | 1.7 % | n.a. |
| 35 | 30.891 | 2.89237 | 58.5 | 107 | 0.6 % | n.a. |
| 36 | 31.378 | 2.84857 | 152 | 202 | 1.5 % | n.a. |
| 37 | 31.955 | 2.79843 | 86.3 | 132 | 0.9 % | n.a. |
| 38 | 32.220 | 2.77604 | 40.7 | 82.6 | 0.4 % | n.a. |
| 39 | 32.925 | 2.71819 | 19.7 | 65.6 | 0.2 % | n.a. |
| 40 | 33.443 | 2.67727 | 76.1 | 124 | 0.8 % | n.a. |
| 41 | 33.877 | 2.64392 | 114 | 160 | 1.1 % | n.a. |
| 42 | 34.342 | 2.60921 | 33.1 | 71.6 | 0.3 % | n.a. |
| 43 | 35.190 | 2.54824 | 25.1 | 65.3 | 0.2 % | n.a. |
| 44 | 36.786 | 2.44130 | 62.1 | 96.6 | 0.6 % | n.a. |
| 45 | 38.234 | 2.35210 | 36.8 | 70.3 | 0.4 % | n.a. |
| 46 | 39.448 | 2.28246 | 16.4 | 48.3 | 0.2 % | n.a. |

FIG.74

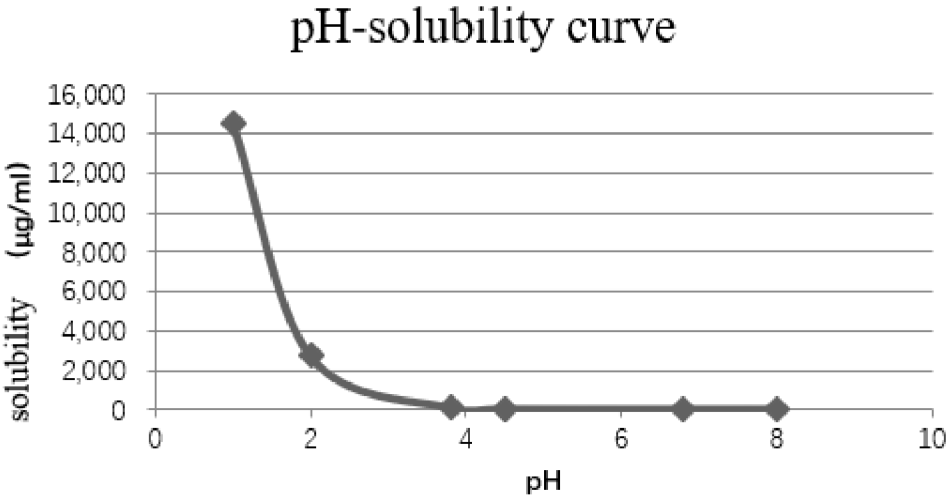


FIG.75

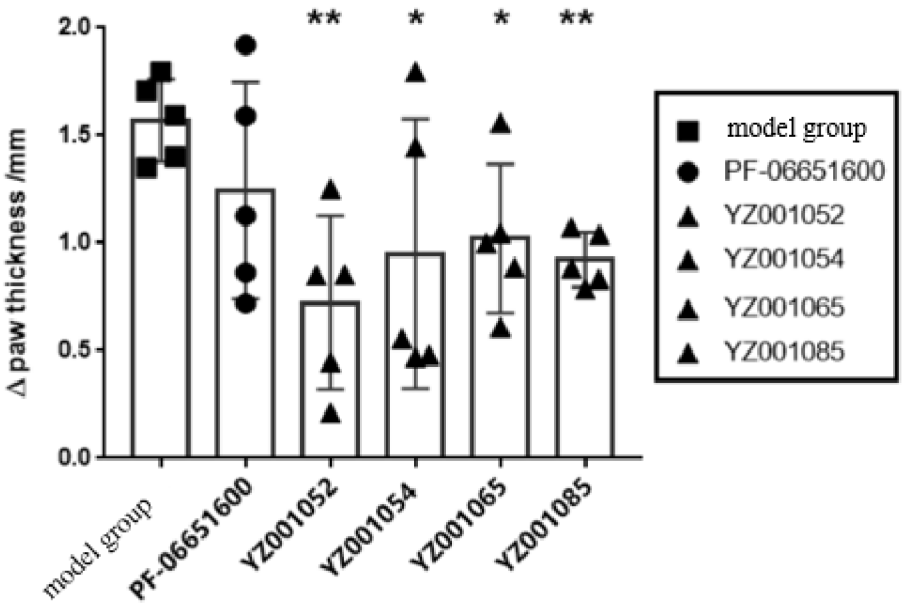


Note: Compared with the model group, *P<0.05, **P<0.01

FIG.76

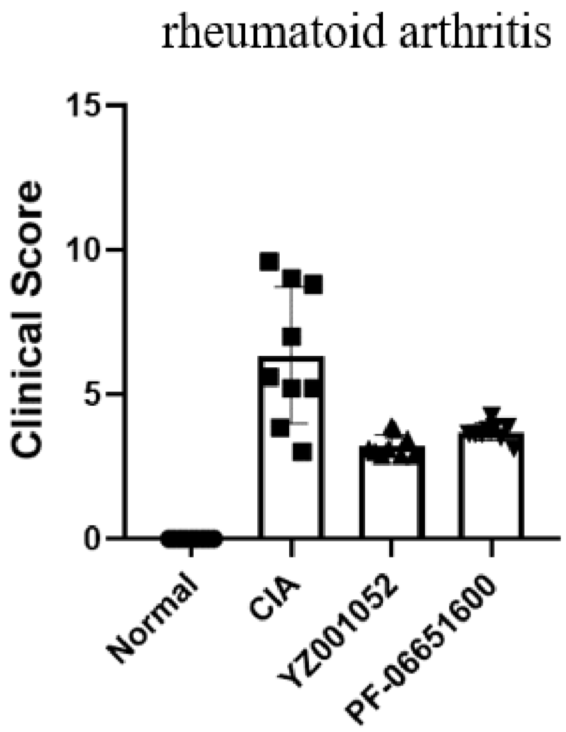
rheumatoid arthritis
Clinical Score
15
10
5
0
Normal
CIA
YZ001052
PF-0665160

FIG.77

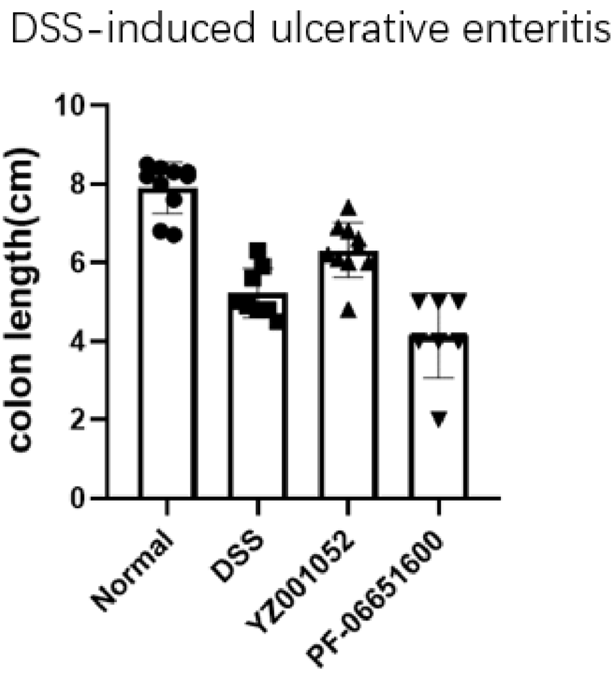
DSS-induced ulcerative enteritis
colon length(cm)
10
8
6
4
2
0
Normal
DSS
YZ001052
PF-06651600

FIG.78

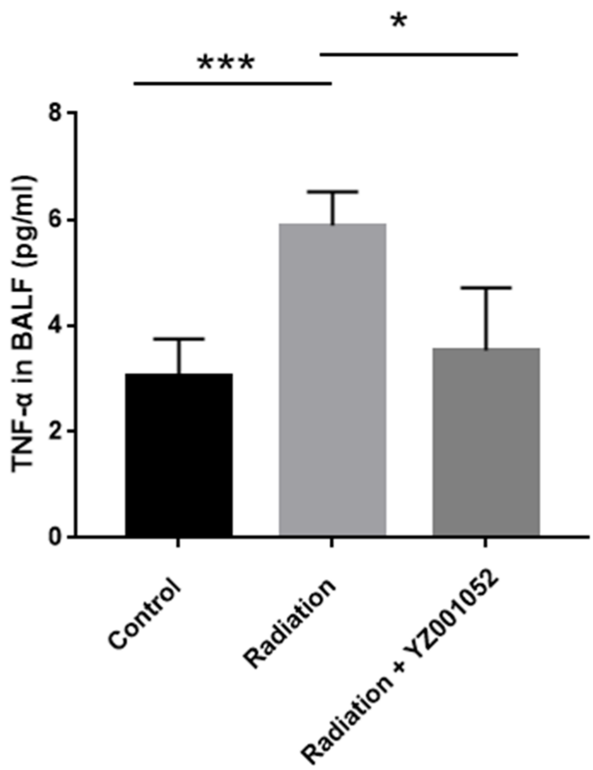
***
*
TNF-α in BALF (pg/ml)
0
2
4
6
8
Control
Radiation
Radiation + YZ001052

FIG.79

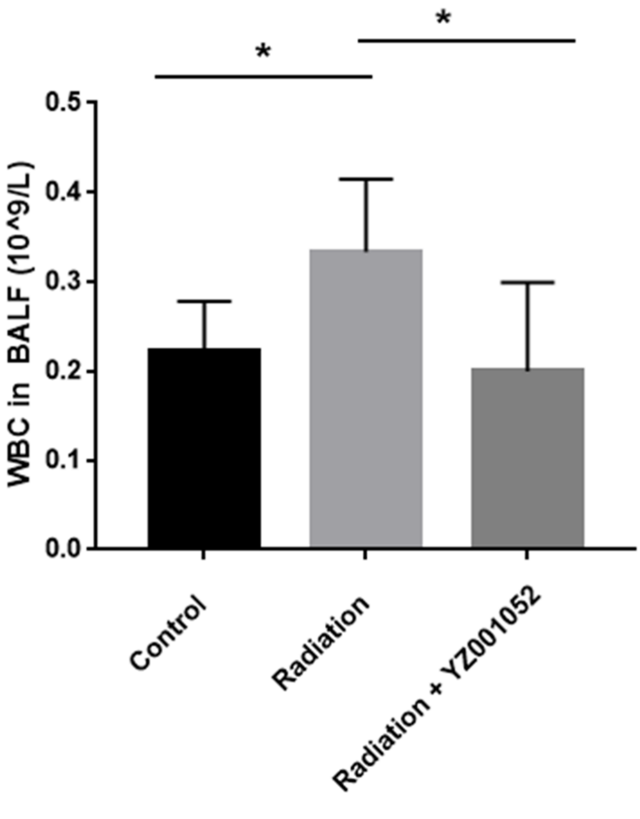
*
*
WBC in BALF (10^9/L)
0.0
0.1
0.2
0.3
0.4
0.5
Control
Radiation
Radiation + YZ001052

FIG.80

**INTERNATIONAL SEARCH REPORT**

International application No.
**PCT/CN2024/072461**

**A. CLASSIFICATION OF SUBJECT MATTER**

C07D519/00(2006.01)i； A61K31/437(2006.01)i； A61P35/00(2006.01)i； A61P17/00(2006.01)i； A61P19/02(2006.01)i； A61P29/00(2006.01)i； A61P5/48(2006.01)i； A61P1/00(2006.01)i； A61P11/00(2006.01)i； A61P25/00(2006.01)i

According to International Patent Classification (IPC) or to both national classification and IPC

**B. FIELDS SEARCHED**

Minimum documentation searched (classification system followed by classification symbols)

IPC:C07D519/-,A61K31/-

Documentation searched other than minimum documentation to the extent that such documents are included in the fields searched

Electronic data base consulted during the international search (name of data base and, where practicable, search terms used)

CNKI, VEN, CNTXT, DWPI, STN(Reg, Caplus): 吡咯并吡啶, 吡唑并, 四氢吡啶, 哌啶, JAK, janus, pyrrolo+, pyridin+, 权利要求1的结构式进行了检索, search conducted the structural formula of claim 1

**C. DOCUMENTS CONSIDERED TO BE RELEVANT**

| Category* | Citation of document, with indication, where appropriate, of the relevant passages | Relevant to claim No. |
|---|---|---|
| A | CN 113429427 A (HUNAN NUCIEN PHARMACEUTICAL CO., LTD. et al.) 24 September 2021 (2021-09-24)<br>entire document | 1-13 |
| PX | CN 115724857 A (HANGZHOU YUHONG PHARMACEUTICAL TECHNOLOGY CO., LTD.) 03 March 2023 (2023-03-03)<br>claims | 1-13 |
| PX | CN 115806561 A (PHARMABLOCK SCIENCES (NANJING), INC.) 17 March 2023 (2023-03-17)<br>entire document | 1-13 |
| PX | CN 116120347 A (HANGZHOU YUHONG PHARMACEUTICAL TECHNOLOGY CO., LTD.) 16 May 2023 (2023-05-16)<br>claims 1-13 | 1-13 |
| PX | CN 115947741 A (HANGZHOU YUHONG PHARMACEUTICAL TECHNOLOGY CO., LTD.) 11 April 2023 (2023-04-11)<br>entire document | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 April 2024** | **30 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/072461**

**C. DOCUMENTS CONSIDERED TO BE RELEVANT**

| Category* | Citation of document, with indication, where appropriate, of the relevant passages | Relevant to claim No. |
|---|---|---|
| A | WO 2015083028 A1 (PFIZER INC.) 11 June 2015 (2015-06-11)<br>entire document | 1-13 |

Form PCT/ISA/210 (second sheet) (July 2022)

**INTERNATIONAL SEARCH REPORT**
**Information on patent family members**

International application No.
**PCT/CN2024/072461**

| Patent document cited in search report | Publication date (day/month/year) | Patent family member(s) | Publication date (day/month/year) |
|---|---|---|---|
| CN 113429427 A | 24 September 2021 | None | |
| CN 115724857 A | 03 March 2023 | CA 3228746 A1 | 02 March 2023 |
| | | AU 2022334383 A1 | 22 February 2024 |
| | | WO 2023025182 A1 | 02 March 2023 |
| CN 115806561 A | 17 March 2023 | None | |
| CN 116120347 A | 16 May 2023 | None | |
| CN 115947741 A | 11 April 2023 | None | |
| WO 2015083028 A1 | 11 June 2015 | BR 112016012262 A2 | 30 June 2020 |
| | | BR 112016012262 B1 | 13 April 2021 |
| | | HUE 054560 T2 | 28 September 2021 |
| | | SI 3318565 T1 | 30 July 2021 |
| | | RS 56728 B1 | 30 March 2018 |
| | | ES 2654051 T3 | 12 February 2018 |
| | | MX 2016007156 A | 21 July 2016 |
| | | MX 368464 B | 02 October 2019 |
| | | AP 201609269 A0 | 30 June 2016 |
| | | SV 2016005209 A | 11 April 2018 |
| | | MY 187446 A | 22 September 2021 |
| | | US 2015158864 A1 | 11 June 2015 |
| | | US 9617258 B2 | 11 April 2017 |
| | | PT 3318565 T | 28 May 2021 |
| | | EP 3077395 A1 | 12 October 2016 |
| | | EP 3077395 B1 | 15 November 2017 |
| | | US 2017247372 A1 | 31 August 2017 |
| | | US 11111242 B2 | 07 September 2021 |
| | | CU 20160077 A7 | 10 January 2017 |
| | | CU 24396 B1 | 04 April 2019 |
| | | HRP 20171846 T1 | 12 January 2018 |
| | | ES 2871524 T3 | 29 October 2021 |
| | | CL 2016001216 A1 | 20 January 2017 |
| | | AR 099363 A1 | 20 July 2016 |
| | | MA 39092 A1 | 29 June 2018 |
| | | MA 39092 B1 | 28 September 2018 |
| | | PT 3077395 T | 03 January 2018 |
| | | KR 20160092012 A | 03 August 2016 |
| | | KR 101930603 B1 | 18 December 2018 |
| | | EP 3318565 A1 | 09 May 2018 |
| | | EP 3318565 B1 | 14 April 2021 |
| | | TW 201524977 A | 01 July 2015 |
| | | TWI 548636 B | 11 September 2016 |
| | | UA 117040 C2 | 11 June 2018 |
| | | JP 2016539137 A | 15 December 2016 |
| | | JP 6192839 B2 | 06 September 2017 |
| | | NL 301245 I2 | 20 November 2023 |
| | | DK 3077395 T3 | 02 January 2018 |
| | | GT 201600098 A | 17 October 2019 |
| | | SI 3077395 T1 | 30 March 2018 |
| | | HRP 20210770 T1 | 25 June 2021 |
| | | CR 20160250 A | 19 September 2016 |

Form PCT/ISA/210 (patent family annex) (July 2022)

**INTERNATIONAL SEARCH REPORT**
**Information on patent family members**

International application No.
**PCT/CN2024/072461**

| Patent document cited in search report | Publication date (day/month/year) | Patent family member(s) | | | Publication date (day/month/year) |
|---|---|---|---|---|---|
| | | FIC | 20230035 | I1 | 22 November 2023 |
| | | MD | 20160058 | A2 | 30 November 2016 |
| | | MD | 4649 | B1 | 30 September 2019 |
| | | MD | 4649 | C1 | 30 April 2020 |
| | | CY | 1124793 | T1 | 25 November 2022 |
| | | HUS | 2300040 | I1 | 28 December 2023 |
| | | JP | 2018008996 | A | 18 January 2018 |
| | | NO | 2024001 | I1 | 09 January 2024 |
| | | CY | 1119778 | T1 | 27 June 2018 |
| | | NI | 201600075 | A | 09 August 2016 |
| | | ME | 02883 | B | 20 April 2018 |
| | | NZ | 720092 | A | 31 May 2019 |
| | | AU | 2014358792 | A1 | 02 June 2016 |
| | | AU | 2014358792 | B2 | 08 June 2017 |
| | | AU | 2014358792 | C1 | 26 August 2021 |
| | | US | 2023009153 | A1 | 12 January 2023 |
| | | PL | 3318565 | T3 | 04 October 2021 |
| | | PE | 20161246 | A1 | 25 November 2016 |
| | | DK | 3318565 | T3 | 25 May 2021 |
| | | NO | 3134430 | T3 | 18 August 2018 |
| | | CA | 2932425 | E | 11 June 2015 |
| | | CA | 2932425 | A1 | 17 July 2018 |
| | | IL | 246038 | A0 | 02 August 2016 |
| | | IL | 246038 | B | 29 October 2020 |
| | | LT | 3077395 | T | 12 February 2018 |
| | | RS | 61897 | B1 | 30 June 2021 |
| | | RS | 61897 | B9 | 30 September 2021 |
| | | TN | 2016000227 | A1 | 06 October 2017 |
| | | PL | 3077395 | T3 | 30 April 2018 |
| | | PH | 12016500938 | A1 | 27 June 2016 |
| | | EA | 201600373 | A1 | 31 October 2016 |
| | | EA | 030472 | B1 | 31 August 2018 |
| | | DOP | 2016000124 | A | 31 October 2016 |
| | | LT | 3318565 | T | 25 June 2021 |
| | | UY | 35861 | A | 31 July 2015 |
| | | GEP | 20186840 | B | 10 April 2018 |
| | | HUE | 035264 | T2 | 02 May 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211019771